# EUROPEAN PATENT APPLICATION

(11) **EP 3 915 966 A1**
(43) Date of publication of application: **01.12.2021**
(21) Application number: 21175511.1
(22) Date of filing: 24.05.2021
(51) Int. Cl.: C07B 59/00, C07C 211/54, H01L 51/50

(54) **AMINE COMPOUND AND ORGANIC LIGHT-EMITTING DEVICE COMPRISING THE SAME**

(30) Priority: 26.05.2020 KR 20200063270
(71) Applicant: Samsung Display Co., Ltd., Gyeonggi-do 17113 (KR)
(72) Inventor: Kim, Minji, Yongin-si 17113 (KR); Jeong, Eunjae, Yongin-si 17113 (KR); Han, Sanghyun, Yongin-si 17113 (KR); Kim, Dongjun, Yongin-si 17113 (KR); Pak, Hankyu, Yongin-si 17113 (KR); Jo, Sohee, Yongin-si 17113 (KR)
(74) Representative: Russell, Tim

(57) **Abstract**

Provided are an amine compound represented by Formula 1 and an organic light-emitting device comprising the same. The organic light-emitting device comprises: a first electrode; a second electrode facing the first electrode; and an organic layer between the first electrode and the second electrode and comprising an emission layer; and at least one amine compound represented by Formula 1.

## Description

### CROSS-REFERENCE TO RELATED APPLICATION

This application claims priority to and the benefit of Korean Patent Application No. 10-2020-0063270, filed on May 26, 2020, in the Korean Intellectual Property Office, the entire content of which is hereby incorporated by reference.

### BACKGROUND

### 1. Field

One or more embodiments of the present disclosure relate to an amine compound and an organic light-emitting device comprising the same.

### 2. Description of Related Art

Organic light-emitting devices (OLEDs) are self-emission devices that, as compared with other devices, have wide viewing angles, high contrast ratios, short response times, and excellent characteristics in terms of luminance, driving voltage, and response speed, and produce full-color images.

OLEDs may comprise a first electrode on a substrate, and a hole transport region, an emission layer, an electron transport region, and a second electrode sequentially stacked on the first electrode. Holes provided from the first electrode may move toward the emission layer through the hole transport region, and electrons provided from the second electrode may move toward the emission layer through the electron transport region. Carriers, such as holes and electrons, recombine in the emission layer to produce excitons. These excitons transition (e.g., relax) from an excited state to a ground state to thereby generate light.

### SUMMARY

The invention is defined by the claims.

One or more embodiments comprise an amine compound and an organic light-emitting device comprising the same.

Additional aspects of embodiments will be set forth in part in the description which follows and, in part, will be apparent from the description, or may be learned by practice of the presented embodiments of the disclosure.

According to one aspect of an embodiment, there is provided an amine compound represented by Formula 1.

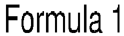

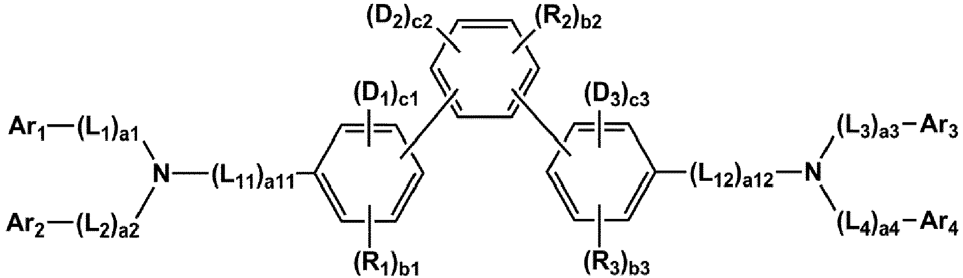

In Formula 1, L₁ to L₄, L₁₁, and L₁₂ are each independently a substituted or unsubstituted C₅-C₆₀ carbocyclic group, or a substituted or unsubstituted C₁-C₆₀ heterocyclic group,
a1 to a4, a11, and a12 are each independently an integer from 0 to 5,
Ar₁ to Ar₄ are each independently a substituted or unsubstituted C₅-C₆₀ carbocyclic group, or a substituted or unsubstituted C₁-C₆₀ heterocyclic group,
provided up to one of Ar₁ to Ar₄ comprises an amine group (e.g., Ar₁ to Ar₄ do not each comprise an amine group),
R₁ to R₃ are each indepednently hydrogen, deuterium, -F, -Cl, -Br, -I, a hydroxyl group, a cyano group, a nitro group, an amidino group, a hydrazino group, a hydrazono group, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, - Si(Q₁)(Q₂)(Q₃), -N(Q₁)(Q₂), -B(Q₁)(Q₂), -C(=O)(Q₁), -S(=O)₂(Q₁), or -P(=O)(Q₁)(Q₂),
D₁ to D₃ are each deuterium,
b1 to b3 and c1 to c3 are each independently an integer from 0 to 4,
the sum of b1 and c1, the sum of b2 and c2, and the sum of b3 and c3 are each 4,
the sum of c1, c2, and c3 is 1 or more, and
at least one substituent of the substituted C₅-C₆₀ carbocyclic group, the substituted C₁-C₆₀ heterocyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group is selected from:
deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, and a C₁-C₆₀ alkoxy group,
a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F, - CI, -Br, -I, a hydroxyl group, a cyano group, a nitro group, an amidino group, a hydrazino group, a hydrazono group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -Si(Q₁₁)(Q₁₂)(Q₁₃), - N(Q₁₁)(Q₁₂), -B(Q₁₁)(Q₁₂), -C(=O)(Q₁₁), -S(=O)₂(Q₁₁), and -P(=O)(Q₁₁)(Q₁₂),
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group,
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one selected from deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -Si(Q₂₁)(Q₂₂)(Q₂₃), - N(Q₂₁)(Q₂₂), -B(Q₂₁)(Q₂₂), -C(=O)(Q₂₁), -S(=O)₂(Q₂₁), and -P(=O)(Q₂₁)(Q₂₂), and
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and -P(=O)(Q₃₁)(Q₃₂),
wherein Q₁ to Q₃, Q₁₁ to Q₁₃, Q₂₁ to Q₂₃, and Q₃₁ to Q₃₃ are each independently be selected from hydrogen, deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, a monovalent non-aromatic condensed heteropolycyclic group, a biphenyl group, and a terphenyl group.

Another aspect of an embodiment of the present disclosure provides an organic light-emitting device comprising a first electrode, a second electrode facing the first electrode, an organic layer between the first electrode and the second electrode and comprising an emission layer, and at least one amine compound represented by Formula 1 as described above.

### BRIEF DESCRIPTION OF THE DRAWINGS

The above and other aspects and features of certain embodiments of the disclosure will be more apparent from the following description taken in conjunction with the accompanying drawings, in which:
FIG. 1 is a schematic cross-sectional view of an embodiment of an organic light-emitting device;
FIG. 2 is a schematic cross-sectional view of an embodiment of an organic light-emitting device;
FIG. 3 is a schematic cross-sectional view of an embodiment of an organic light-emitting device; and
FIG. 4 is a schematic cross-sectional view of an embodiment of an organic light-emitting device.

### DETAILED DESCRIPTION

Reference will now be made in more detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of embodiments of the present description. An amine compound according to an embodiment is represented by Formula 1:

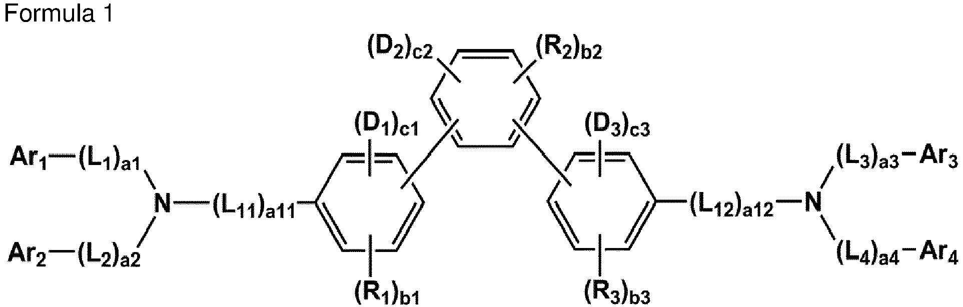

L₁ to L₄, L₁₁, and L₁₂ in Formula 1 are each independently a substituted or unsubstituted C₅-C₆₀ carbocyclic group, or a substituted or unsubstituted C₁-C₆₀ heterocyclic group.

In an embodiment, L₁ to L₄, L₁₁, and L₁₂ may each independently be selected from a phenylene group, a pentalenylene group, an indenylene group, a naphthylene group, an azulenylene group, a heptalenylene group, an indacenylene group, an acenaphthylene group, a fluorenylene group, a spiro-bifluorenylene group, a spiro-fluorene-benzofluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, a ovalenylene group, and a carbazolylene group; and
a phenylene group, a pentalenylene group, an indenylene group, a naphthylene group, an azulenylene group, a heptalenylene group, an indacenylene group, an acenaphthylene group, a fluorenylene group, a spiro-bifluorenylene group, a spiro-fluorene-benzofluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, and a carbazolylene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a dibenzosilolylene group, a benzocarbazolyl group, a dibenzocarbazolyl group, a thiadiazolyl group, an imidazopyridinyl group, an imidazopyrimidinyl group, -Si(Q₃₁)(Q₃₂)(Q₃₃), -N(Q₃₁)(Q₃₂), -B(Q₃₁)(Q₃₂), -C(=O)(Q₃₁), - S(=O)₂(Q₃₁), and -P(=O)(Q₃₁)(Q₃₂),
wherein Q₃₁ to Q₃₃ may each independently be selected from a C₁-C₁₀ alkyl group, a C₁-C₁₀ alkoxy group, a phenyl group, a phenyl group substituted with a C₁-C₁₀ alkyl group, a biphenyl group, a terphenyl group, and a naphthyl group.

For example, L₁ to L₄, L₁₁, and L₁₂ may each independently be a group represented by one of Formulae 3-1 to 3-27: 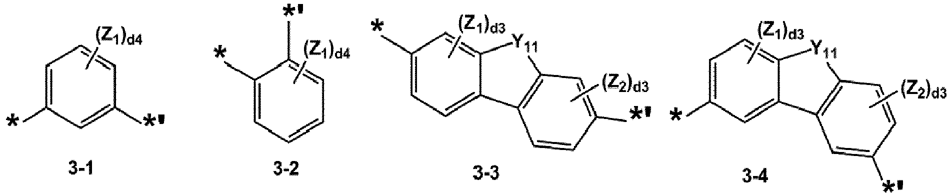 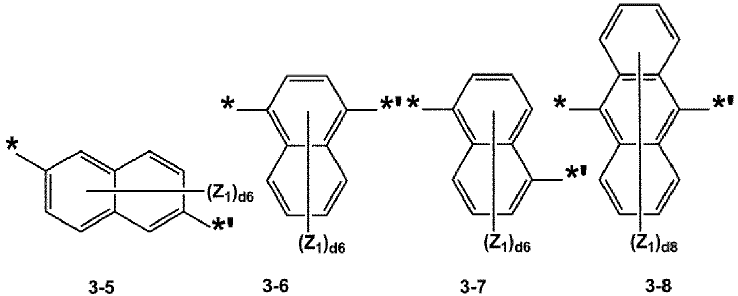 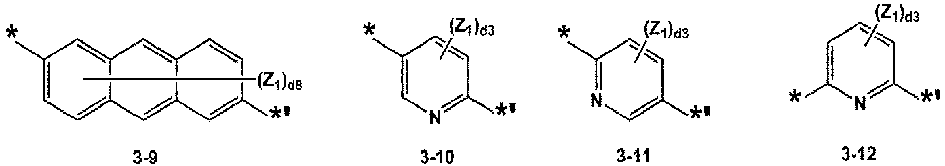 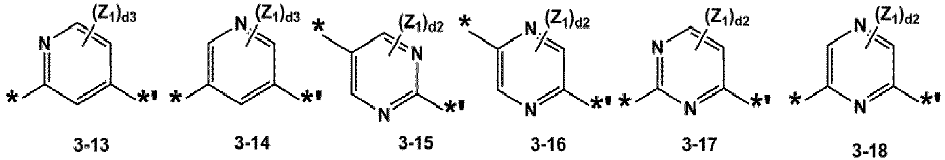 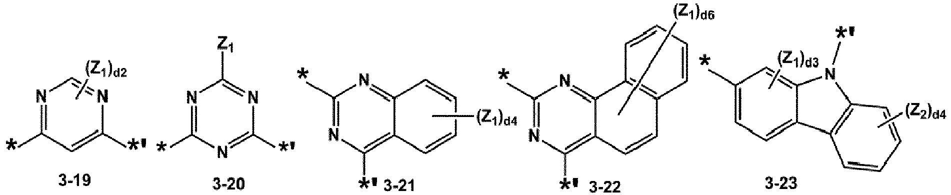 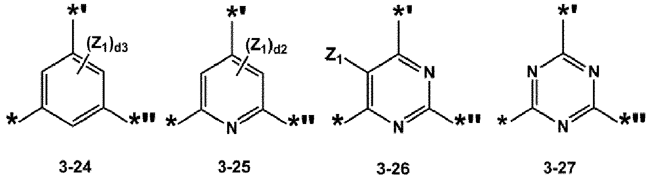 wherein, in Formulae 3-1 to 3-27,
Y₁₁ may be selected from C(Z₃)(Z₄), N(Z₅), Si(Z₆)(Z₇), O, and S, and
Z₁ to Z₇ may each independently be selected from hydrogen, deuterium, -F, - Cl, -Br, -I, a hydroxyl group, a cyano group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a pyrenyl group, a chrysenyl group, a pyrrolyl group, a thiophenyl group, a furanyl group, a silolyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, a benzofuranyl group, a benzothiophenyl group, a benzosilolyl group, a dibenzosilolyl group, and - Si(Q₃₁)(Q₃₂)(Q₃₃),
wherein Q₃₁ to Q₃₃ may each independently be selected from a C₁-C₂₀ alkyl group, a C₁-C₂₀ alkoxy group, a phenyl group, a biphenyl group, a terphenyl group, a naphthyl group, and a pyridinyl group,
d2 may be an integer from 0 to 2,
d3 may be an integer from 0 to 3,
d4 may be an integer from 0 to 4,
d6 may be an integer from 0 to 6,
d8 may be an integer from 0 to 8, and
* and *' each indicate a binding site to a neighboring atom.

In Formula 1, a1 to a4, a11, and a12 are each independently an integer from 0 to 5.

In an embodiment, a11 and a12 are each 0.

In an embodiment, a1 to a4 may each independently be an integer from 0 to 3.

For example, a1 to a4 may each independently be an integer from 0 to 2.

Ar₁ to Ar₄ in Formula 1 are each independently a substituted or unsubstituted C₅-C₆₀ carbocyclic group, or a substituted or unsubstituted C₁-C₆₀ heterocyclic group, and provided up to one of Ar₁ to Ar₄ comprises an amine group(e.g., Ar₁ to Ar₄ do not each comprise an amine group). In other words, Ar₁ to Ar₄ in Formula 1 are each independently a substituted or unsubstituted C₅-C₆₀ carbocyclic group, or a substituted or unsubstituted C₁-C₆₀ heterocyclic group, provided that not more than one of Ar₁ to Ar4 comprises an amine group.

In an embodiment, Ar₁ to Ar₄ may each independently be selected from:
a phenyl group, a naphthyl group, a fluorenyl group, a spiro-bifluorenyl group, a spiro-fluorene-benzofluorenyl group, a benzofluorenyl group, a dibenzofluorenyl group, a benzonaphthofluorenyl group, a pyrenyl group, a phenalenyl group, a phenanthrenyl group, an anthracenyl group, a fluoranthenyl group, a triphenylenyl group, a pyrrolyl group, a thiophenyl group, a furanyl group, a silolyl group, an imidazolyl group, a pyrazolyl group, a thiazolyl group, an isothiazolyl group, an oxazolyl group, an isoxazolyl group, a pyridinyl group, a pyrimidyl group, a quinolinyl group, an isoquinolinyl group, a benzoquinolinyl group, a naphthyridinyl group, a quinoxalinyl group, a quinazolinyl group, a carbazolyl group, a phenanthridinyl group, an acridinyl group, a phenanthrolinyl group, a phenazinyl group, a triazinyl group, a dibenzofuranyl group, a dibenzothiophenyl group, a biphenyl group, and a terphenyl group; and
a phenyl group, a naphthyl group, a fluorenyl group, a spiro-bifluorenyl group, a spiro-fluorene-benzofluorenyl group, a benzofluorenyl group, a dibenzofluorenyl group, a benzonaphthofluorenyl group, a pyrenyl group, a phenalenyl group, a phenanthrenyl group, an anthracenyl group, a fluoranthenyl group, a triphenylenyl group, a pyrrolyl group, a thiophenyl group, a furanyl group, a silolyl group, an imidazolyl group, a pyrazolyl group, a thiazolyl group, an isothiazolyl group, an oxazolyl group, an isoxazolyl group, a pyridinyl group, a pyrimidyl group, a quinolinyl group, an isoquinolinyl group, a benzoquinolinyl group, a naphthyridinyl group, a quinoxalinyl group, a quinazolinyl group, a carbazolyl group, a phenanthridinyl group, an acridinyl group, a phenanthrolinyl group, a phenazinyl group, a triazinyl group, a dibenzofuranyl group, a dibenzothiophenyl group, a biphenyl group, and a terphenyl group, each substituted with at least one selected from deuterium, -F, -CI, -Br, -I, a hydroxyl group, a cyano group, a nitro group, an amidino group, a hydrazino group, a hydrazono group, a methyl group, an ethyl group, a propyl group, an isobutyl group, a sec-butyl group, a ter-butyl group, pentyl group, an isoamyl group, a hexyl group, a C₁-C₂₀ alkoxy group, a cyclopentyl group, a cyclohexyl group, a cycloheptyl group, a cyclopentenyl group, a cyclohexenyl group, a phenyl group, a naphthyl group, a fluorenyl group, a spiro-bifluorenyl group, a spiro-fluorene-benzofluorenyl group, a benzofluorenyl group, a dibenzofluorenyl group, a benzonaphthofluorenyl group, a pyrenyl group, a phenalenyl group, a phenanthrenyl group, an anthracenyl group, a fluoranthenyl group, a triphenylenyl group, a pyrrolyl group, a thiophenyl group, a furanyl group, a silolyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an indolyl group, an isoindolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, a benzofuranyl group, a benzothiophenyl group, a benzosilolyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a dibenzosilolyl group, a carbazolyl group, a benzocarbazolyl group, a dibenzocarbazolyl group, a thiadiazolyl group, an imidazopyridinyl group, an imidazopyrimidinyl group, an oxazolopyridinyl group, a thiazolopyridinyl group, a benzonaphthyridinyl group, an azafluorenyl group, an azaspiro-bifluorenyl group, an azacarbazolyl group, an azadibenzofuranyl group, an azadibenzothiophenyl group, an azadibenzosilolyl group, a biphenyl group, and a terphenyl group.

For example, Ar₁ to Ar₄ may each independently be a group represented by one of Formulae 5-1 to 5-27: 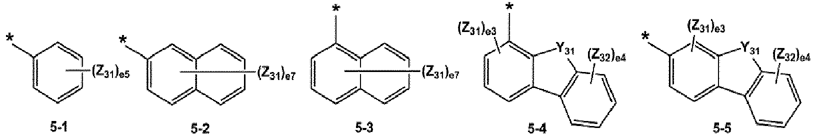 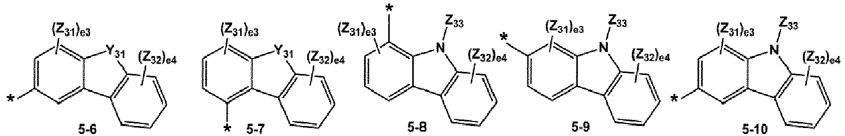 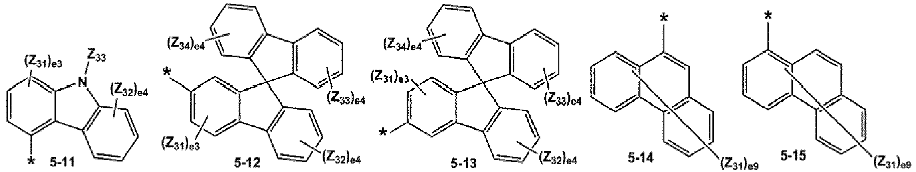 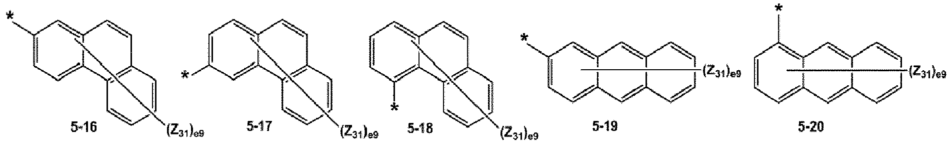 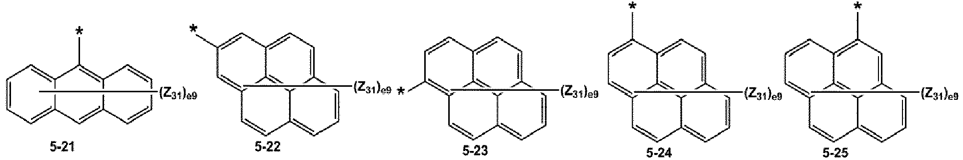 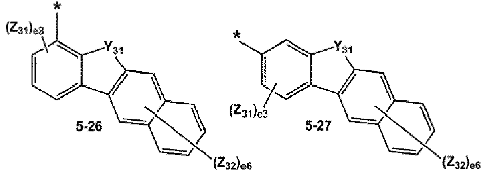 wherein, in Formulae 5-1 to 5-27,
Y₃₁ may be O, S, C(Z₃₅)(Z₃₆), or Si(Z₃₇)(Z₃₈);
Z₃₁ to Z₃₈ may each independently be any one selected from hydrogen, deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, an anthracenyl group, a phenanthrenyl group, an imidazolyl group, a pyrazole, a thiazolyl group, an isothiazolyl group, an oxazolyl group, an isoxazolyl group, a pyridinyl group, a pyrazinyl group, pyrimidinyl group, a pyridazinyl group, an indazolyl group, purinyl(purinyl) group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an oxadiazolyl group, a triazinyl group, a thiadiazolyl group, an imidazopyridinyl group, an imidazopyrimidinyl group, and an azacarbazolyl group;
e3 may be an integer from 0 to 3,
e4 may be an integer from 0 to 4,
e5 may be an integer from 0 to 5,
e6 may be an integer from 0 to 6,
e7 may be an integer from 0 to 7,
e9 may be an integer from 0 to 9, and
* indicates a binding site to an adjacent atom.

In an embodiment, none of Ar₁ to Ar₄ comprises an amine group (e.g. Ar₁ to Ar4 each may not comprise an amine group).

In Formula 1, R₁ to R₃ are each independently hydrogen, deuterium, -F, -Cl, -Br, -I, a hydroxyl group, a cyano group, a nitro group, an amidino group, a hydrazino group, a hydrazono group, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, -Si(Q₁)(Q₂)(Q₃), -N(Q₁)(Q₂), -B(Q₁)(Q₂), -C(=O)(Q₁), - S(=O)₂(Q₁), or -P(=O)(Q₁)(Q₂), and
D₁ to D₃ are each deuterium.

In an embodiment, R₁ to R₃ may each independently be selected from:
hydrogen, deuterium, -F, -Cl, -Br, -I, a hydroxyl group, a cyano group, a nitro group, a methyl group, an ethyl group, a propyl group, an isobutyl group, a sec-butyl group, a ter-butyl group, a C₁-C₂₀ alkoxy group, a cyclopentyl group, a cyclohexyl group, a phenyl group, a naphthyl group, a fluorenyl group, a carbazolyl group, a dibenzofuranyl group, a dibenzothiophenyl group, a biphenyl group, and a terphenyl group; and
a methyl group, an ethyl group, a propyl group, an isobutyl group, a sec-butyl group, a ter-butyl group, a C₁-C₂₀ alkoxy group, a cyclopentyl group, a cyclohexyl group, a cyclopentyl group, a cyclohexyl group, a phenyl group, a naphthyl group, a fluorenyl group, a carbazolyl group, a dibenzofuranyl group, a dibenzothiophenyl group, a biphenyl group, and a terphenyl group, each substituted with at least one selected from deuterium, -F, -Cl, -Br, -I, a hydroxyl group, a cyano group, a nitro group, an amidino group, a hydrazino group, a hydrazono group, a methyl group, an ethyl group, a propyl group, an isobutyl group, a sec-butyl group, a ter-butyl group, a phenyl group, a naphthyl group, a fluorenyl group, a carbazolyl group, a dibenzofuranyl group, a dibenzothiophenyl group, a biphenyl group, and a terphenyl group.

For example, at least one of R₁ to R₃ may be hydrogen.

For example, each of R₁ to R₃ may be hydrogen.

In Formula 1, b1 to b3 and c1 to c3 each independently an integer from 0 to 4,
the sum of b1 and c1, the sum of b2 and c2, and the sum of b3 and c3 are each 4, and
the sum of c1, c2, and c3 may be 1 or more.

In an embodiment, one of c1 to c3 may be 4 and the other two may each be 0,
two of c1 to c3 may each be 4 and the other one may be 0, or
c1 to c3 are each 4.

For example, c1 may be 4 and c2 and c3 may each be 0. For example, c2 may be 4 and c1 and c3 may each be 0. For example, c3 may be 4 and c1 and c2 may each be 0. For example, c1 and c2 may each be 4 and c3 may be 0. For example, c1 and c3 may each be 4 and c2 may be 0. For example, c2 and c3 may each be 4 and c1 may be 0. For example, c1, c2, and c3 may each be 4.

In one or more embodiments, the sum of c1, c2, and c3 may be 2 or more (or 4 or more).

For example, the sum of c1, c2, and c3 may be 3 or more. For example, the sum of c1, c2, and c3 may be 4 or more. For example, the sum of c1, c2, and c3 may be 4, 8, or 12.

In an embodiment, a moiety represented by 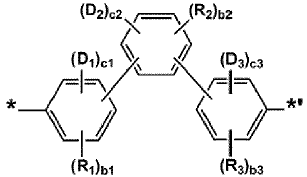 in Formula 1 may be a group represented by one of Formulae 2-1 to 2-6: 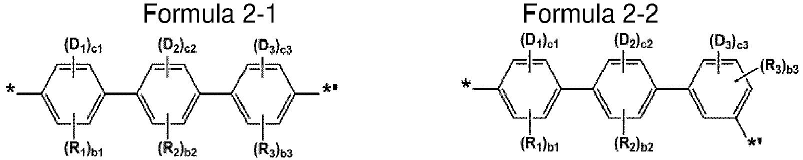 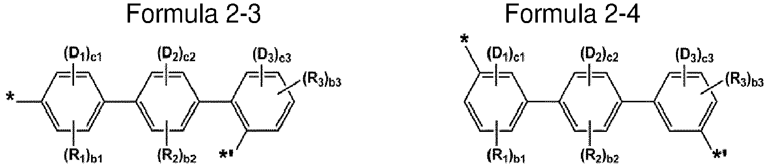 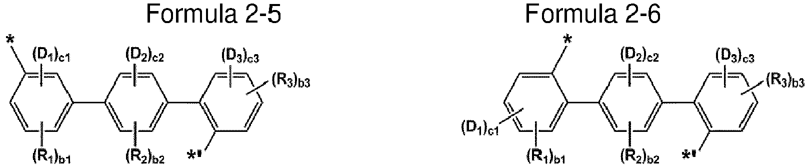 wherein, in Formulae 2-1 to 2-6,
R₁ to R₃, D₁ to D₃, b1 to b3, and c1 to c3 are the same as described above, and
* and *' each indicate a binding site to a neighboring atom.

For example, a moiety represented by 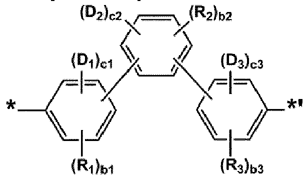 in Formula 1 may be a group represented by Formula 2-1.

Thus, in some embodiments, the amine compound represented by Formula 1 may be represented by Formula 10:

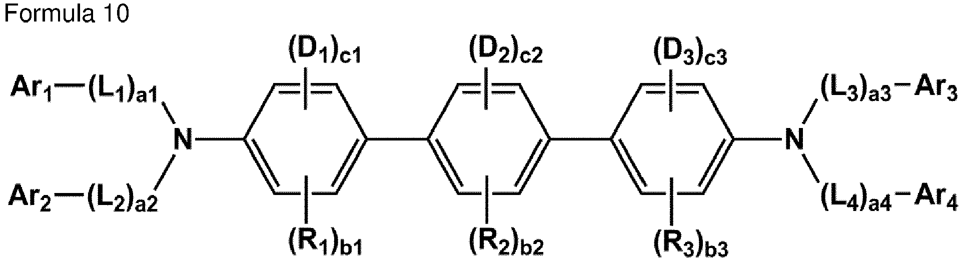

In Formula 10,
L₁ to L₄, a1 to a4, Ar₁ to Ar₄, R₁ to R₃, D₁ to D₃, b1 to b3, and c1 to c3 are the same as described above.

In an embodiment, the amine compound represented by Formula 1 may comprise at least four deuterium (e.g., at least four deuterium atoms).

In an embodiment, the amine compound may be selected from Compounds 1 to 192:

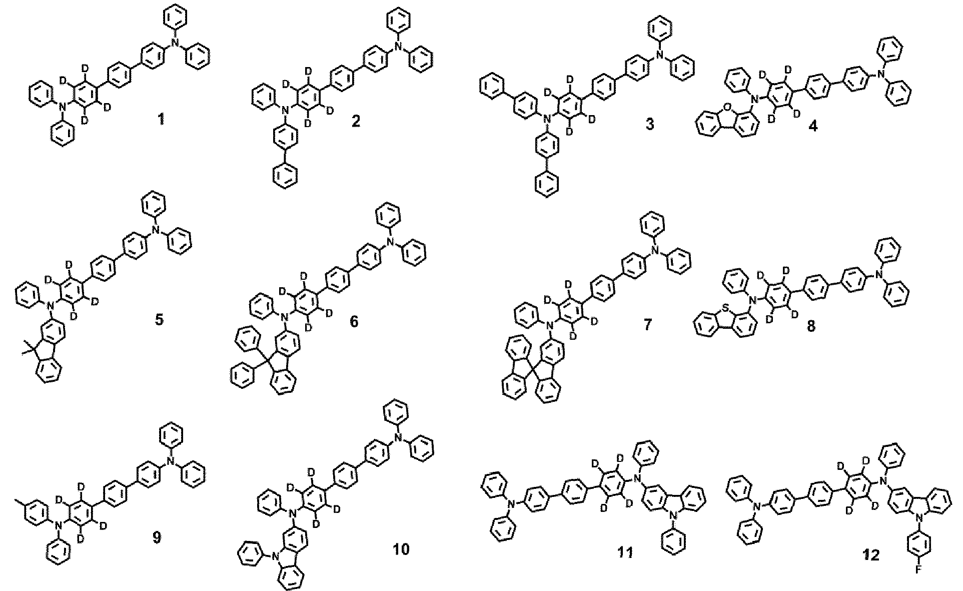

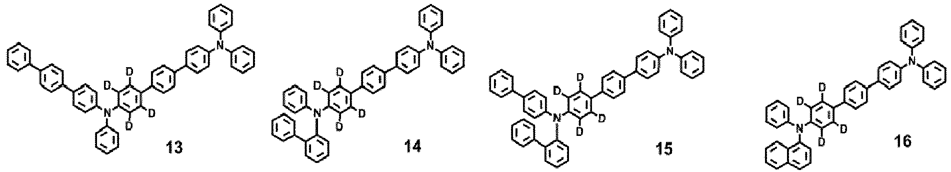

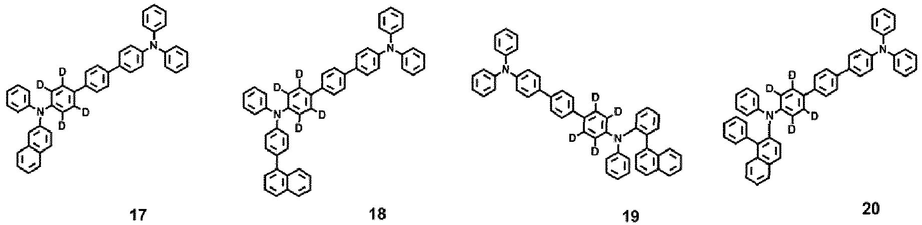

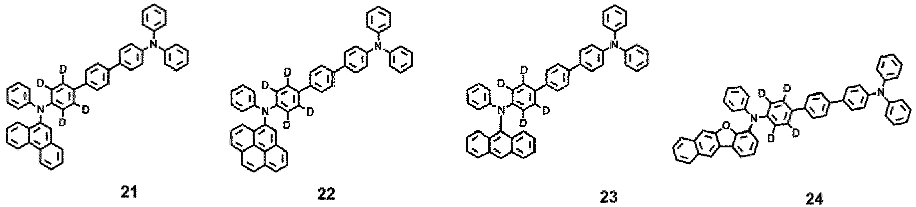

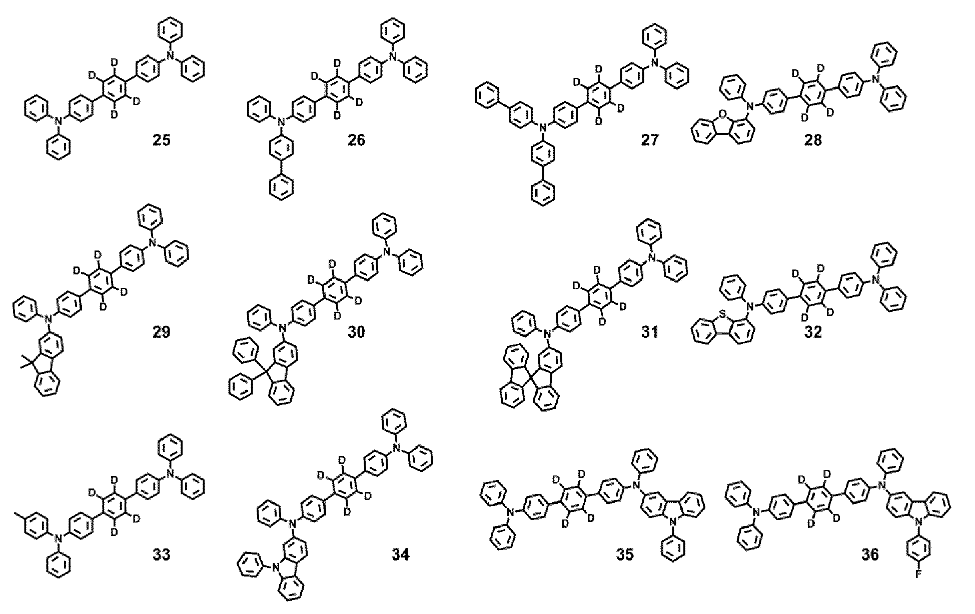

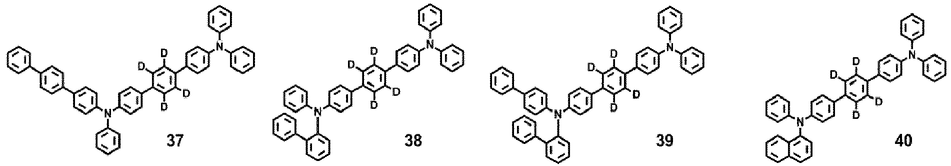

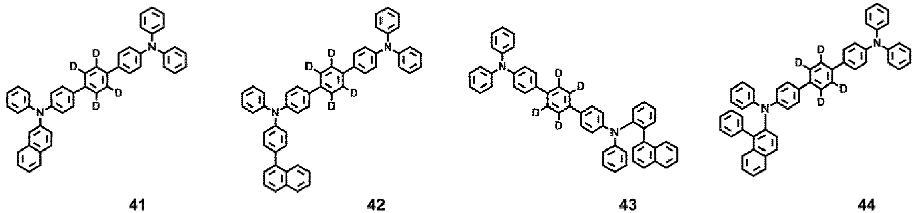

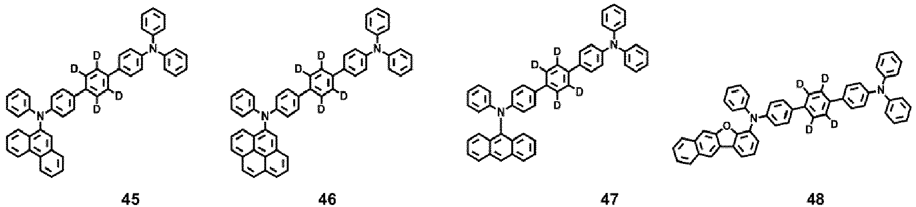

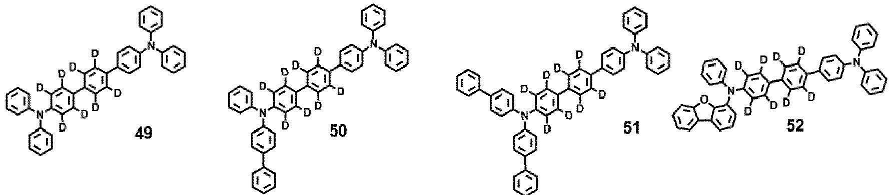

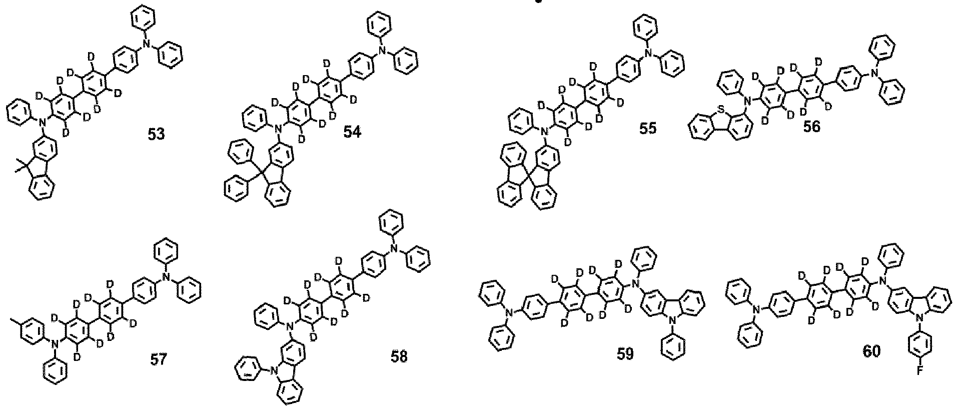

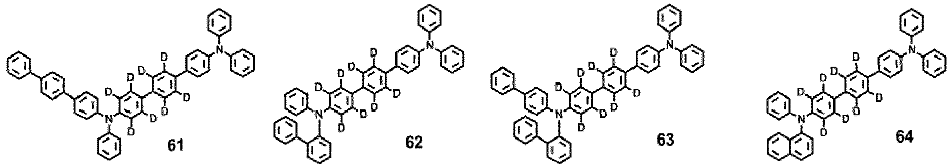

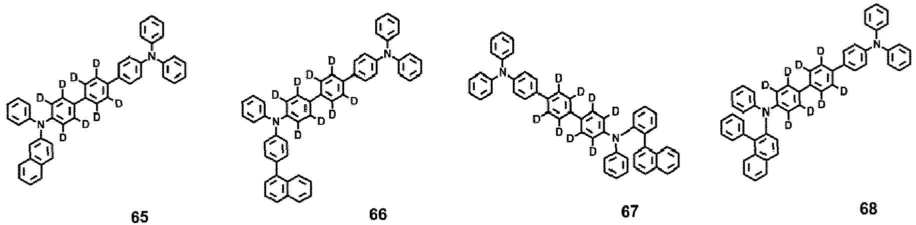

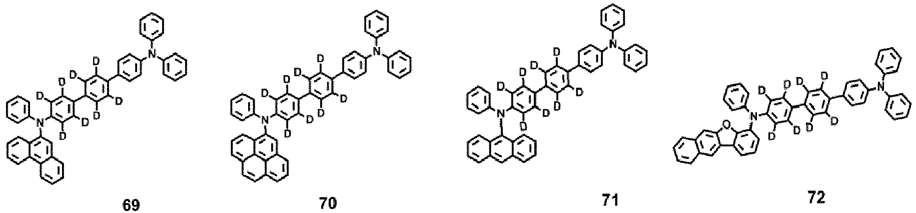

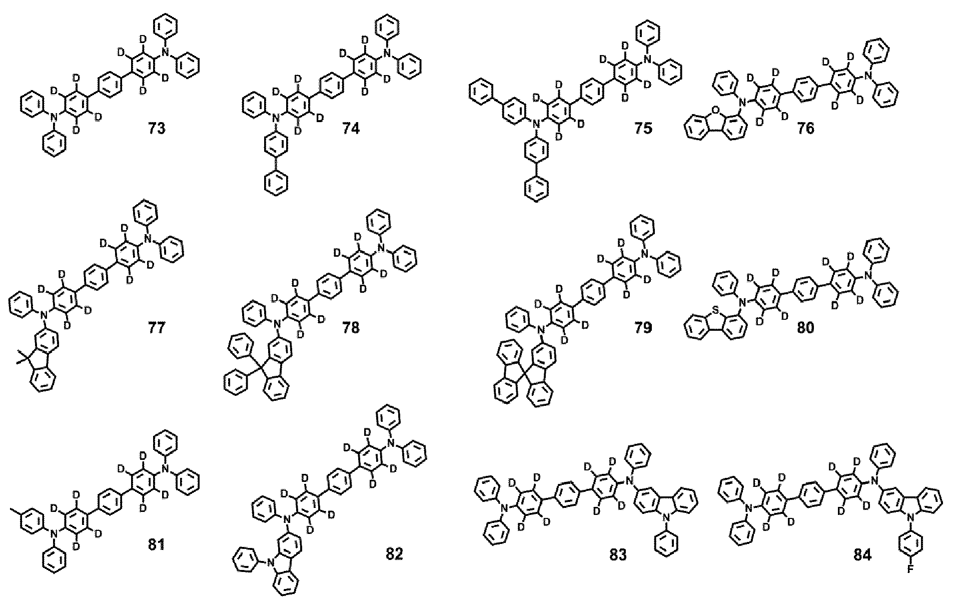

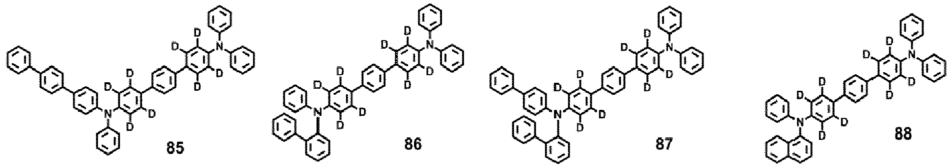

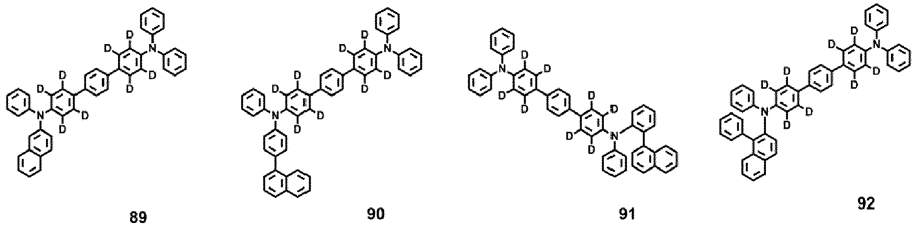

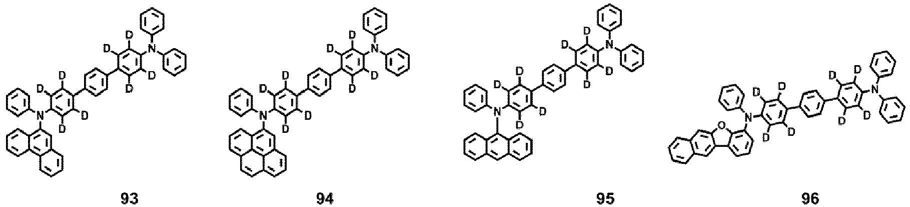

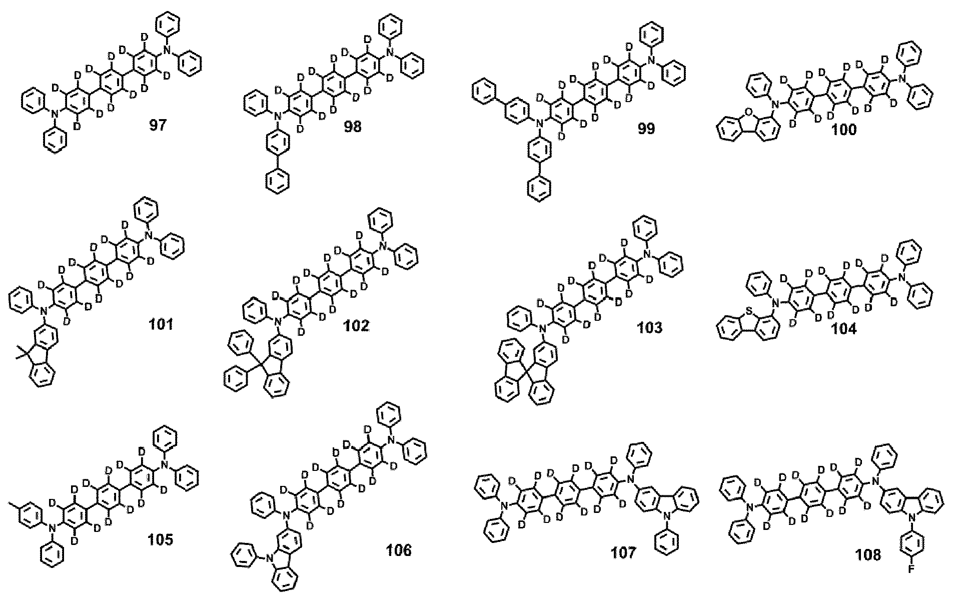

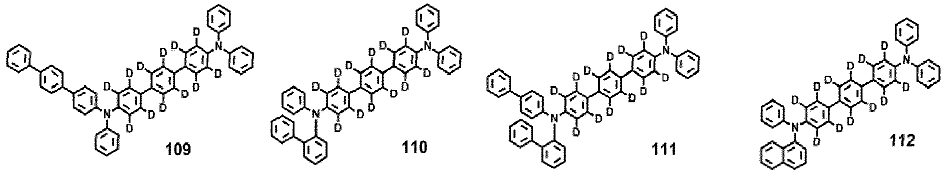

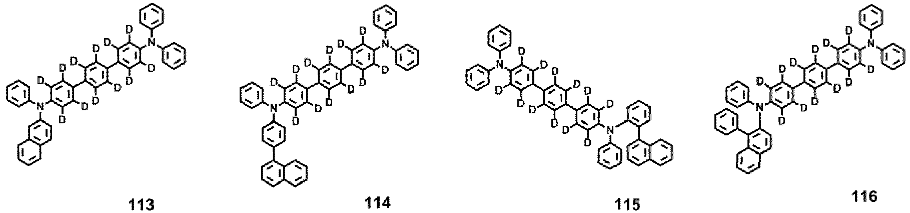

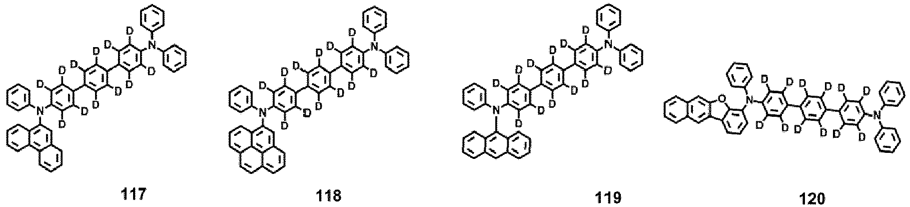

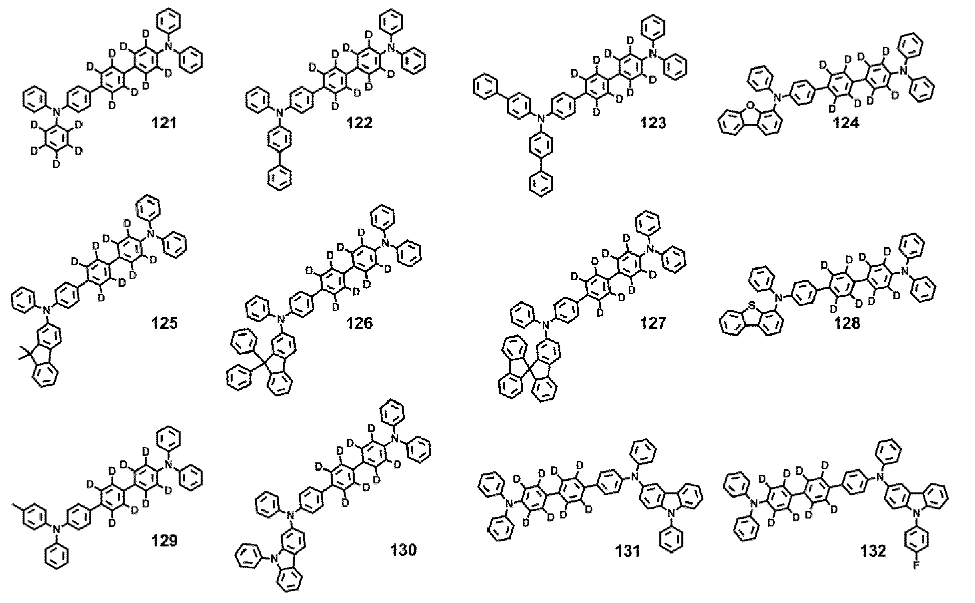

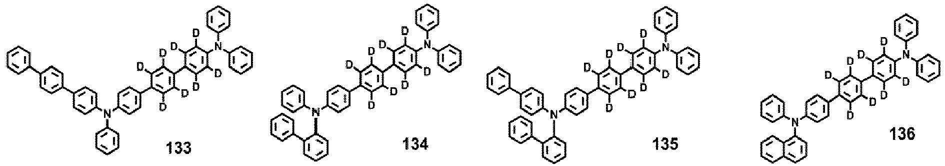

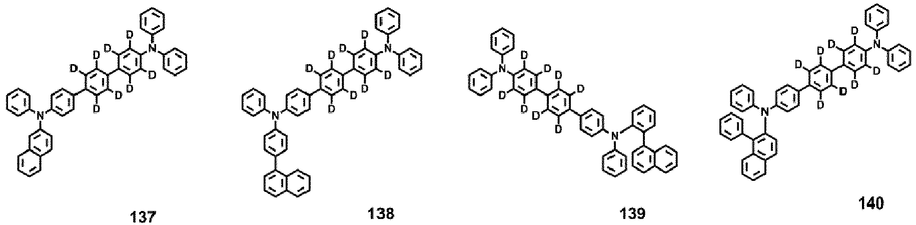

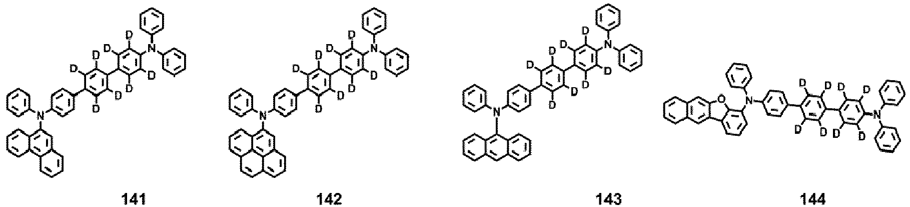

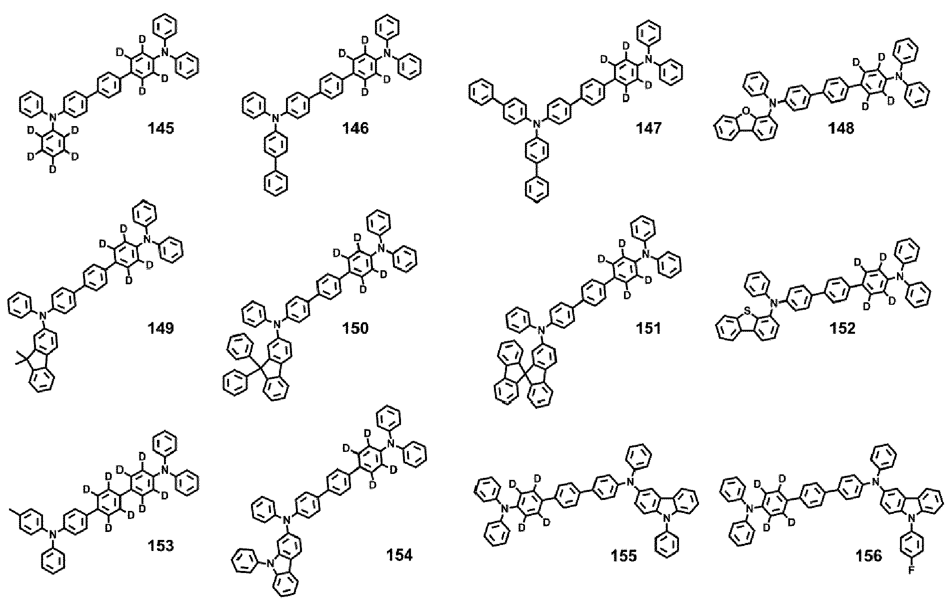

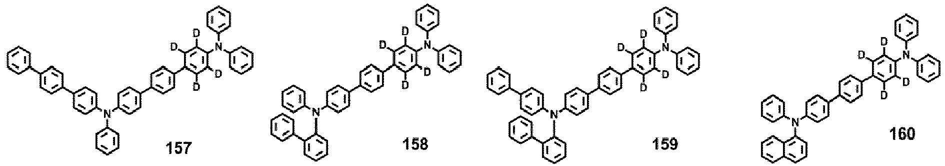

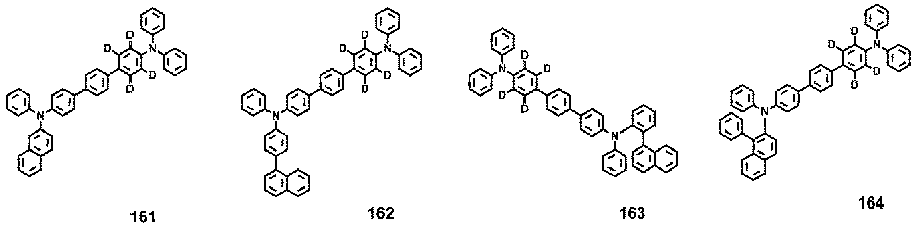

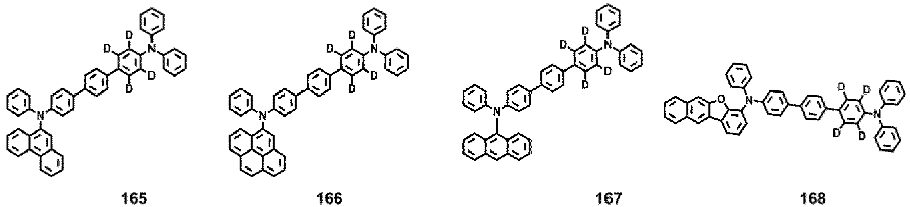

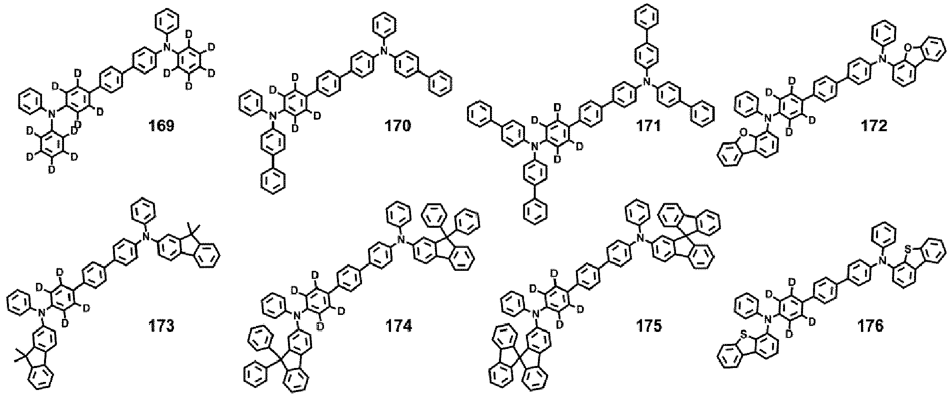

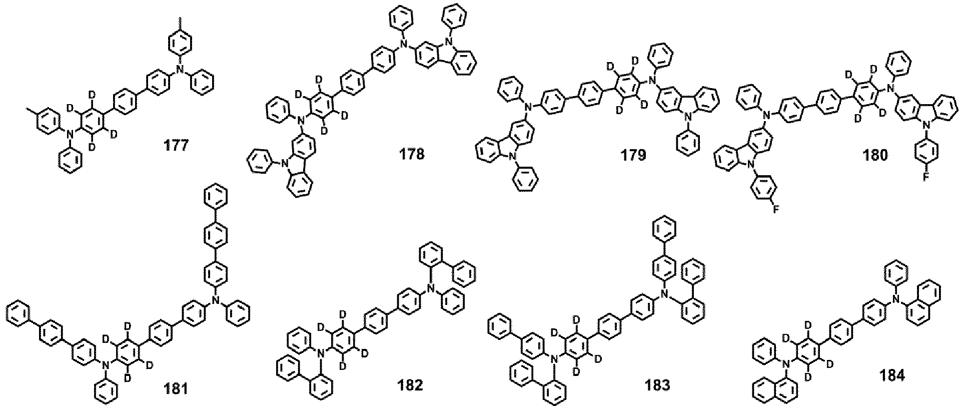

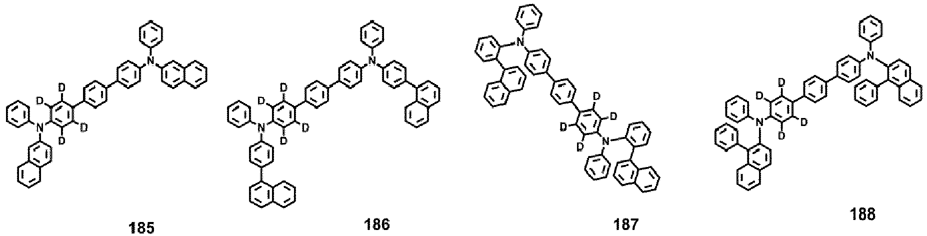

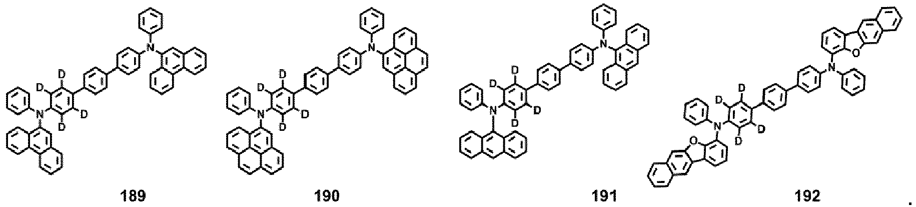

The amine compound is represented by Formula 1, which comprises two amine groups. Due to this structure, the amine compound has a flat structure and is easy to laminate, leading to a low refractive index effect. For example, due to its structure, the majority of the amine compound is in substantially a single plane, thereby improving the ability to stack the amine compound (e.g., making it relatively easier to laminate the amine compound), for example, to stack the amine compound on other molecules of the amine compound.

In addition, the amine compound comprises three or more phenylene groups as linkers among the amine groups, so that the glass transition temperature and/or thermal stability of the amine compound are increased.

In addition, in the amine compound, the amine groups and the three or more phenylene group linkers are optionally connected (e.g., bonded) at the para position with respect to each other. Thus, intermolecular interactions between molecules of the amine compound may be decreased and the glass transition temperature of the amine compound is increased, the synthesis yield is improved, and high efficiency is exhibited.

Furthermore, due to the inclusion of one or more (optionally, four or more) deuterium (e.g., four or more deuterium atoms) in linkers between the amine groups, the amine compound may exhibit a long lifespan.

In addition, because the amine compound does not comprise two or more amine groups in the substituents of the amine group, the energy level in the device is adjusted and a suitable or appropriate energy level for a device using the amine compound may be obtained.

Therefore, an electronic device, for example, an organic light-emitting device, using the amine compound represented by Formula 1 may have a low driving voltage, high luminance, high efficiency, and a long lifespan.

Synthesis methods of the amine compound represented by Formula 1 may be recognizable to one of ordinary skill in the art by referring to Examples provided below.

At least one of such amine compounds represented by Formula 1 may be used between a pair of electrodes of an organic light-emitting device. For example, the amine compound may be comprised in at least one selected from a hole transport region and an emission layer. In one or more embodiments, the amine compound represented by Formula 1 may be used as a material for a capping layer outside the pair of electrodes of an organic light-emitting device.

Accordingly, another aspect of an embodiments of the present disclosure provides an organic light-emitting device comprising: a first electrode; a second electrode facing the first electrode; an organic layer between the first electrode and the second electrode; and at least one amine compound represented by Formula 1. For example, the organic layer comprises at least one of the amine compound.

For example, the organic layer may comprise one or more amine compounds represented by Formula 1.

The expression "(an organic layer) comprises at least one amine compound represented by Formula 1" used herein may comprise a case in which "(an organic layer) comprises identical amine compounds represented by Formula 1" and a case in which "(an organic layer) comprises two or more different amine compounds represented by Formula 1."

For example, the organic layer may comprise, as the amine compound, only Compound 1. In this embodiment, Compound 1 may be comprised in the emission layer of the organic light-emitting device. In one or more embodiments, the organic layer may comprise, as the amine compound, Compound 1 and Compound 2. In this embodiment, Compounds 1 and 2 may be comprised in the same layer (for example, both Compounds 1 and 2 may be comprised in an emission layer) or in different layers (for example, Compound 1 may be comprised in an emission layer, and Compound 2 may be comprised in an electron transport layer).

In some embodiments,
the first electrode of the organic light-emitting device may be an anode,
the second electrode of the organic light-emitting device may be a cathode, and
the organic layer of the organic light-emitting device may further comprise a hole transport region between the first electrode and the emission layer and an electron transport region between the emission layer and the second electrode,
the hole transport region comprises at least one selected from a hole injection layer, a hole transport layer, an emission auxiliary layer, and an electron blocking layer, and
the electron transport region may comprise at least one selected from a buffer layer, a hole blocking layer, an electron control layer, an electron transport layer, and an electron injection layer.

In an embodiment, the amine compound represented by Formula 1 may be comprised in the hole transport region.

In an embodiment, the hole transport region may comprise a hole transport layer, and the amine compound may be comprised in the hole transport layer.

In an embodiment, the hole transport region may comprise a first hole transport layer between the first electrode and the emission layer, and a second hole transport layer between the first hole transport layer and the emission layer, and
the amine compound may be comprised in the first hole transport layer.

In an embodiment, the hole transport region may comprise a first hole transport layer between the first electrode and the emission layer, and a second hole transport layer between the first hole transport layer and the emission layer, and
the amine compound may be comprised in the second hole transport layer.

In an embodiment, the emission layer comprises a host and a dopant, and the dopant may be a fluorescent dopant or phosphorescent dopant.

In an embodiment, the electron transport region may comprise an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combination thereof.

The term "organic layer," as used herein, refers to a single layer and/or all layers between the first electrode and the second electrode of the organic light-emitting device. A material comprised in "the organic layer" is not limited to an organic material. For example, the organic layer may comprise an inorganic material.

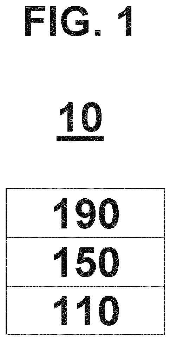

### Description of FIG. 1

FIG. 1 is a schematic cross-sectional view of an organic light-emitting device 10 according to an embodiment. The organic light-emitting device 10 comprises a first electrode 110, an organic layer 150, and a second electrode 190.

Hereinafter, the structure of the organic light-emitting device 10 according to an embodiment and a method of manufacturing the organic light-emitting device 10 will be described in connection with FIG. 1.

### First electrode 110

In FIG. 1, a substrate may be additionally under the first electrode 110 or above the second electrode 190. The substrate may be a glass substrate and/or a plastic substrate . Each may have excellent mechanical strength, thermal stability, transparency, surface smoothness, ease of handling, and/or water resistance.

The first electrode 110 may be formed by, for example, depositing and/or sputtering a material for forming the first electrode 110 on the substrate. When the first electrode 110 is an anode, the material for the first electrode 110 may be selected from materials having a high work function to facilitate hole injection.

The first electrode 110 may be a reflective electrode, a semi-transmissive electrode, or a transmissive electrode. When the first electrode 110 is a transmissive electrode, a material for forming the first electrode 110 may be selected from indium tin oxide (ITO), indium zinc oxide (IZO), tin oxide (SnO₂), zinc oxide (ZnO), and any combination thereof, but embodiments of the present disclosure are not limited thereto. In one or more embodiments, when the first electrode 110 is a semi-transmissive electrode or a reflective electrode, a material for forming the first electrode 110 may be selected from magnesium (Mg), silver (Ag), aluminum (Al), aluminum-lithium (Al-Li), calcium (Ca), magnesium-indium (Mg-In), magnesium-silver (Mg-Ag), and any combination thereof, but embodiments of the present disclosure are not limited thereto.

The first electrode 110 may have a single-layered structure or a multi-layered structure comprising two or more layers. For example, the first electrode 110 may have a three-layered structure of ITO/Ag/ITO, but the structure of the first electrode 110 is not limited thereto.

### Organic layer 150

The organic layer 150 is on the first electrode 110. The organic layer 150 may comprise an emission layer.

The organic layer 150 may further comprise a hole transport region between the first electrode 110 and the emission layer and an electron transport region between the emission layer and the second electrode 190.

### Hole transport region in organic layer 150

The hole transport region may have i) a single-layered structure comprising a single layer comprising a single material, ii) a single-layered structure comprising a single layer comprising a plurality of different materials, or iii) a multi-layered structure having a plurality of layers comprising a plurality of different materials.

The hole transport region may comprise at least one layer selected from a hole injection layer, a hole transport layer, an emission auxiliary layer, and an electron blocking layer.

In an embodiment, the hole transport region may have a single-layered structure comprising a single layer comprising a plurality of different materials, or a multi-layered structure having a hole injection layer/hole transport layer structure, a hole injection layer/hole transport layer/emission auxiliary layer structure, a hole injection layer/emission auxiliary layer structure, a hole transport layer/emission auxiliary layer structure, or a hole injection layer/hole transport layer/electron blocking layer structure, wherein for each structure, constituting layers are sequentially stacked from the first electrode 110 in this stated order, but the structure of the hole transport region is not limited thereto.

The hole transport region may comprise at least one selected from m-MTDATA, TDATA, 2-TNATA, NPB (NPD), β-NPB, TPD, spiro-TPD, spiro-NPB, methylated-NPB, TAPC, HMTPD, 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor sulfonic acid (PANI/CSA), polyaniline/poly(4-styrenesulfonate) (PANI/PSS), a compound represented by Formula 201, and a compound represented by Formula 202: 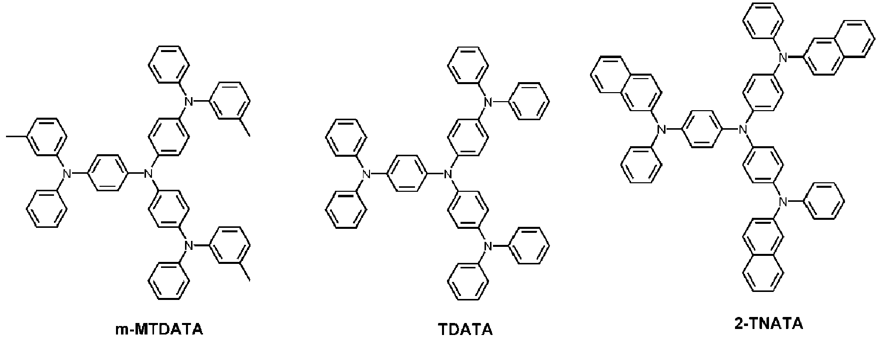 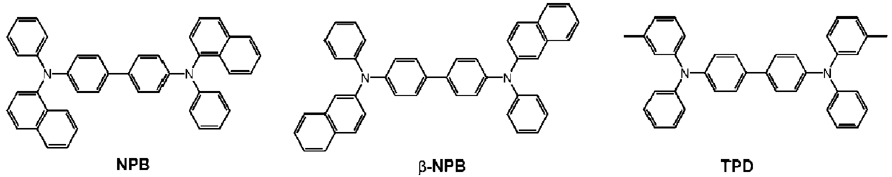 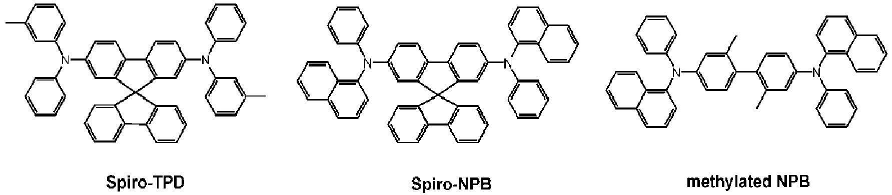 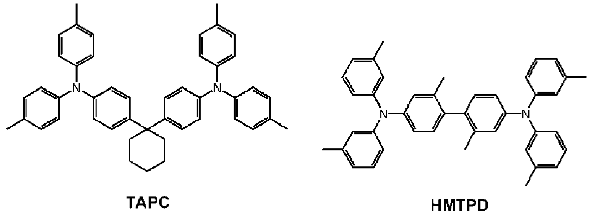 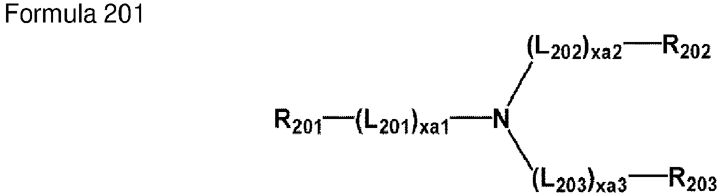 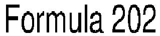 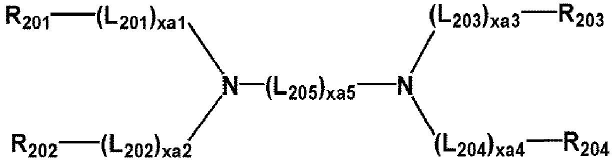 wherein, in Formulae 201 and 202,
L₂₀₁ to L₂₀₄ may each independently be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
L₂₀₅ may be selected from *-O-*', *-S-*', *-N(Q₂₀₁)-*', a substituted or unsubstituted C₁-C₂₀ alkylene group, a substituted or unsubstituted C₂-C₂₀ alkenylene group, a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
xa1 to xa4 are each independently an integer from 0 to 3,
xa5 is an integer from 1 to 10,
R₂₀₁ to R₂₀₄ and Q₂₀₁ may each independently be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group, and
R₂₀₁ and R₂₀₂ in Formula 202 may optionally be linked to each other via a single bond, a dimethyl-methylene group, or a diphenyl-methylene group, and R₂₀₃ and R₂₀₄ may optionally be linked to each other via a single bond, a dimethyl-methylene group, or a diphenyl-methylene group.

In an embodiment, in Formulae 201 and 202,
L₂₀₁ to L₂₀₅ may each independently be selected from:
a phenylene group, a pentalenylene group, an indenylene group, a naphthylene group, an azulenylene group, a heptalenylene group, an indacenylene group, an acenaphthylene group, a fluorenylene group, a spiro-bifluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group; and
a phenylene group, a pentalenylene group, an indenylene group, a naphthylene group, an azulenylene group, a heptalenylene group, an indacenylene group, an acenaphthylene group, a fluorenylene group, a spiro-bifluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, -Si(Q₃₁)(Q₃₂)(Q₃₃) and -N(Q₃₁)(Q₃₂),
wherein Q₃₁ to Q₃₃ may each independently be selected from a C₁-C₁₀ alkyl group, a C₁-C₁₀ alkoxy group, a phenyl group, a biphenyl group, a terphenyl group, and a naphthyl group.

In one or more embodiments, xa1 to xa4 may each independently be 0, 1, or 2.

In one or more embodiments, xa5 may be 1, 2, 3, or 4.

In one or more embodiments, R₂₀₁ to R₂₀₄ and Q₂₀₁ may each independently be selected from: a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group; and
a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group, each substituted with at least one selected from deuterium, -F, -CI, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, -Si(Q₃₁)(Q₃₂)(Q₃₃), and -N(Q₃₁)(Q₃₂),
wherein Q₃₁ to Q₃₃ are the same as described above.

In one or more embodiments, at least one selected from R₂₀₁ to R₂₀₃ in Formula 201 may each independently be selected from:
a fluorenyl group, a spiro-bifluorenyl group, a carbazolyl group, a dibenzofuranyl group, and a dibenzothiophenyl group; and
a fluorenyl group, a spiro-bifluorenyl group, a carbazolyl group, a dibenzofuranyl group, and a dibenzothiophenyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a naphthyl group, a fluorenyl group, a spiro-bifluorenyl group, a carbazolyl group, a dibenzofuranyl group, and a dibenzothiophenyl group,
but embodiments of the present disclosure are not limited thereto.

In one or more embodiments, in Formula 202, i) R₂₀₁ and R₂₀₂ may be linked to each other via a single bond, and/or ii) R₂₀₃ and R₂₀₄ may be linked to each other via a single bond.

In one or more embodiments, at least one of R₂₀₁ to R₂₀₄ in Formula 202 may be selected from:
a carbazolyl group; and
a carbazolyl group substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a naphthyl group, a fluorenyl group, a spiro-bifluorenyl group, a carbazolyl group, a dibenzofuranyl group, and a dibenzothiophenyl group,
but embodiments of the present disclosure are not limited thereto.

The compound represented by Formula 201 may be represented by Formula 201-1 below:

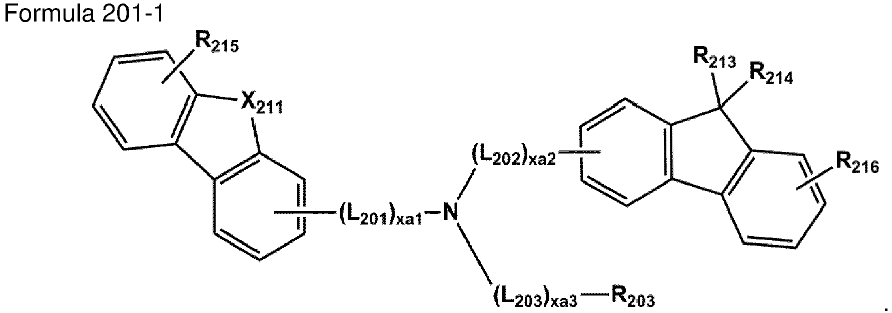

In an embodiment, the compound represented by Formula 201 may be represented by Formula 201-2 below, but embodiments of the present disclosure are not limited thereto:

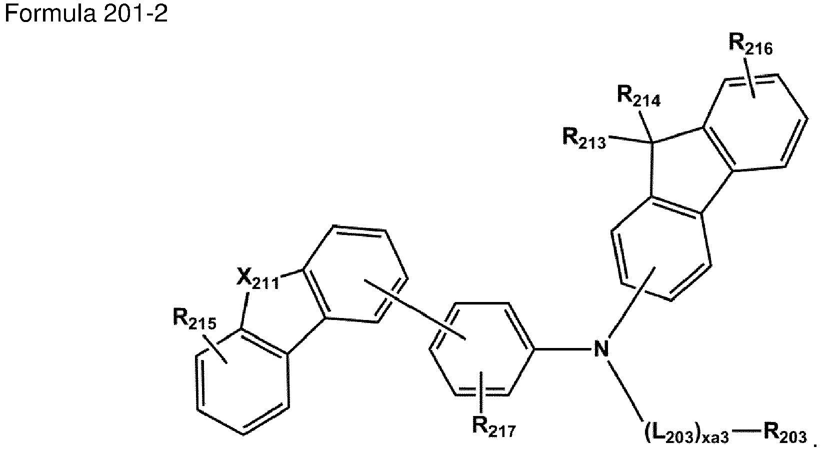

In one or more embodiments, the compound represented by Formula 201 may be represented by Formula 201-2(1) below, but embodiments of the present disclosure are not limited thereto:

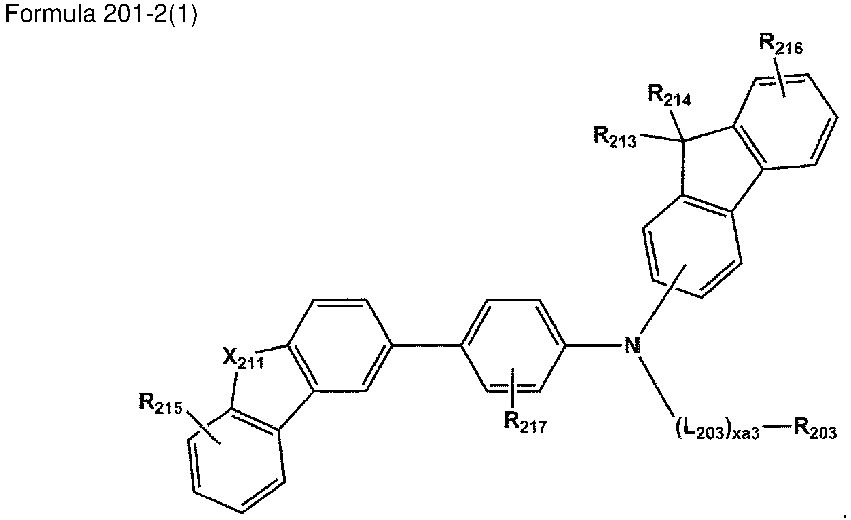

In one or more embodiments, the compound represented by Formula 201 may be represented by Formula 201A below:

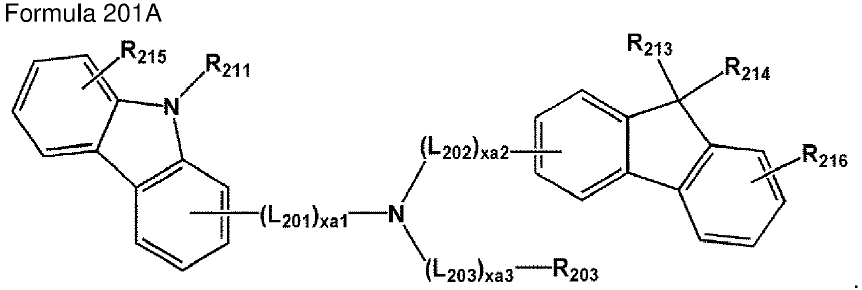

In one or more embodiments, the compound represented by Formula 201 may be represented by Formula 201A(1) below, but embodiments of the present disclosure are not limited thereto:

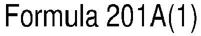

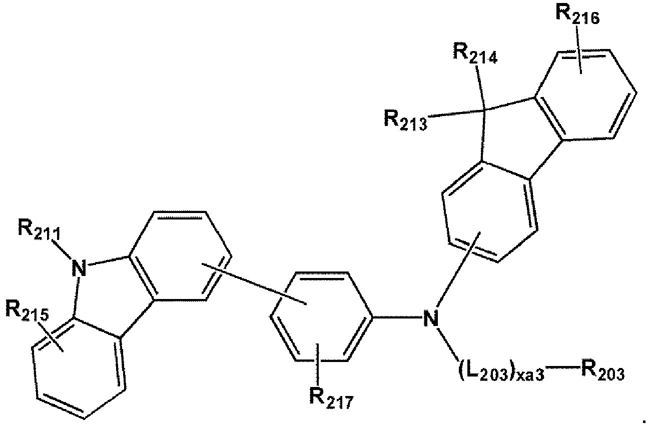

In one or more embodiments, the compound represented by Formula 201 may be represented by Formula 201A-1 below, but embodiments of the present disclosure are not limited thereto:

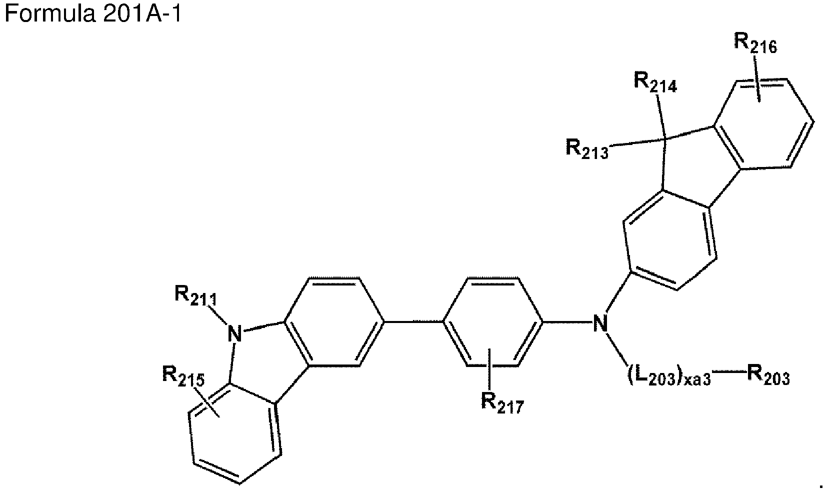

In an embodiment, the compound represented by Formula 202 may be represented by Formula 202-1 below:

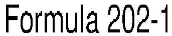

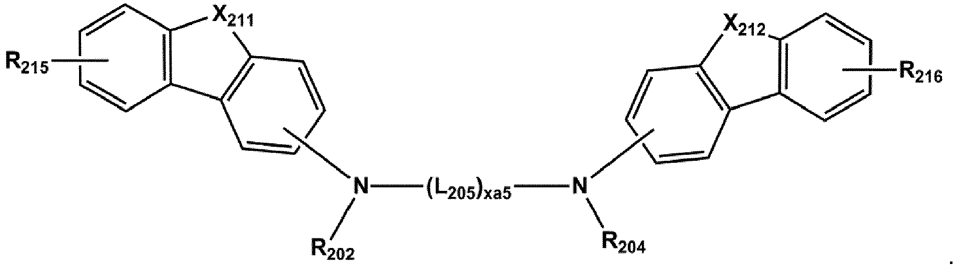

In one or more embodiments, the compound represented by Formula 202 may be represented by Formula 202 -1 (1) below:

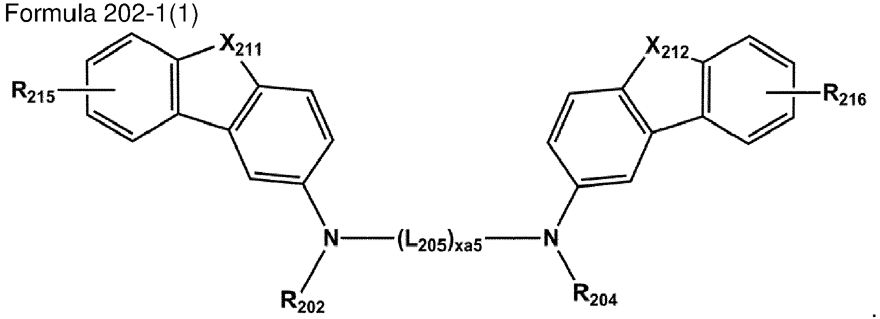

In one or more embodiments, the compound represented by Formula 202 may be represented by Formula 202A below:

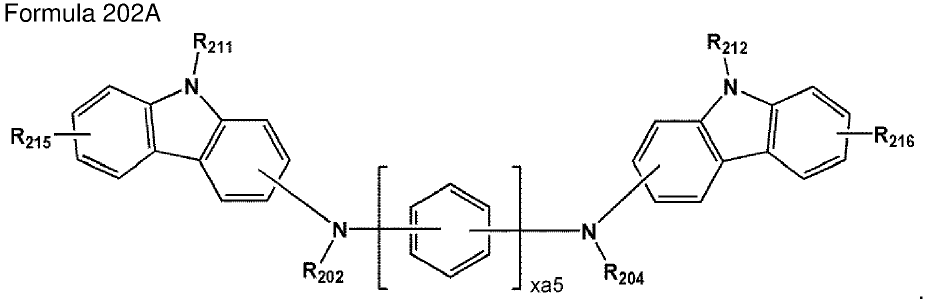

In one or more embodiments, the compound represented by Formula 202 may be represented by Formula 202A-1 below:

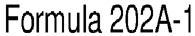

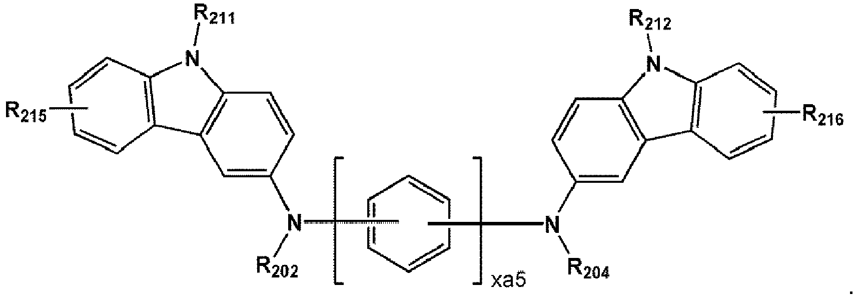

In Formulae 201-1, 201-2, 201-2(1), 201A, 201A(1), 201A-1, 202-1, 202-1(1), 202A, and 202A-1,
L₂₀₁ to L₂₀₃, xa1 to xa3, xa5, and R₂₀₂ to R₂₀₄ are the same as described above,
L₂₀₅ may be selected from a phenylene group, and a fluorenylene group,
X₂₁₁ may be selected from O, S, and N(R₂₁₁),
X₂₁₂ may be selected from O, S, and N(R₂₁₂),
R₂₁₁ and R₂₁₂ are the same as described in connection with R₂₀₃, and
R₂₁₃ to R₂₁₇ may each independently be selected from hydrogen, deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a phenyl group substituted with a C₁-C₁₀ alkyl group, a phenyl group substituted with -F, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group.

The hole transport region may comprise at least one compound selected from Compounds HT1 to HT48, but embodiments of the present disclosure are not limited thereto:

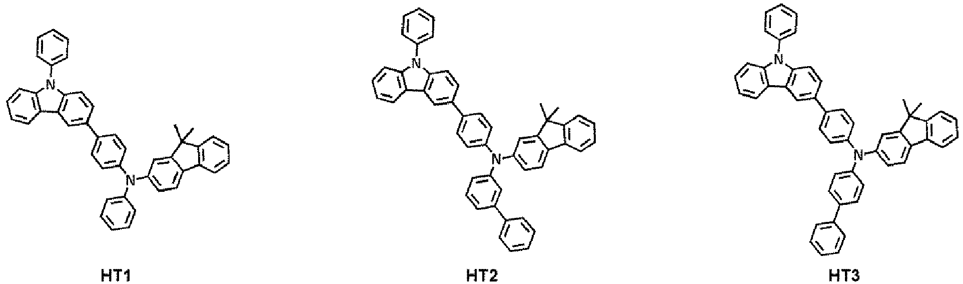

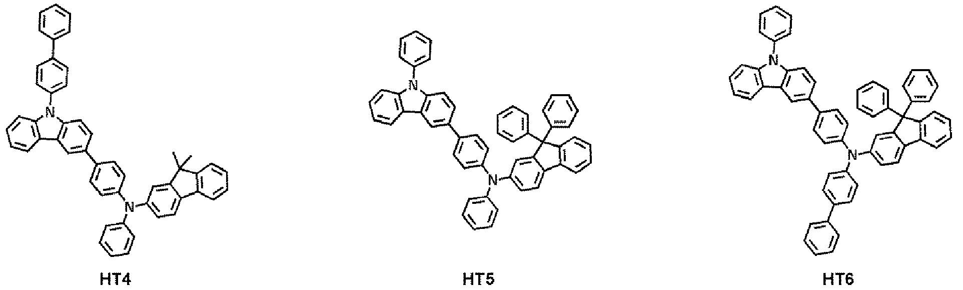

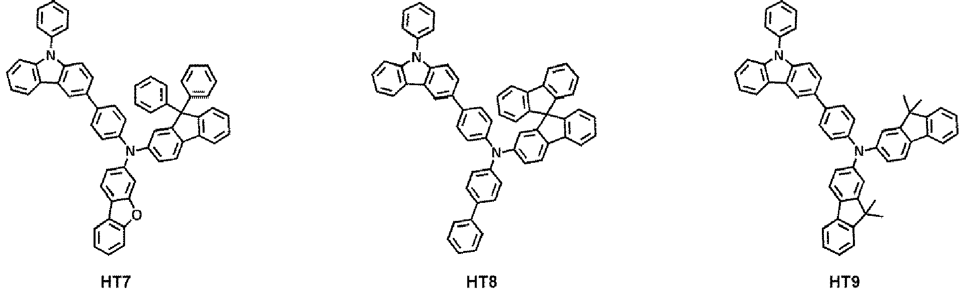

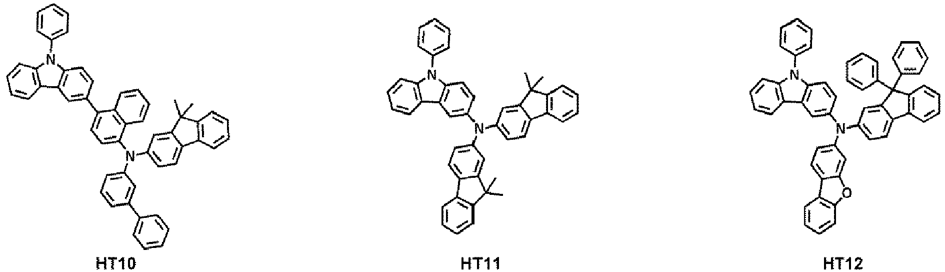

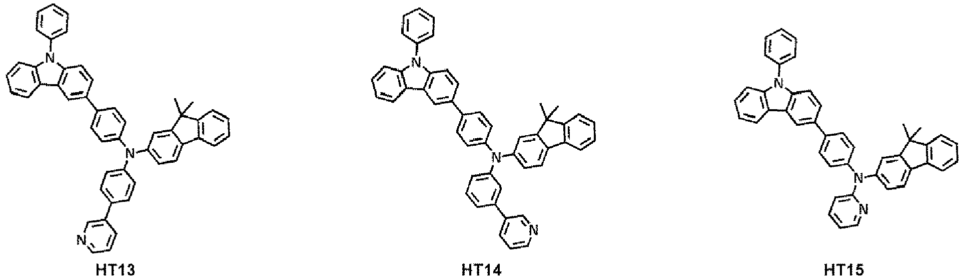

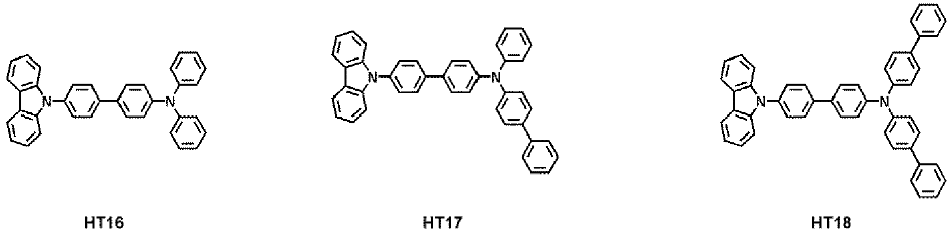

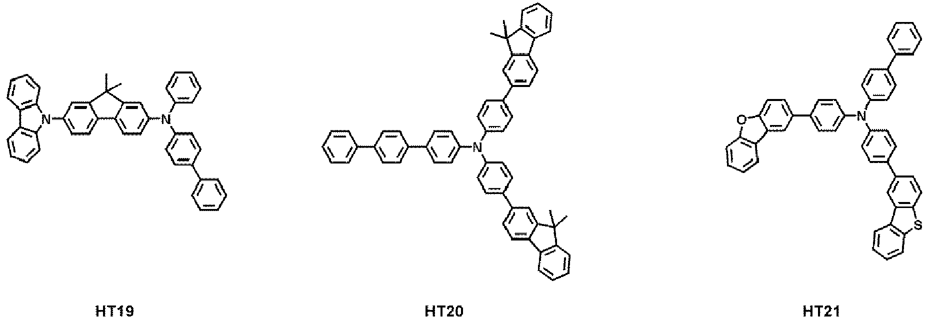

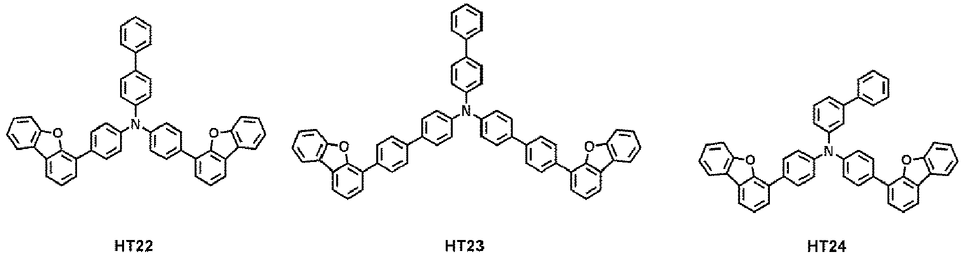

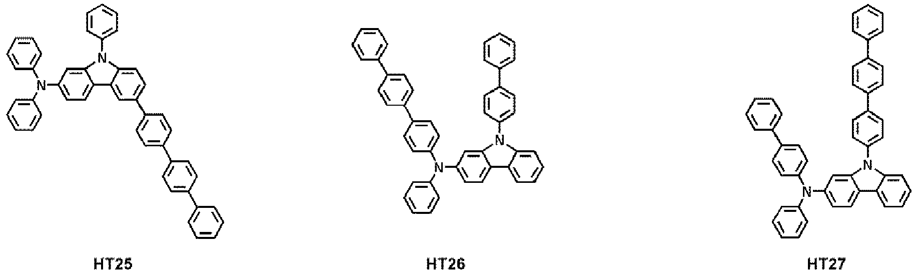

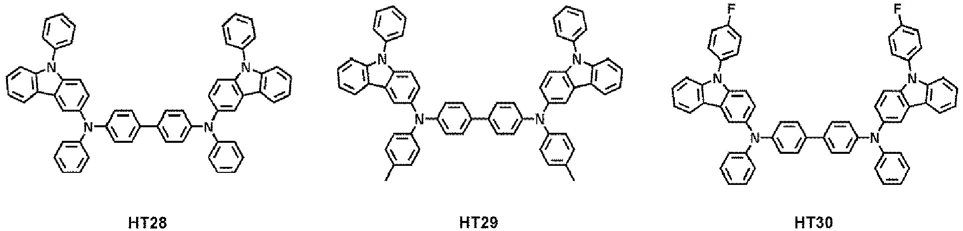

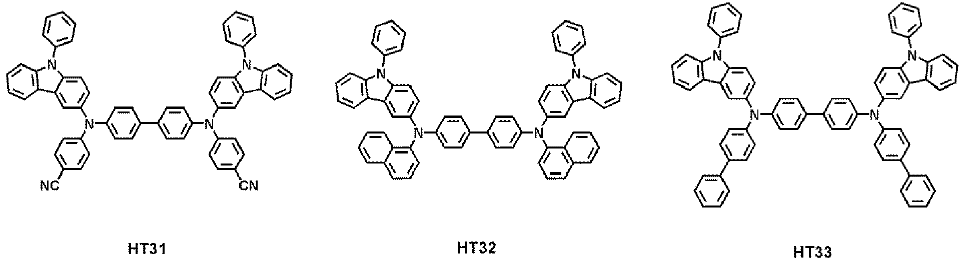

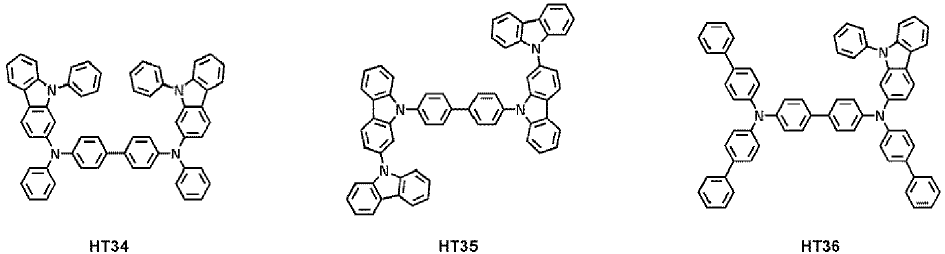

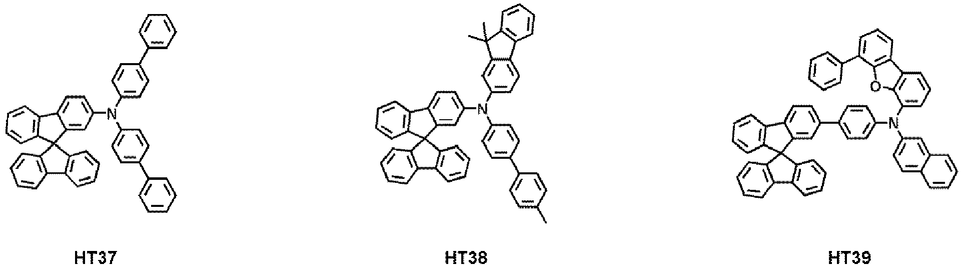

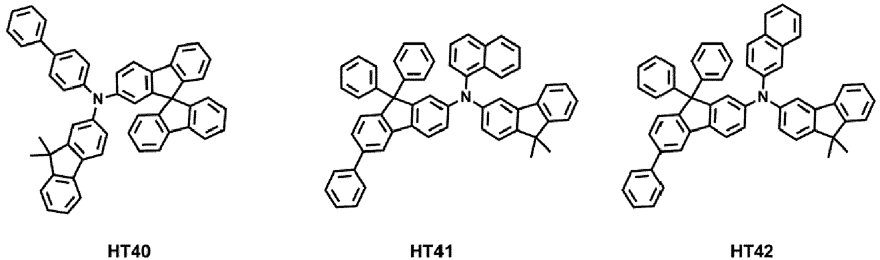

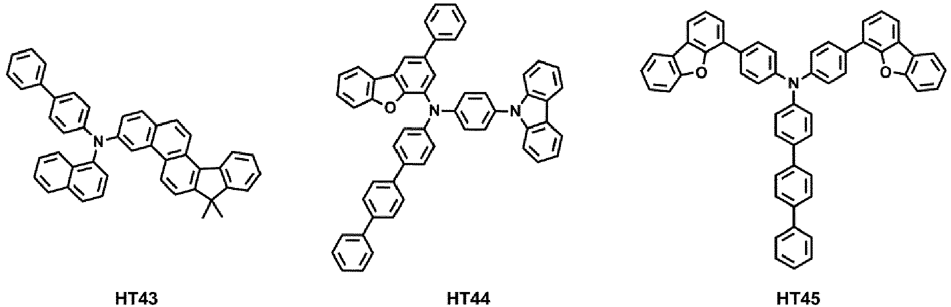

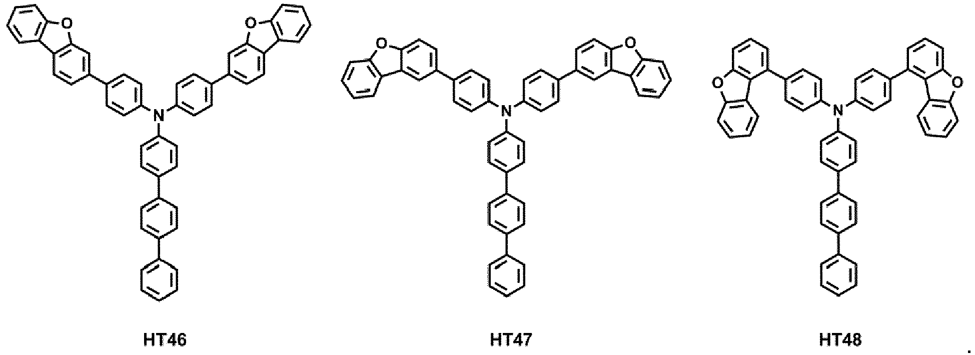

A thickness of the hole transport region may be in a range of about 100 Å to about 10,000 Å, for example, about 100 Å to about 1,000 Å. When the hole transport region comprises at least one selected from a hole injection layer and a hole transport layer, the thickness of the hole injection layer may be in a range of about 100 Å to about 9,000 Å, for example, about 100 Å to about 1,000 Å, and the thickness of the hole transport layer may be in a range of about 50 Å to about 2,000 Å, for example, about 100 Å to about 1,500 Å. When the thicknesses of the hole transport region, the hole injection layer and the hole transport layer are within these ranges, suitable or satisfactory hole transporting characteristics may be obtained without a substantial increase in driving voltage.

The emission auxiliary layer may increase light-emission efficiency by compensating for an optical resonance distance according to the wavelength of light emitted by an emission layer, and the electron blocking layer may block or reduce the flow of electrons from an electron transport region. The emission auxiliary layer and the electron blocking layer may comprise the materials as described above.

### P-dopant

The hole transport region may further comprise, in addition to these materials, a charge-generation material for the improvement of conductive (e.g., electrically conductive) properties. The charge-generation material may be homogeneously or non-homogeneously dispersed in the hole transport region.

The charge-generation material may be, for example, a p-dopant.

In an embodiment, a lowest unoccupied molecular orbital (LUMO) energy level of the p-dopant may be -3.5 eV or less.

The p-dopant may comprise at least one selected from a quinone derivative, a metal oxide, and a cyano group-containing compound, but embodiments of the present disclosure are not limited thereto.

In an embodiment, the p-dopant may comprise at least one selected from:
a quinone derivative, such as tetracyanoquinodimethane (TCNQ) and 2,3,5,6-tetrafluoro-7,7,8,8-tetracyanoquinodimethane (F4-TCNQ);
a metal oxide, such as tungsten oxide and/or molybdenum oxide;
1,4,5,8,9,12-hexaazatriphenylene-hexacarbonitrile (HAT-CN); and
a compound represented by Formula 221 below,
but embodiments of the present disclosure are not limited thereto: 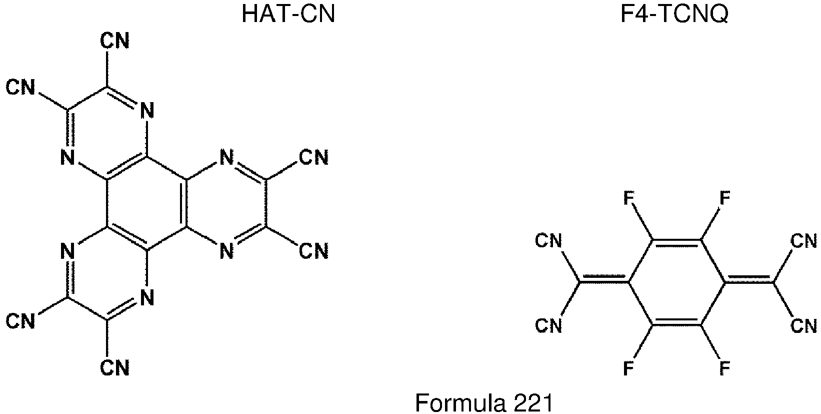 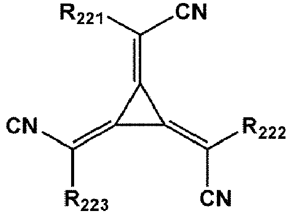 wherein, in Formula 221,
R₂₂₁ to R₂₂₃ may each independently be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group, and at least one selected from R₂₂₁ to R₂₂₃ may have at least one substituent selected from a cyano group, -F, -Cl, -Br, -I, a C₁-C₂₀ alkyl group substituted with -F, a C₁-C₂₀ alkyl group substituted with -Cl, a C₁-C₂₀ alkyl group substituted with -Br, and a C₁-C₂₀ alkyl group substituted with -I.

### Emission layer in organic layer 150

When the organic light-emitting device 10 is a full-color organic light-emitting device, the emission layer may be patterned into a red emission layer, a green emission layer, or a blue emission layer, according to a sub-pixel. In one or more embodiments, the emission layer may have a stacked structure of two or more layers selected from a red emission layer, a green emission layer, and a blue emission layer, in which the two or more layers contact (e.g., physically contact) each other or are separated (e.g., spaced apart) from each other. In one or more embodiments, the emission layer may comprise two or more materials selected from a red light-emitting material, a green light-emitting material, and a blue light-emitting material, in which the two or more materials are mixed together with each other in a single layer to emit white light.

The emission layer may comprise a host and a luminescent material. The luminescent material may comprise at least one selected from a phosphorescent dopant, a fluorescent dopant, and a quantum dot.

An amount of a dopant in the emission layer may be, based on about 100 parts by weight of the host, in the range of about 0.01 to about 15 parts by weight, but embodiments of the present disclosure are not limited thereto.

A thickness of the emission layer may be in a range of about 100 Å to about 1,000 Å, for example, about 200 Å to about 600 Å. When the thickness of the emission layer is within any of the foregoing ranges, excellent light-emission characteristics may be obtained without a substantial increase in driving voltage.

### Host in emission layer

The host may comprise the amine compound represented by Formula 1.

The host may further comprise a compound represented by Formula 301. Formula 301

[Ar₃₀₁]_{xb11}-[(L₃₀₁)_{xb1}-R₃₀₁]_{xb21}

wherein, in Formula 301,
Ar₃₀₁ may be a substituted or unsubstituted C₅-C₆₀ carbocyclic group, or a substituted or unsubstituted C₁-C₆₀ heterocyclic group,
xb11 may be 1, 2, or 3,
L₃₀₁ may be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
xb1 may be an integer from 0 to 5,
R₃₀₁ may be selected from hydrogen, deuterium, -F, -Cl, -Br, -I, a hydroxyl group, a cyano group, a nitro group, an amidino group, a hydrazino group, a hydrazono group, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, - Si(Q₃₀₁)(Q₃₀₂)(Q₃₀₃), -N(Q₃₀₁)(Q₃₀₂), -B(Q₃₀₁)(Q₃₀₂), -C(=O)(Q₃₀₁), -S(=O)₂(Q₃₀₁), and - P(=O)(Q₃₀₁)(Q₃₀₂), and
xb21 may be an integer from 1 to 5,
wherein Q₃₀₁ to Q₃₀₃ may each independently be selected from a C₁-C₁₀ alkyl group, a C₁-C₁₀ alkoxy group, a phenyl group, a biphenyl group, a terphenyl group, and a naphthyl group, but embodiments of the present disclosure are not limited thereto.

In an embodiment, Ar₃₀₁ in Formula 301 may be selected from:
a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, and a dibenzothiophene group; and
a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, and a dibenzothiophene group, each substituted with at least one selected from deuterium, - F, -CI, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, - Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and - P(=O)(Q₃₁)(Q₃₂),
wherein Q₃₁ to Q₃₃ may each independently be selected from a C₁-C₁₀ alkyl group, a C₁-C₁₀ alkoxy group, a phenyl group, a biphenyl group, a terphenyl group, and a naphthyl group, but embodiments of the present disclosure are not limited thereto.

When xb11 in Formula 301 is two or more, two or more of Ar₃₀₁(S) may be linked via a single bond.

In one or more embodiments, the compound represented by Formula 301 may be represented by one of Formula 301-1 and Formula 301-2:

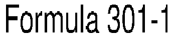

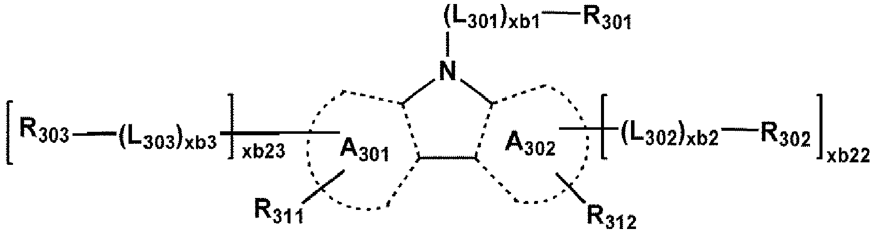

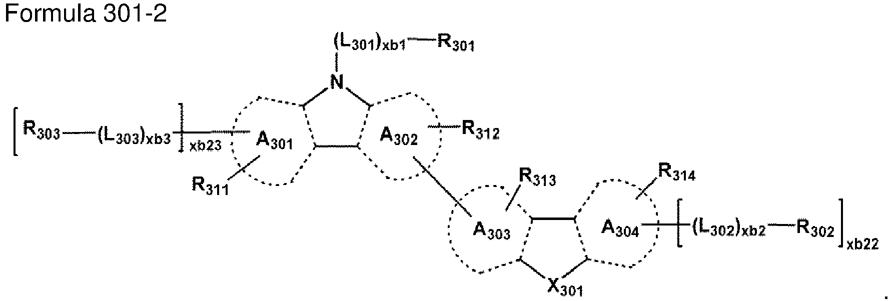

In Formulae 301-1 and 301-2
A₃₀₁ to A₃₀₄ may each independently be selected from a benzene ring, a naphthalene ring, a phenanthrene ring, a fluoranthene ring, a triphenylene ring, a pyrene ring, a chrysene ring, a pyridine ring, a pyrimidine ring, an indene ring, a fluorene ring, a spiro-bifluorene ring, a benzofluorene ring, a dibenzofluorene ring, an indole ring, a carbazole ring, a benzocarbazole ring, a dibenzocarbazole ring, a furan ring, a benzofuran ring, a dibenzofuran ring, a naphthofuran ring, a benzonaphthofuran ring, a dinaphthofuran ring, a thiophene ring, a benzothiophene ring, a dibenzothiophene ring, a naphthothiophene ring, a benzonaphthothiophene ring, and a dinaphthothiophene ring,
X₃₀₁ may be O, S, or N-[(L₃₀₄)_{xb4}-R₃₀₄],
R₃₁₁ to R₃₁₄ may each independently be selected from hydrogen, deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group -Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and -P(=O)(Q₃₁)(Q₃₂),
xb22 and xb23 may each independently be 0, 1, or 2,
L₃₀₁, xb1, R₃₀₁ and Q₃₁ to Q₃₃ are the same as described above,
L₃₀₂ to L₃₀₄ are each independently the same as described in connection with L₃₀₁,
xb2 to xb4 may each independently be the same as described in connection with xb1, and
R₃₀₂ to R₃₀₄ are each independently the same as described in connection with R₃₀₁.

For example, L₃₀₁ to L₃₀₄ in Formulae 301, 301-1, and 301-2 may each independently be selected from:
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, a benzoisoxazolylene group, a triazolylene group, a tetrazolylene group, an imidazopyridinylene group, an imidazopyrimidinylene group, and an azacarbazolylene group; and
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, an isobenzothiazolylene group, a benzoxazolylene group, a benzoisoxazolylene group, a triazolylene group, a tetrazolylene group, an imidazopyridinylene group, an imidazopyrimidinylene group, and an azacarbazolylene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, a benzoisoxazolyl group, a triazolyl group, a tetrazolyl group, an imidazopyridinyl group, an imidazopyrimidinyl group, an azacarbazolyl group, -Si(Q₃₁)(Q₃₂)(Q₃₃), -N(Q₃₁)(Q₃₂), - B(Q₃₁)(Q₃₂), -C(=O)(Q₃₁), -S(=O)₂(Q₃₁), and -P(=O)(Q₃₁)(Q₃₂),
wherein Q₃₁ to Q₃₃ are the same as described above.

In an embodiment, R₃₀₁ to R₃₀₄ in Formulae 301, 301-1, and 301-2 may each independently be selected from:
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group; and
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, an azacarbazolyl group, -Si(Q₃₁)(Q₃₂)(Q₃₃), -N(Q₃₁)(Q₃₂), - B(Q₃₁)(Q₃₂), -C(=O)(Q₃₁), -S(=O)₂(Q₃₁), and -P(=O)(Q₃₁)(Q₃₂),
wherein Q₃₁ to Q₃₃ are the same as described above.

In one or more embodiments, the host may comprise an alkaline earth metal complex. For example, the host may be selected from a Be complex (for example, Compound H55), an Mg complex, and a Zn complex.

The host may comprise at least one selected from 9,10-di(2-naphthyl)anthracene (ADN), 2-methyl-9,10-bis(naphthalen-2-yl)anthracene (MADN), 9,10-di-(2-naphthyl)-2-t-butyl-anthracene (TBADN), 4,4'-bis(N-carbazolyl)-1,1'-biphenyl (CBP), 1,3-di-9-carbazolylbenzene (mCP), 1,3,5-tri(carbazol-9-yl)benzene (TCP), and at least one selected from Compounds H1 to H55, but embodiments of the present disclosure are not limited thereto:

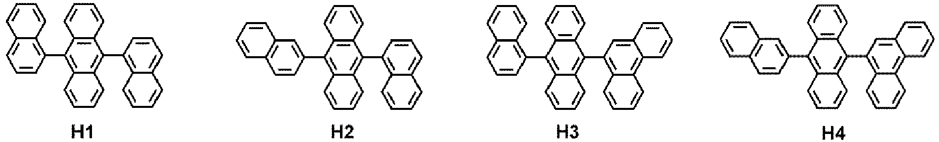

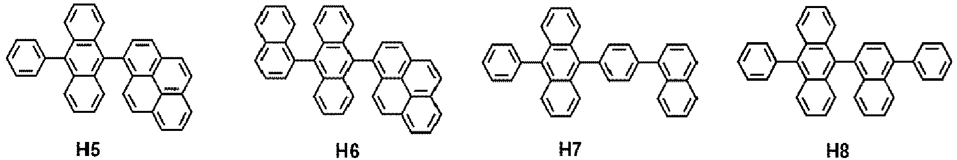

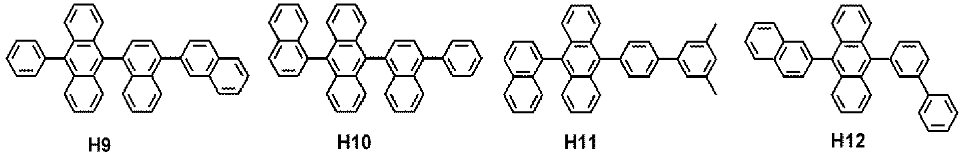

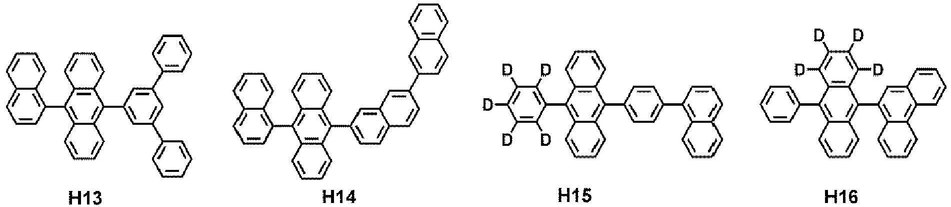

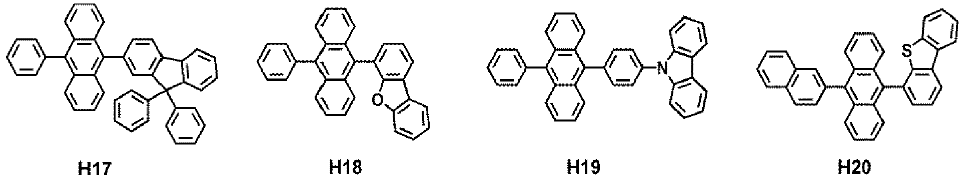

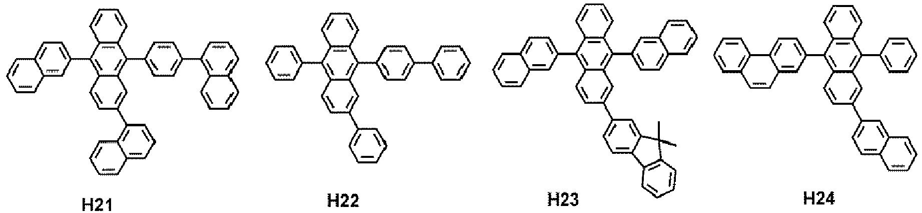

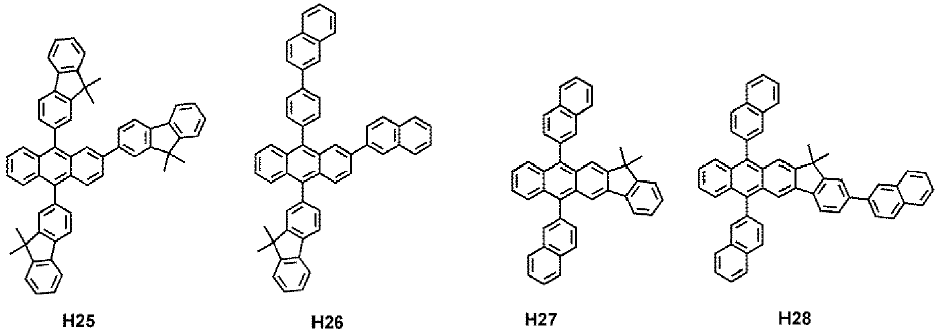

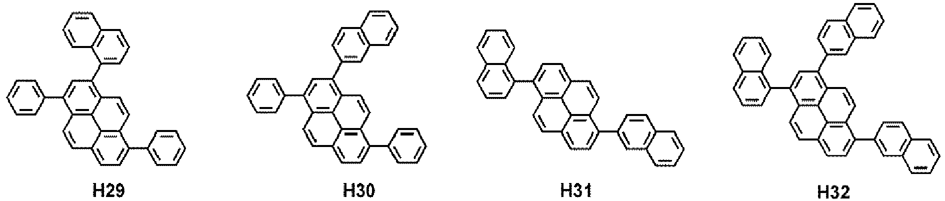

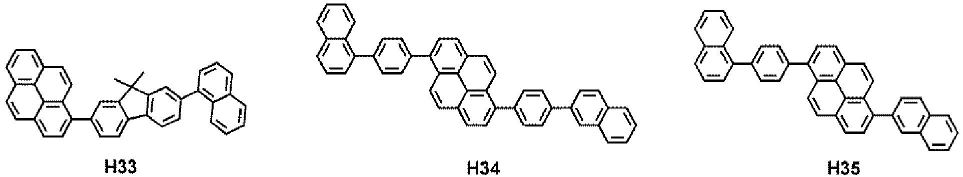

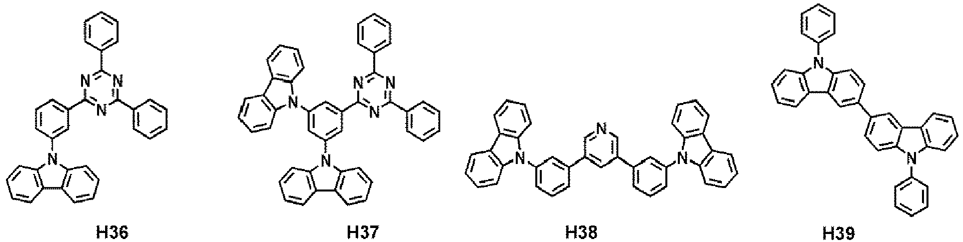

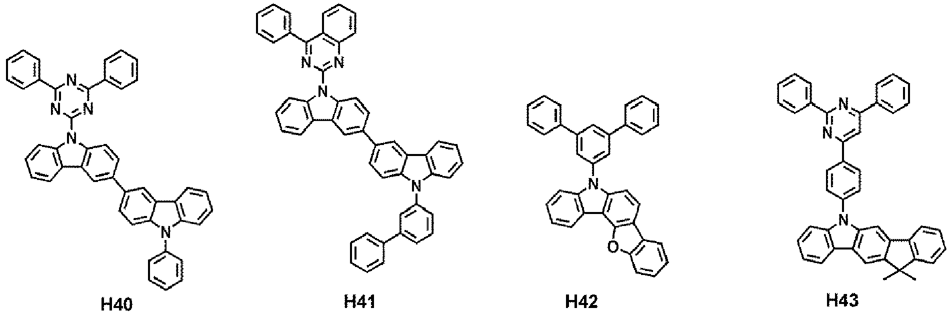

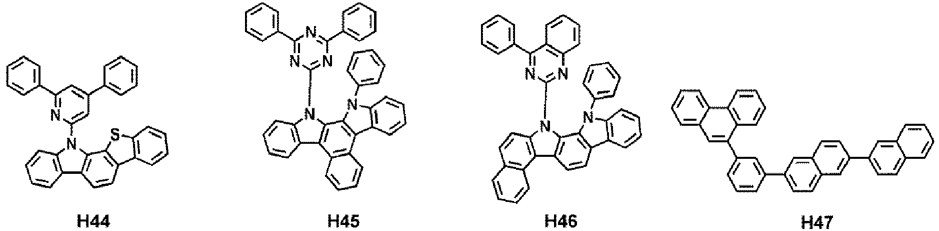

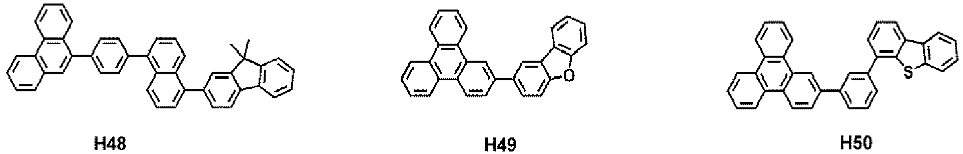

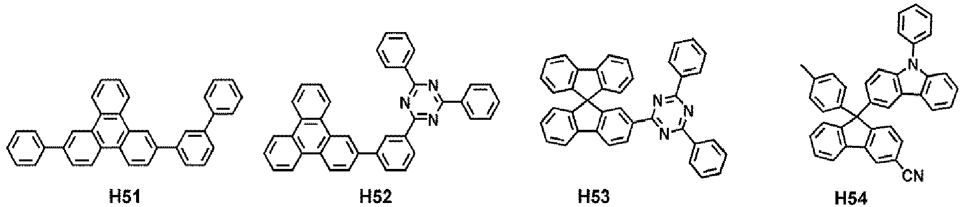

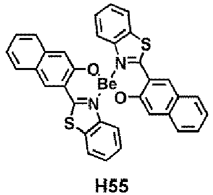

### Phosphorescent dopant comprised in emission layer in organic layer 150

The phosphorescent dopant may comprise an organometallic complex represented by Formula 401 below:

Formula 401 M(L₄₀₁)_{xc1}(L₄₀₂)_{xc2}

wherein, in Formulae 401,
M may be selected from iridium (Ir), platinum (Pt), palladium (Pd), osmium (Os), titanium (Ti), zirconium (Zr), hafnium (Hf), europium (Eu), terbium (Tb), rhodium (Rh), and thulium (Tm),
L₄₀₁ may be a ligand represented by Formula 402, and xc1 may be 1, 2, or 3, wherein when xc1 is two or more, two or more of L₄₀₁(s) may be identical to or different from each other,
L₄₀₂ may be an organic ligand, and xc2 may be an integer from 0 to 4, wherein when xc2 is two or more, two or more of L₄₀₂(s) may be identical to or different from each other, 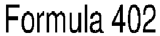 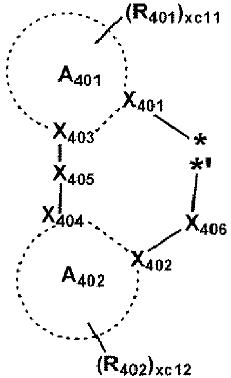
wherein, in Formulae 402,
X₄₀₁ to X₄₀₄ may each independently be nitrogen or carbon,
X₄₀₁ and X₄₀₃ may be linked via a single bond or a double bond, and X₄₀₂ and X₄₀₄ may be linked via a single bond or a double bond,
A₄₀₁ and A₄₀₂ may each independently be a C₅-C₆₀ carbocyclic group, or a C1-C60 heterocyclic group,
X₄₀₅ may be a single bond, *-O-*', *-S-*', *-C(=O)-*', *-N(Q₄₁₁)-*', *-C(Q₄₁₁)(Q₄₁₂)-*', *-C(Q₄₁₁)=C(Q₄₁₂)-*', *-C(Q₄₁₁)=*' or *=C=*', wherein Q₄₁₁ and Q₄₁₂ may be hydrogen, deuterium, a C₁-C₂₀ alkyl group, a C₁-C₂₀ alkoxy group, a phenyl group, a biphenyl group, a terphenyl group, or a naphthyl group,
X₄₀₆ may be a single bond, O, or S,
R₄₀₁ and R₄₀₂ may each independently be selected from hydrogen, deuterium, -F, -CI, -Br, -I, a hydroxyl group, a cyano group, a nitro group, an amidino group, a hydrazino group, a hydrazono group, a substituted or unsubstituted C₁-C₂₀ alkyl group, a substituted or unsubstituted C₁-C₂₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group, -Si(Q₄₀₁)(Q₄₀₂)(Q₄₀₃), -N(Q₄₀₁)(Q₄₀₂), -B(Q₄₀₁)(Q₄₀₂), -C(=O)(Q₄₀₁), -S(=O)₂(Q₄₀₁), and -P(=O)(Q₄₀₁)(Q₄₀₂), and Q₄₀₁ to Q₄₀₃ may each independently be selected from a C₁-C₁₀ alkyl group, a C₁-C₁₀ alkoxy group, a C₆-C₂₀ aryl group, and a C₁-C₂₀ heteroaryl group,
xc11 and xc12 may each independently be an integer from 0 to 10, and
* and *' in Formula 402 each indicate a binding site to M in Formula 401.

In an embodiment, A₄₀₁ and A₄₀₂ in Formula 402 may each independently be selected from a benzene group, a naphthalene group, a fluorene group, a spiro-bifluorene group, an indene group, a pyrrole group, a thiophene group, a furan group, an imidazole group, a pyrazole group, a thiazole group, an isothiazole group, an oxazole group, an isoxazole group, a pyridine group, a pyrazine group, a pyrimidine group, a pyridazine group, a quinoline group, an isoquinoline group, a benzoquinoline group, a quinoxaline group, a quinazoline group, a carbazole group, a benzimidazole group, a benzofuran group, a benzothiophene group, an isobenzothiophene group, a benzoxazole group, an isobenzoxazole group, a triazole group, a tetrazole group, an oxadiazole group, a triazine group, a dibenzofuran group, and a dibenzothiophene group.

In one or more embodiments, in Formula 402, i) X₄₀₁ may be nitrogen and X₄₀₂ may be carbon, or ii) X₄₀₁ and X₄₀₂ are each nitrogen at the same time.

In one or more embodiments, R₄₀₁ and R₄₀₂ in Formula 402 may each independently be selected from:
hydrogen, deuterium, -F, -Cl, -Br, -I, a hydroxyl group, a cyano group, a nitro group, an amidino group, a hydrazino group, a hydrazono group, a C₁-C₂₀ alkyl group, and a C₁-C₂₀ alkoxy group;
a C₁-C₂₀ alkyl group, and a C₁-C₂₀ alkoxy group, each substituted with at least one selected from deuterium, -F, -Cl, -Br, -I, a hydroxyl group, a cyano group, a nitro group, an amidino group, a hydrazino group, a hydrazono group, a phenyl group, a naphthyl group, a cyclopentyl group, a cyclohexyl group, an adamantly group, a norbornanyl group, and a norbornenyl group;
a cyclopentyl group, a cyclohexyl group, an adamantly group, a norbornanyl group, a norbornenyl group, a phenyl group, a biphenyl group, a terphenyl group, a naphthyl group, a fluorenyl group, a pyridinyl group, a pyrazinyl group, a pyrimidinyl group, a pyridazinyl group, a triazinyl group, a quinolinyl group, an isoquinolinyl group, a quinoxalinyl group, a quinazolinyl group, a carbazolyl group, a dibenzofuranyl group, and a dibenzothiophenyl group;
a cyclopentyl group, a cyclohexyl group, an adamantly group, a norbornanyl group, a norbornenyl group a phenyl group, a biphenyl group, a terphenyl group, a naphthyl group, a fluorenyl group, a pyridinyl group, a pyrazinyl group, a pyrimidinyl group, a pyridazinyl group, a triazinyl group, a quinolinyl group, an isoquinolinyl group, a quinoxalinyl group, a quinazolinyl group, a carbazolyl group, a dibenzofuranyl group, and a dibenzothiophenyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, an adamantly group, a norbornanyl group, a norbornenyl group, a phenyl group, a biphenyl group, a terphenyl group, a naphthyl group, a fluorenyl group, a pyridinyl group, a pyrazinyl group, a pyrimidinyl group, a pyridazinyl group, a triazinyl group, a quinolinyl group, an isoquinolinyl group, a quinoxalinyl group, a quinazolinyl group, a carbazolyl group, a dibenzofuranyl group, and a dibenzothiophenyl group; and
-Si(Q₄₀₁)(Q₄₀₂)(Q₄₀₃), -N(Q₄₀₁)(Q₄₀₂), -B(Q₄₀₁)(Q₄₀₂), -C(=O)(Q₄₀₁), - S(=O)₂(Q₄₀₁), and -P(=O)(Q₄₀₁)(Q₄₀₂),
wherein Q₄₀₁ to Q₄₀₃ may each independently be selected from a C₁-C₁₀ alkyl group, a C₁-C₁₀ alkoxy group, a phenyl group, a biphenyl group, and a naphthyl group, but embodiments of the present disclosure are not limited thereto.

In one or more embodiments, when xc1 in Formula 401 is two or more, two A₄₀₁(s) in two or more L₄₀₁(s) may optionally be linked to each other via X₄₀₇, which is a linking group, two A₄₀₂(s) may optionally be linked to each other via X₄₀₈, which is a linking group (see Compounds PD1 to PD4 and PD7). X₄₀₇ and X₄₀₈ may each independently be a single bond, *-O-*', *-S-*', *-C(=O)-*', *-N(Q₄₁₃)-*', *-C(Q₄₁₃)(Q₄₁₄)-*' or *-C(Q₄₁₃)=C(Q₄₁₄)-*' (where Q₄₁₃ and Q₄₁₄ may each independently be hydrogen, deuterium, a C₁-C₂₀ alkyl group, a C₁-C₂₀ alkoxy group, a phenyl group, a biphenyl group, a terphenyl group, or a naphthyl group), but embodiments of the present disclosure are not limited thereto.

L₄₀₂ in Formula 401 may be a monovalent, divalent, or trivalent organic ligand. For example, L₄₀₂ may be selected from halogen, diketone (for example, acetylacetonate), carboxylic acid (for example, picolinate), -C(=O), isonitrile, -CN, and phosphorus (for example, phosphine, and/or phosphite), but embodiments of the present disclosure are not limited thereto.

In one or more embodiments, the phosphorescent dopant may be selected from, for example, Compounds PD1 to PD25, but embodiments of the present disclosure are not limited thereto:

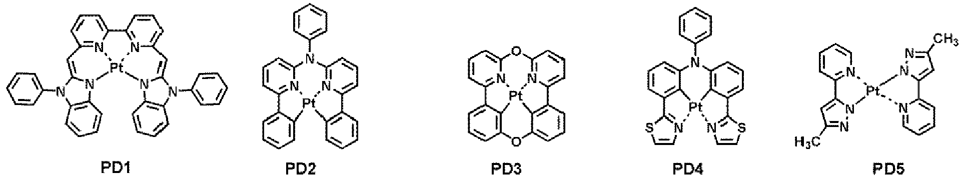

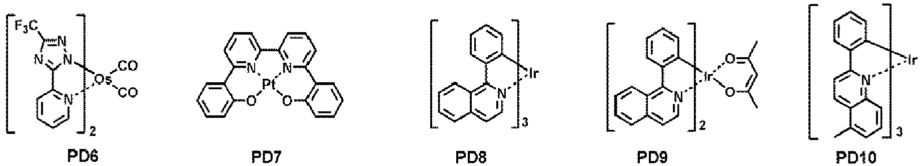

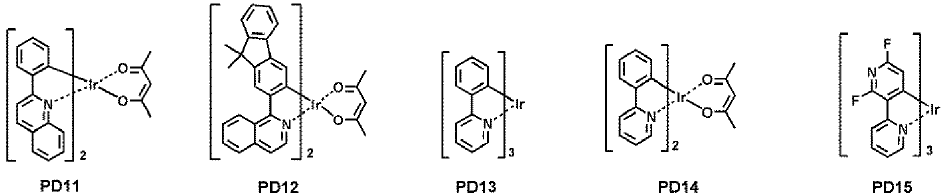

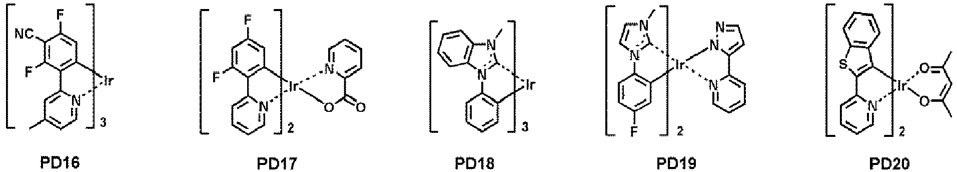

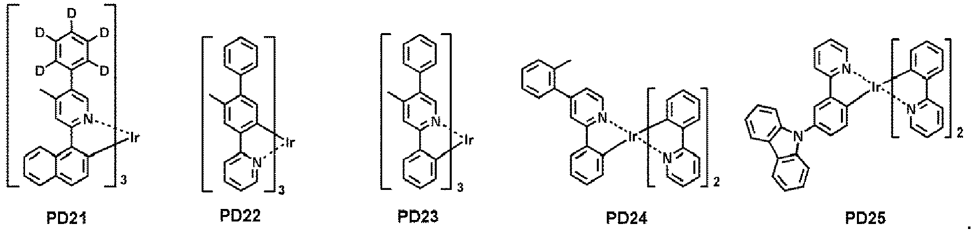

### Fluorescent dopant in emission layer

The fluorescent dopant may comprise the amine compound represented by Formula 1.

The fluorescent dopant may comprise an arylamine compound and/or a styrylamine compound.

The fluorescent dopant may comprise a compound represented by Formula 501 below: 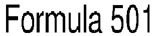 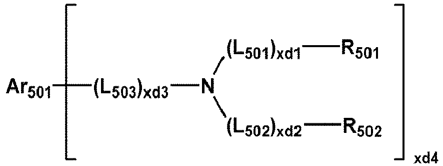 wherein, in Formula 501,
Ar₅₀₁ may be a substituted or unsubstituted C₅-C₆₀ carbocyclic group, or a substituted or unsubstituted C₁-C₆₀ heterocyclic group,
L₅₀₁ to L₅₀₃ may each independently be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
xd1 to xd3 may each independently be an integer from 0 to 3,
R₅₀₁ and R₅₀₂ may each independently be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, and a substituted or unsubstituted monovalent non-aromatic condensed heteropolycyclic group, and
xd4 may be an integer from 1 to 6.

In an embodiment, Ar₅₀₁ in Formula 501 may be selected from:
a naphthalene group, a heptalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, and an indenophenanthrene group; and
a naphthalene group, a heptalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, and an indenophenanthrene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, and a naphthyl group.

In one or more embodiments, L₅₀₁ to L₅₀₃ in Formula 501 may each independently be selected from:
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group; and
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, and a pyridinylene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group.

In one or more embodiments, R₅₀₁ and R₅₀₂ in Formula 501 may each independently be selected from:
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group; and
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, and a pyridinyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, and - Si(Q₃₁)(Q₃₂)(Q₃₃),
wherein Q₃₁ to Q₃₃ may be selected from a C₁-C₁₀ alkyl group, a C₁-C₁₀ alkoxy group, a phenyl group, a biphenyl group, a terphenyl group, and a naphthyl group.

In one or more embodiments, xd4 in Formula 501 may be 2, but embodiments of the present disclosure are not limited thereto.

For example, the fluorescent dopant may be selected from Compounds FD1 to FD22:

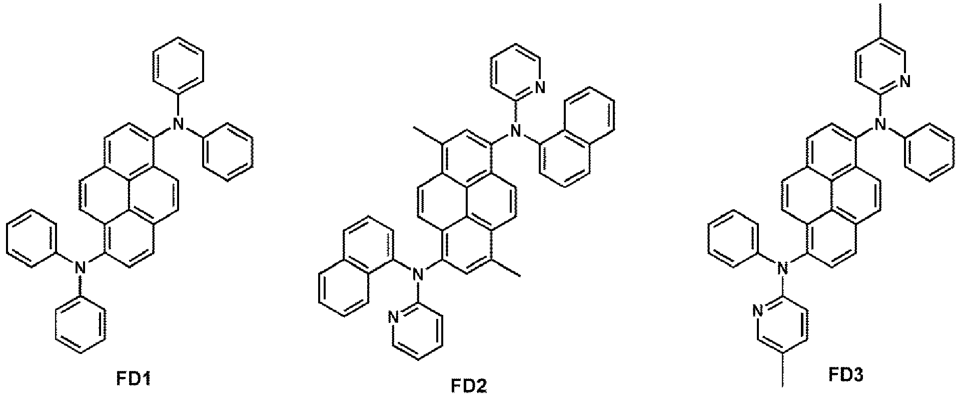

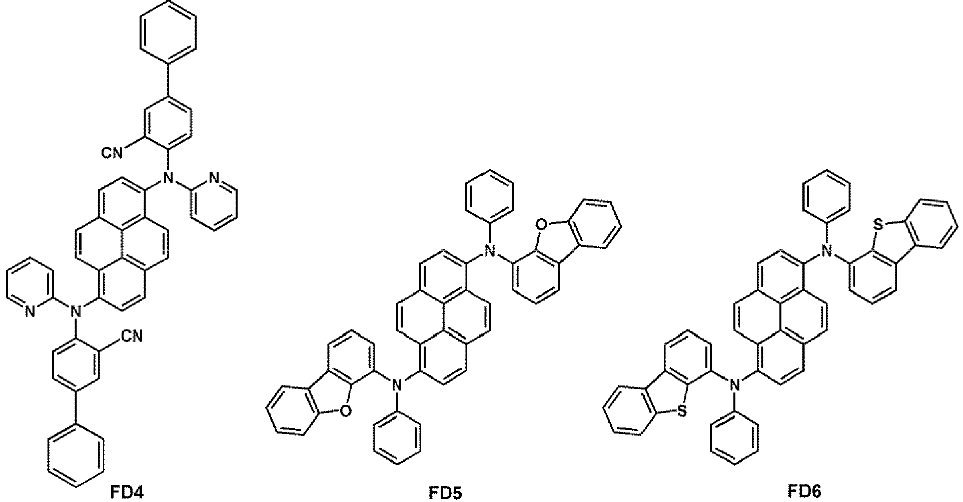

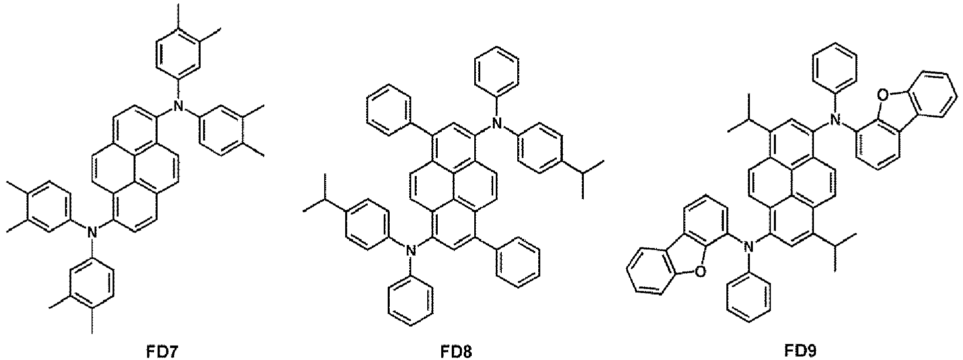

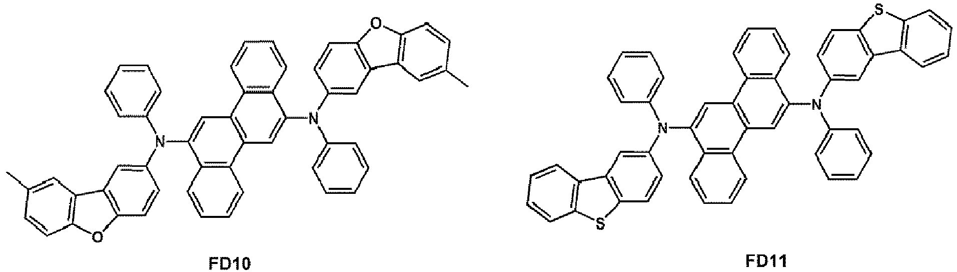

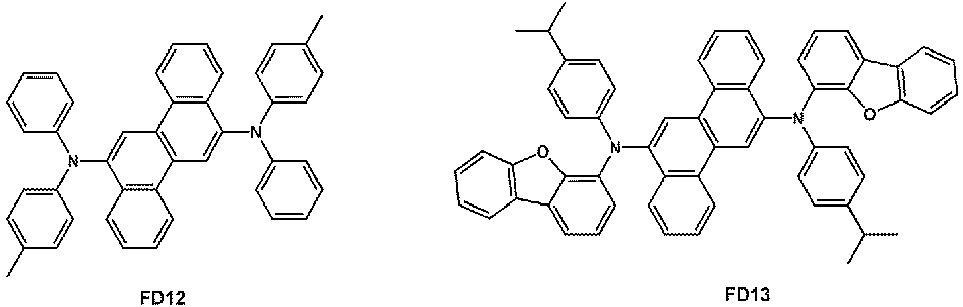

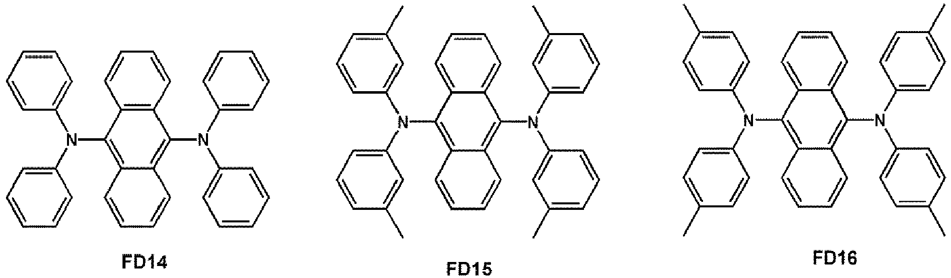

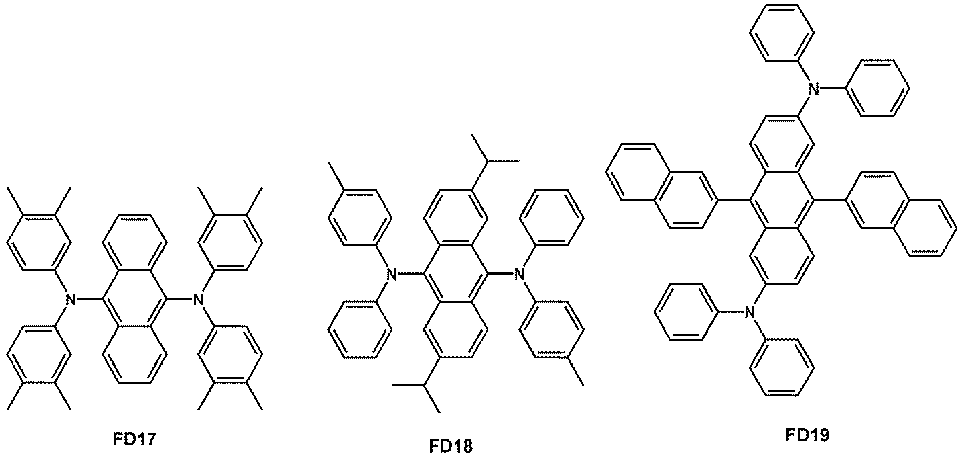

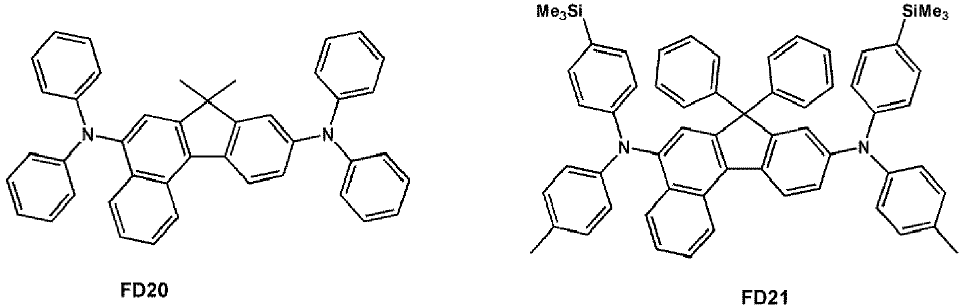

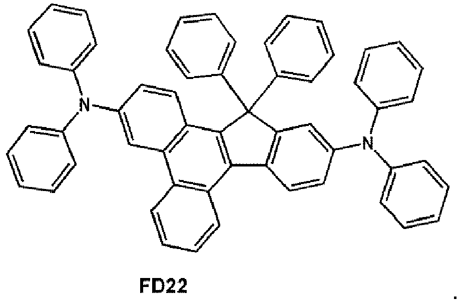

In one or more embodiments, the fluorescent dopant may be selected from the following compounds, but embodiments of the present disclosure are not limited thereto.

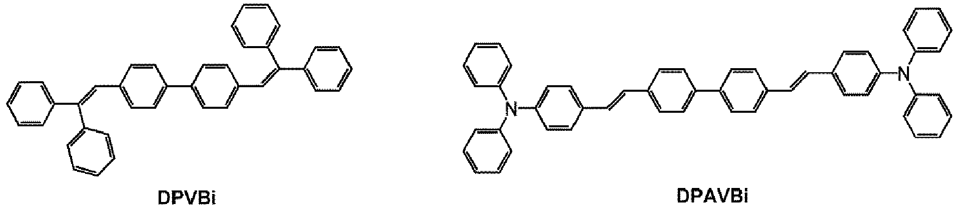

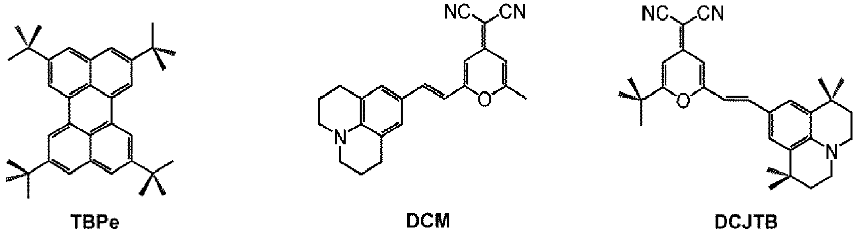

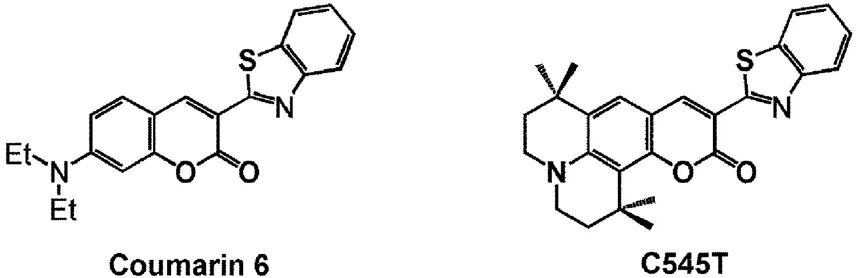

### Quantum dot

An emission layer comprised in an organic light-emitting device of the present disclosure may comprise a quantum dot.

The quantum dot is a particle having a crystal structure of several to several tens of nanometers, and comprises hundreds to thousands of atoms.

Because the quantum dot is very small in size, a quantum confinement effect may occur. The quantum confinement effect refers to a phenomenon in which a band gap of an object becomes large when the object becomes smaller than a nanometer size (e.g., has a size in a nanometer scale and greater than 1 nanometer). Accordingly, when light having a wavelength having an energy intensity that is greater than the band gap of the quantum dot is irradiated to the quantum dot, the quantum dot is excited by absorbing the light and then emits light having a set or specific wavelength as it transits (e.g., transitions or relaxes) to the ground state. In this case, the wavelength of the emitted light has a value corresponding to the band gap.

The core of the quantum dot may comprise a Group II-VI compound, a Group III-VI compound, a Group III-V compound, a Group IV-VI compound, a Group IV element or compound, a Group I-III-VI compound, or a combination thereof.

The Group II-VI compound may be selected from a binary compound selected from CdS, CdSe, CdTe, ZnS, ZnSe, ZnTe, ZnO, HgS, HgSe, HgTe, MgSe, MgS, and any mixture thereof; a ternary compound selected from CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, MgZnS, and any mixture thereof; and a quaternary compound selected from CdZnSeS, CdZnSeTe, CdZnSTe, CdHgSeS, CdHgSeTe, CdHgSTe, HgZnSeS, HgZnSeTe, HgZnSTe, and any mixture thereof.

The Group III-VI compound may comprise: a binary compound, such as In₂S₃ and/or In₂Se; a ternary compound, such as InGaS₃ or InGaSe₃; or any combination thereof.

For example, the Group III-V compound may be selected from: a binary compound selected from GaN, GaP, GaAs, GaSb, AIN, AlP, AlAs, AlSb, InN, InP, InAs, InSb, and any mixture thereof; a ternary compound selected from GaNP, GaNAs, GaNSb, GaPAs, GaPSb, AINP, AINAs, AINSb, AlPAs, AlPSb, InGaP, InAlP, InNP, InNAs, InNSb, InPAs, InPSb, GaAINP, and any mixture thereof; and a quaternary compound selected from GaAlNAs, GaAlNSb, GaAlPAs, GaAlPSb, GaInNP, GaInNAs, GaInNSb, GaInPAs, GaInPSb, InAlNP, InAlNAs, InAlNSb, InAlPAs, InAlPSb, and any mixture thereof, but embodiments of the present disclosure are not limited thereto. The Group III-V semiconductor compound may further comprise a Group II metal (for example, InZnP, etc.).

The Group IV-VI group compound may be selected from: a binary compound selected from SnS, SnSe, SnTe, PbS, PbSe, PbTe, and any mixture thereof; a ternary compound selected from SnSeS, SnSeTe, SnSTe, PbSeS, PbSeTe, PbSTe, SnPbS, SnPbSe, SnPbTe, and any mixture thereof; and a quaternary compound selected from SnPbSSe, SnPbSeTe, SnPbSTe, and any mixture thereof. The IV group element may be selected from Si, Ge, and any mixture thereof. The IV group compound may be a binary compound selected from SiC, SiGe, and any mixture thereof.

The binary compound, the ternary compound, or the quaternary compound may exist in particles at uniform (e.g., substantially uniform) concentration, or may exist in the same particle in a state in which a concentration distribution is partially different. In addition, the binary compound, the ternary compound, and/or the quaternary compound may have a core-shell structure in which one quantum dot surrounds another quantum dot. An interface between the core and the shell may have a concentration gradient in which the concentration of elements existing in the shell decreases along a direction toward the center.

In one or more embodiments, the quantum dot may have a core-shell structure comprising a core with the above-described nanoparticles and a shell surrounding the core. The shell of the quantum dot may function as a protective layer for maintaining semiconductor characteristics by preventing or reducing chemical degeneration of the core and/or may function as a charging layer for imparting electrophoretic characteristics to the quantum dot. The shell may be a single layer or a multilayer. An interface between the core and the shell may have a concentration gradient in which the concentration of elements existing in the shell decreases along a direction toward the center. Examples of the shell of the quantum dot are a metal or non-metal oxide, a semiconductor compound, or any combination thereof.

Examples of the metal or non-metal oxide are a binary compound such as SiO₂, Al₂O₃, TiO₂, ZnO, MnO, Mn₂O₃, Mn₃O₄, CuO, FeO, Fe₂O₃, Fe₃O₄, CoO, Co₃O₄, and NiO, and/or a ternary compound such as MgAl₂O₄, CoFe₂O₄, NiFe₂O₄, and/or CoMn₂O₄, but embodiments of the present disclosure are not limited thereto.

In addition, examples of the semiconductor compound are CdS, CdSe, CdTe, ZnS, ZnSe, ZnTe, ZnSeS, ZnTeS, GaAs, GaP, GaSb, HgS, HgSe, HgTe, InAs, InP, InGaP, InSb, AlAs, AlP, AlSb, and the like, but embodiments of the present disclosure are not limited thereto.

A full width of half maximum (FWHM) of an emission wavelength spectrum of the quantum dot may be about 45 nm or less, for example, about 40 nm or less, for example, about 30 nm or less. In addition, light emitted through such quantum dots is irradiated in omnidirection (e.g., in substantially every direction). Accordingly, a wide viewing angle may be increased.

The shape of the quantum dot is not limited as long as the shape is generally used in the art. In one or more embodiments, the quantum dot may comprise spherical, pyramidal, multi-arm, and/or cubic nanoparticles, a nanotube, a nanowire, a nanofiber, a nano-plate particle, and/or the like.

The quantum dot may control the color of light emitted according to the particle size, and accordingly, the quantum dot may have various suitable emission colors such as blue, red, and green.

### Electron transport region in organic layer 150

The electron transport region may have i) a single-layered structure comprising a single layer comprising a single material, ii) a single-layered structure comprising a single layer comprising a plurality of different materials, or iii) a multi-layered structure having a plurality of layers comprising a plurality of different materials.

The electron transport region may comprise at least one layer selected from a buffer layer, a hole blocking layer, an electron control layer, an electron transport layer, and an electron injection layer, but embodiments of the present disclosure are not limited thereto.

For example, the electron transport region may have an electron transport layer/electron injection layer structure, a hole blocking layer/electron transport layer/electron injection layer structure, an electron control layer/electron transport layer/electron injection layer structure, or a buffer layer/electron transport layer/electron injection layer structure, wherein for each structure, constituting layers are sequentially stacked from an emission layer. However, embodiments of the structure of the electron transport region are not limited thereto.

The electron transport region (for example, a buffer layer, a hole blocking layer, an electron control layer, or an electron transport layer in the electron transport region) may comprise a metal-free compound containing at least one π electron-depleted nitrogen-containing ring.

The term "π electron-depleted nitrogen-containing ring," as used herein, indicates a C₁-C₆₀ heterocyclic group having at least one *-N=*' moiety as a ring-forming moiety.

For example, the "π electron-depleted nitrogen-containing ring" may be i) a 5-membered to 7-membered heteromonocyclic group having at least one *-N=*' moiety, ii) a heteropolycyclic group in which two or more 5-membered to 7-membered heteromonocyclic groups each having at least one *-N=*' moiety are condensed with each other (e.g., combined together with each other), or iii) a heteropolycyclic group in which at least one of 5-membered to 7-membered heteromonocyclic groups, each having at least one *-N=*' moiety, is condensed with at least one C₅-C₆₀ carbocyclic group.

Examples of the π electron-deficient nitrogen-containing ring are an imidazole, a pyrazole, a thiazole, an isothiazole, an oxazole, an isoxazole, a pyridine, a pyrazine, a pyrimidine, a pyridazine, an indazole, a purine, a quinoline, an isoquinoline, a benzoquinoline, a phthalazine, a naphthyridine, a quinoxaline, a quinazoline, a cinnoline, a phenanthridine, an acridine, a phenanthroline, a phenazine, a benzimidazole, benzoisothiazole, a benzoxazole, benzoisoxazole, a triazole, a tetrazole, an oxadiazole, a triazine, a thiadiazole, an imidazopyridine, an imidazopyrimidine, and an azacarbazole, but embodiments of the present disclosure are not limited thereto.

For example, the electron transport region may comprise a compound represented by Formula 601 below:

Formula 601 [Ar₆₀₁]ₓₑ₁₁-[(L₆₀₁)ₓₑ₁-R₆₀₁]ₓₑ₂₁

wherein, in Formula 601,
Ar₆₀₁ may be a substituted or unsubstituted C₅-C₆₀ carbocyclic group, or a substituted or unsubstituted C₁-C₆₀ heterocyclic group,
xe11 may be 1, 2, or 3,
L₆₀₁ may be selected from a substituted or unsubstituted C₃-C₁₀ cycloalkylene group, a substituted or unsubstituted C₁-C₁₀ heterocycloalkylene group, a substituted or unsubstituted C₃-C₁₀ cycloalkenylene group, a substituted or unsubstituted C₁-C₁₀ heterocycloalkenylene group, a substituted or unsubstituted C₆-C₆₀ arylene group, a substituted or unsubstituted C₁-C₆₀ heteroarylene group, a substituted or unsubstituted divalent non-aromatic condensed polycyclic group, and a substituted or unsubstituted divalent non-aromatic condensed heteropolycyclic group,
xe1 may be an integer from 0 to 5,
R₆₀₁ may be selected from a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group,-Si(Q₆₀₁)(Q₆₀₂)(Q₆₀₃), -C(=O)(O₆₀₁), -S(=O)₂(Q₆₀₁), and -P(=O)(Q₆₀₁)(Q₆₀₂),
Q₆₀₁ to Q₆₀₃ may each independently be a C₁-C₁₀ alkyl group, a C₁-C₁₀ alkoxy group, a phenyl group, a biphenyl group, a terphenyl group, or a naphthyl group, and
xe21 may be an integer from 1 to 5.

In an embodiment, at least one of Ar₆₀₁ (s) and R₆₀₁(s) may comprise the π electron-deficient nitrogen-containing ring.

In an embodiment, Ar₆₀₁ in Formula 601 may be selected from:
a benzene group, a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, a dibenzothiophene group, a carbazole group, an imidazole group, a pyrazole group, a thiazole group, an isothiazole group, an oxazole group, an isoxazole group, a pyridine group, a pyrazine group, a pyrimidine group, a pyridazine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, a benzoisothiazole group, a benzoxazole group, a benzoisoxazole group, a triazole group, a tetrazole group, an oxadiazole group, a triazine group, a thiadiazole group, an imidazopyridine group, an imidazopyrimidine group, and an azacarbazole group; and
a benzene group, a naphthalene group, a fluorene group, a spiro-bifluorene group, a benzofluorene group, a dibenzofluorene group, a phenalene group, a phenanthrene group, an anthracene group, a fluoranthene group, a triphenylene group, a pyrene group, a chrysene group, a naphthacene group, a picene group, a perylene group, a pentaphene group, an indenoanthracene group, a dibenzofuran group, a dibenzothiophene group, a carbazole group, an imidazole group, a pyrazole group, a thiazole group, an isothiazole group, an oxazole group, an isoxazole group, a pyridine group, a pyrazine group, a pyrimidine group, a pyridazine group, an indazole group, a purine group, a quinoline group, an isoquinoline group, a benzoquinoline group, a phthalazine group, a naphthyridine group, a quinoxaline group, a quinazoline group, a cinnoline group, a phenanthridine group, an acridine group, a phenanthroline group, a phenazine group, a benzimidazole group, an isobenzothiazole group, a benzoxazole group, an isobenzoxazole group, a triazole group, a tetrazole group, an oxadiazole group, a triazine group, a thiadiazole group, an imidazopyridine group, an imidazopyrimidine group, and an azacarbazole group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, -Si(Q₃₁)(Q₃₂)(Q₃₃), -S(=O)₂(Q₃₁), and -P(=O)(Q₃₁)(Q₃₂),
wherein Q₃₁ to Q₃₃ may each independently be selected from a C₁-C₁₀ alkyl group, a C₁-C₁₀ alkoxy group, a phenyl group, a biphenyl group, a terphenyl group, and a naphthyl group.

When xe11 in Formula 601 is 2 or more, two or more Ar₆₀₁(s) may be linked to each other via a single bond.

In one or more embodiments, Ar₆₀₁ in Formula 601 may be an anthracene group.

In one or more embodiments, the compound represented by Formula 601 may be represented by Formula 601-1: 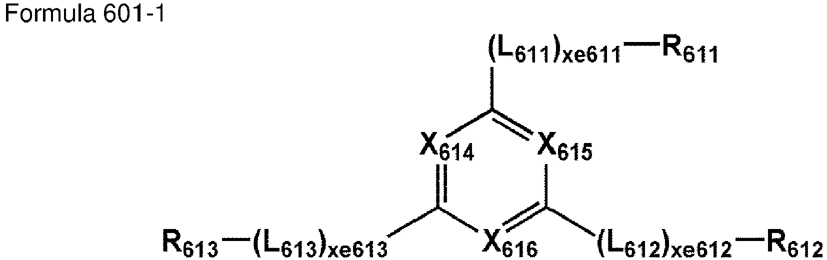 wherein, in Formula 601-1,
X₆₁₄ may be N or C(R₆₁₄), X₆₁₅ may be N or C(R₆₁₅), X₆₁₆ may be N or C(R₆₁₆), and at least one selected from X₆₁₄ to X₆₁₆ may be N,
L₆₁₁ to L₆₁₃ may each independently be the same as described in connection with L₆₀₁,
xe611 to xe613 may each independently be the same as described in connection with xe1,
R₆₁₁ to R₆₁₃ may each independently be the same as described in connection with R₆₀₁, and
R₆₁₄ to R₆₁₆ may each independently be selected from hydrogen, deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, and a naphthyl group.

In an embodiment, L₆₀₁ and L₆₁₁ to L₆₁₃ in Formulae 601 and 601-1 may each independently be selected from:
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, a benzoisothiazolylene group, a benzoxazolylene group, a benzoisoxazolylene group, a triazolylene group, a tetrazolylene group, an imidazopyridinylene group, an imidazopyrimidinylene group, and an azacarbazolylene group; and
a phenylene group, a naphthylene group, a fluorenylene group, a spiro-bifluorenylene group, a benzofluorenylene group, a dibenzofluorenylene group, a phenanthrenylene group, an anthracenylene group, a fluoranthenylene group, a triphenylenylene group, a pyrenylene group, a chrysenylene group, a perylenylene group, a pentaphenylene group, a hexacenylene group, a pentacenylene group, a thiophenylene group, a furanylene group, a carbazolylene group, an indolylene group, an isoindolylene group, a benzofuranylene group, a benzothiophenylene group, a dibenzofuranylene group, a dibenzothiophenylene group, a benzocarbazolylene group, a dibenzocarbazolylene group, a dibenzosilolylene group, a pyridinylene group, an imidazolylene group, a pyrazolylene group, a thiazolylene group, an isothiazolylene group, an oxazolylene group, an isoxazolylene group, a thiadiazolylene group, an oxadiazolylene group, a pyrazinylene group, a pyrimidinylene group, a pyridazinylene group, a triazinylene group, a quinolinylene group, an isoquinolinylene group, a benzoquinolinylene group, a phthalazinylene group, a naphthyridinylene group, a quinoxalinylene group, a quinazolinylene group, a cinnolinylene group, a phenanthridinylene group, an acridinylene group, a phenanthrolinylene group, a phenazinylene group, a benzimidazolylene group, a benzoisothiazolylene group, a benzoxazolylene group, a benzoisoxazolylene group, a triazolylene group, a tetrazolylene group, an imidazopyridinylene group, an imidazopyrimidinylene group, and an azacarbazolylene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, a benzoisothiazolyl group, a benzoxazolyl group, a benzoisoxazolyl group, a triazolyl group, a tetrazolyl group, an imidazopyridinyl group, an imidazopyrimidinyl group, and an azacarbazolyl group,
but embodiments of the present disclosure are not limited thereto.

In one or more embodiments, xe1 and xe611 to xe613 in Formulae 601 and 601-1 may each independently be 0, 1, or 2.

In one or more embodiments, R₆₀₁ and R₆₁₁ to R₆₁₃ in Formulae 601 and 601-1 may each independently be selected from:
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an imidazopyridinyl group, an imidazopyrimidinyl group, and an azacarbazolyl group; and
a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, a benzoisoxazolyl group, a triazolyl group, a tetrazolyl group, an imidazopyridinyl group, an imidazopyrimidinyl group, and an azacarbazolyl group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, a spiro-bifluorenyl group, a benzofluorenyl group, a dibenzofluorenyl group, a phenanthrenyl group, an anthracenyl group, a fluoranthenyl group, a triphenylenyl group, a pyrenyl group, a chrysenyl group, a perylenyl group, a pentaphenyl group, a hexacenyl group, a pentacenyl group, a thiophenyl group, a furanyl group, a carbazolyl group, an indolyl group, an isoindolyl group, a benzofuranyl group, a benzothiophenyl group, a dibenzofuranyl group, a dibenzothiophenyl group, a benzocarbazolyl group, a dibenzocarbazolyl group, a dibenzosilolyl group, a pyridinyl group, an imidazolyl group, a pyrazolyl group, a thiazolyl group, an isothiazolyl group, an oxazolyl group, an isoxazolyl group, a thiadiazolyl group, an oxadiazolyl group, a pyrazinyl group, a pyrimidinyl group, a pyridazinyl group, a triazinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, a benzoisothiazolyl group, a benzoxazolyl group, a benzoisoxazolyl group, a triazolyl group, a tetrazolyl group, an imidazopyridinyl group, an imidazopyrimidinyl group, and an azacarbazolyl group; and
-S(=O)₂(Q₆₀₁) and -P(=O)(O₆₀₁)(O₆₀₂),
wherein Q₆₀₁ and Q₆₀₂ are the same as described above.

The electron transport region may comprise at least one compound selected from Compounds ET1 to ET36, but embodiments of the present disclosure are not limited thereto:

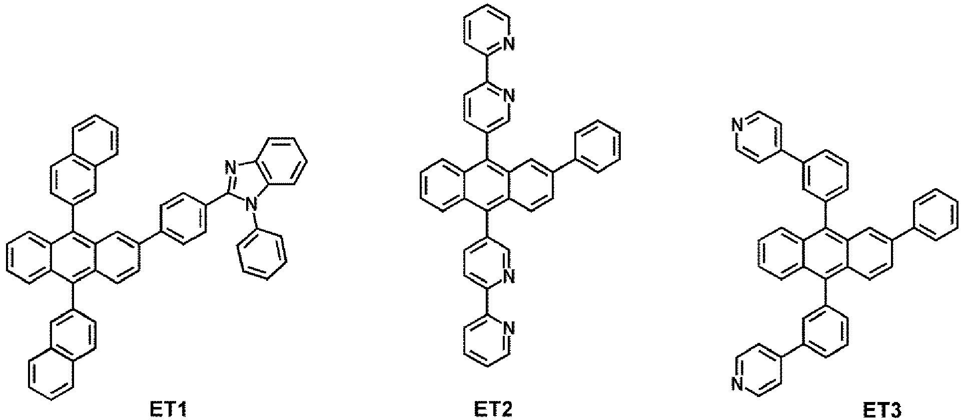

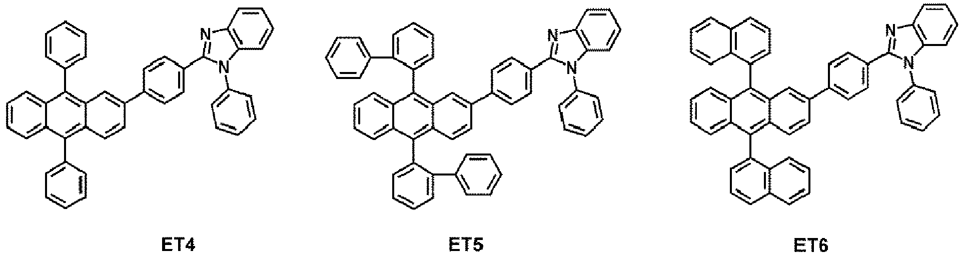

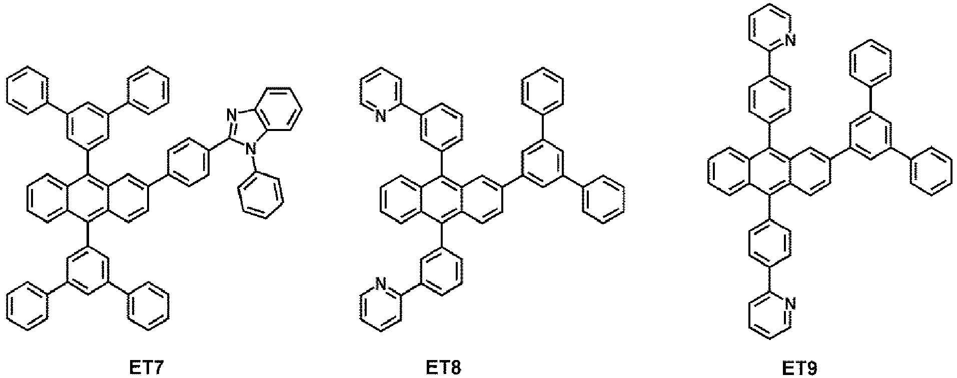

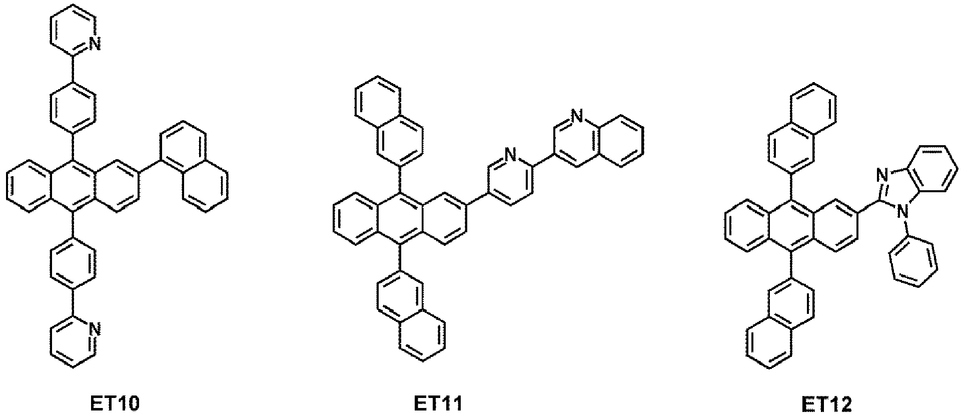

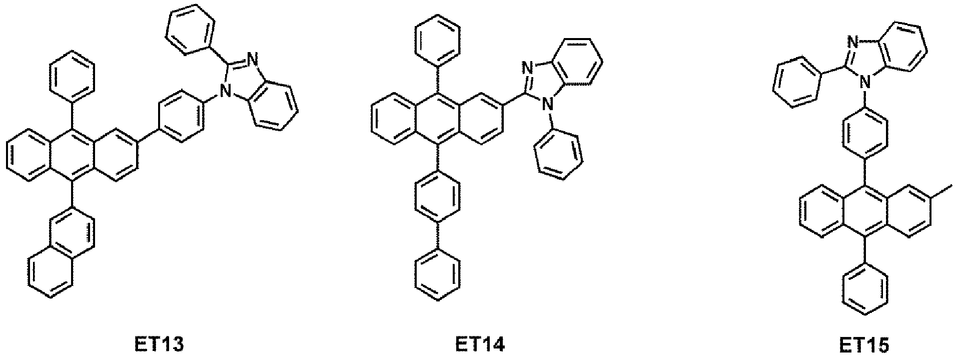

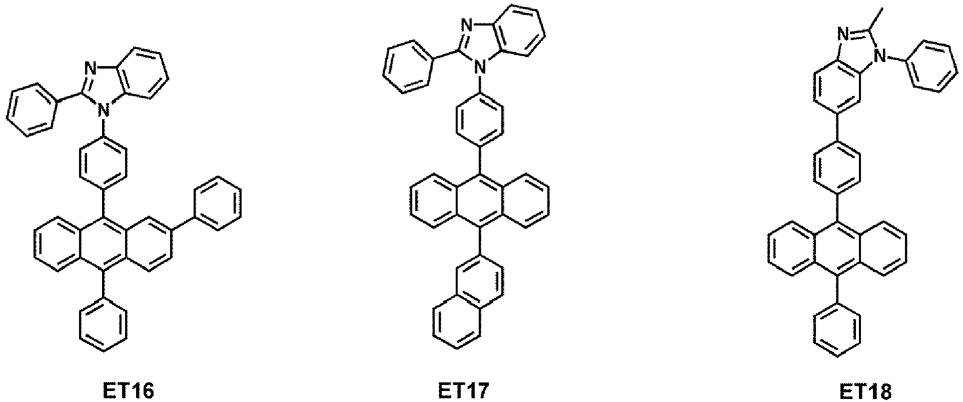

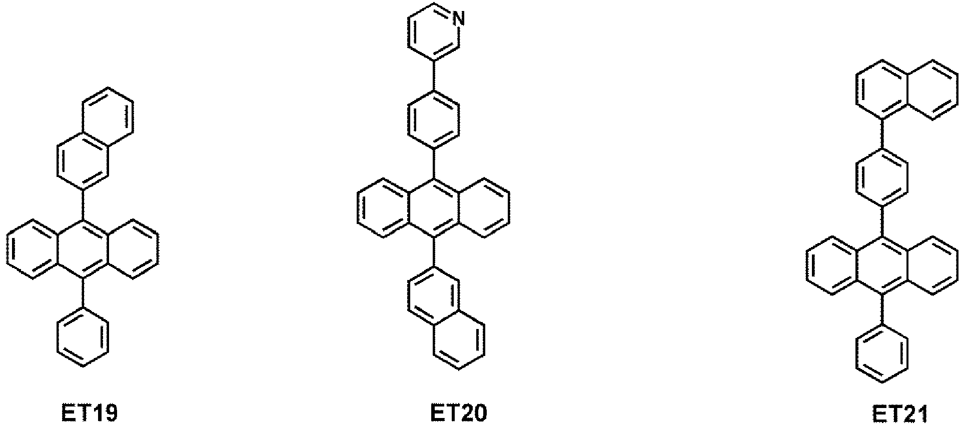

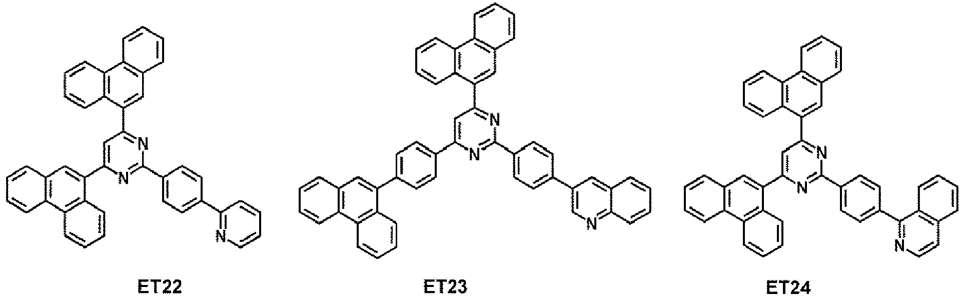

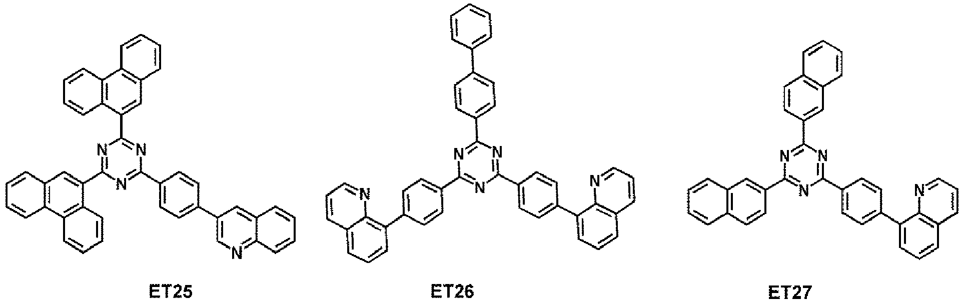

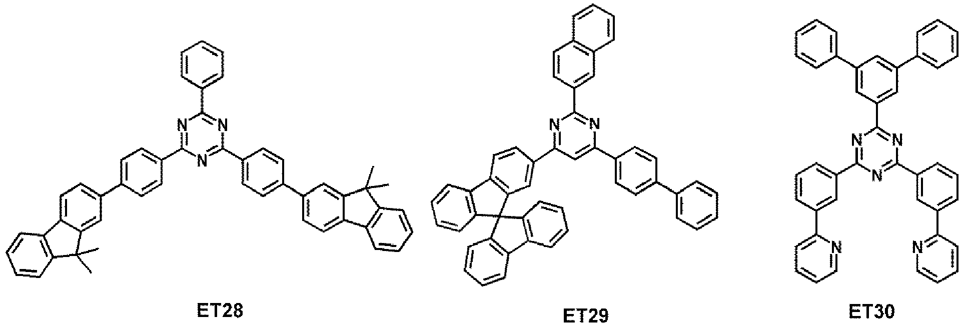

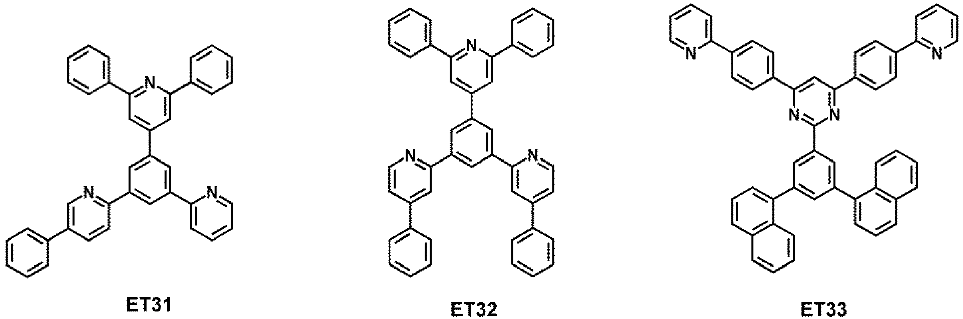

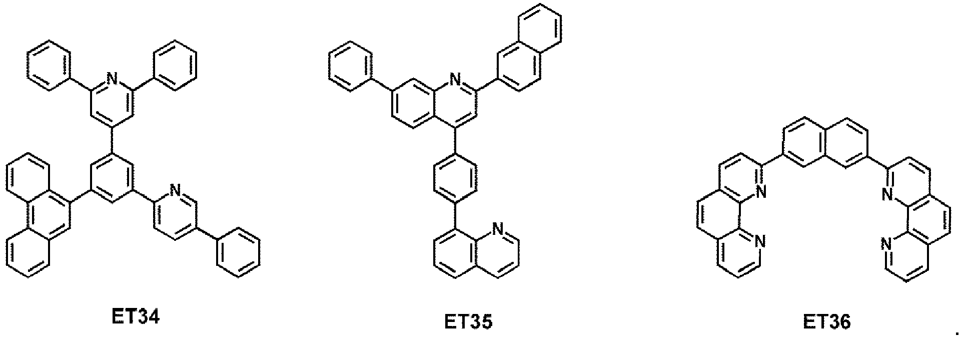

In one or more embodiments, the electron transport region may comprise at least one compound selected from 2,9-dimethyl-4,7-diphenyl-1,10-phenanthroline (BCP), 4,7-diphenyl-1,10-phenanthroline (Bphen), Alq₃, BAIq, 3-(biphenyl-4-yl)-5-(4-tert-butylphenyl)-4-phenyl-4H-1,2,4-triazole (TAZ), and NTAZ.

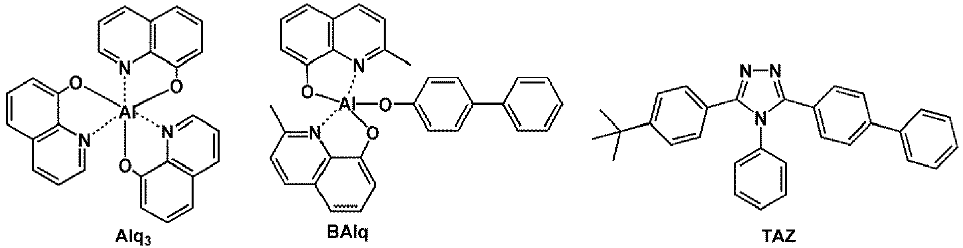

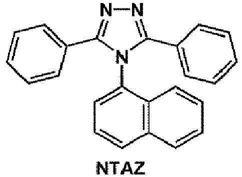

Thicknesses of the buffer layer, the hole blocking layer, and the electron control layer may each independently be in a range of about 20 Å to about 1,000 Å, for example, about 30 Å to about 300 Å. When the thicknesses of the buffer layer, the hole blocking layer, and the electron control layer are within any of the foregoing ranges, excellent hole blocking characteristics and/or excellent electron control characteristics may be obtained without a substantial increase in driving voltage.

A thickness of the electron transport layer may be in a range of about 100 Å to about 1,000 Å, for example, about 150 Å to about 500 Å. When the thickness of the electron transport layer is within any of the ranges described above, the electron transport layer may have suitable or satisfactory electron transport characteristics without a substantial increase in driving voltage.

The electron transport region (for example, the electron transport layer in the electron transport region) may further comprise, in addition to the materials described above, a metal-containing material.

The metal-containing material may comprise at least one selected from alkali metal complex and alkaline earth-metal complex. The alkali metal complex may comprise a metal ion selected from a Li ion, a Na ion, a K ion, a Rb ion, and a Cs ion, and the alkaline earth-metal complex may comprise a metal ion selected from a Be ion, a Mg ion, a Ca ion, a Sr ion, and a Ba ion. A ligand coordinated with the metal ion of the alkali metal complex or the alkaline earth-metal complex may be selected from a hydroxy quinoline, a hydroxy isoquinoline, a hydroxy benzoquinoline, a hydroxy acridine, a hydroxy phenanthridine, a hydroxy phenyloxazole, a hydroxy phenylthiazole, a hydroxy diphenyloxadiazole, a hydroxy diphenylthiadiazole, a hydroxy phenylpyridine, a hydroxy phenylbenzimidazole, a hydroxy phenylbenzothiazole, a bipyridine, a phenanthroline, and a cyclopentadiene, but embodiments of the present disclosure are not limited thereto.

For example, the metal-containing material may comprise a Li complex. The Li complex may comprise, for example, Compound ET-D1 (lithium quinolate, LiQ) or ET-D2:

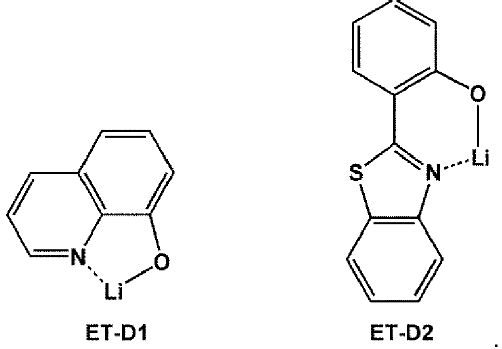

The electron transport region may comprise an electron injection layer that facilitates the injection of electrons from the second electrode 190. The electron injection layer may directly contact the second electrode 190.

The electron injection layer may have i) a single-layered structure comprising a single layer comprising a single material, ii) a single-layered structure comprising a single layer comprising a plurality of different materials, or iii) a multi-layered structure having a plurality of layers comprising a plurality of different materials.

The electron injection layer may comprise an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combinations thereof.

The alkali metal may be selected from Li, Na, K, Rb, and Cs. In an embodiment, the alkali metal may be Li, Na, or Cs. In one or more embodiments, the alkali metal may be Li or Cs, but embodiments of the present disclosure are not limited thereto.

The alkaline earth metal may be selected from Mg, Ca, Sr, and Ba.

The rare earth metal may be selected from Sc, Y, Ce, Tb, Yb, and Gd.

The alkali metal compound, the alkaline earth-metal compound, and the rare earth metal compound may be selected from oxides and halides (for example, fluorides, chlorides, bromides, and/or iodides) of the alkali metal, the alkaline earth-metal, and the rare earth metal.

The alkali metal compound may be selected from alkali metal oxides, such as Li₂O, Cs₂O, and K₂O, and alkali metal halides, such as LiF, NaF, CsF, KF, Lil, Nal, Csl, and KI. In an embodiment, the alkali metal compound may be selected from LiF, Li₂O, NaF, Lil, Nal, Csl, and KI, but embodiments of the present disclosure are not limited thereto.

The alkaline earth-metal compound may be selected from alkaline earth-metal oxides, such as BaO, SrO, CaO, BaₓSr₁₋ₓO (0<x<1), and BaₓCa₁₋ₓO (0<x<1). In an embodiment, the alkaline earth-metal compound may be selected from BaO, SrO, and CaO, but embodiments of the present disclosure are not limited thereto.

The rare earth metal compound may be selected from YbF₃, ScF₃, Sc₂O₃, Y₂O₃, Ce₂O₃, GdF₃ and TbF₃. In an embodiment, the rare earth metal compound may be selected from YbF₃, ScF₃, TbF₃, YbI₃, Scb, and TbI₃, but embodiments of the present disclosure are not limited thereto.

The alkali metal complex, the alkaline earth-metal complex, and the rare earth metal complex may comprise an ion of alkali metal, alkaline earth-metal, and rare earth metal as described above, and a ligand coordinated with a metal ion of the alkali metal complex, the alkaline earth-metal complex, or the rare earth metal complex may be selected from hydroxy quinoline, hydroxy isoquinoline, hydroxy benzoquinoline, hydroxy acridine, hydroxy phenanthridine, hydroxy phenyloxazole, hydroxy phenylthiazole, hydroxy diphenyloxadiazole, hydroxy diphenylthiadiazole, hydroxy phenylpyridine, hydroxy phenylbenzimidazole, hydroxy phenylbenzothiazole, bipyridine, phenanthroline, and cyclopentadiene, but embodiments of the present disclosure are not limited thereto.

The electron injection layer may comprise (or consist of) an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combinations thereof, as described above. In one or more embodiments, the electron injection layer may further comprise an organic material. When the electron injection layer further comprises an organic material, an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combination thereof may be homogeneously or non-homogeneously dispersed in a matrix comprising the organic material.

A thickness of the electron injection layer may be in a range of about 1 Å to about 100 Å, or, for example, about 3 Å to about 90 Å. When the thickness of the electron injection layer is within any of the ranges described above, the electron injection layer may have suitable or satisfactory electron injection characteristics without a substantial increase in driving voltage.

### Second electrode 190

The second electrode 190 is on the organic layer 150 having such a structure. The second electrode 190 may be a cathode which is an electron injection electrode, and in this regard, a material for forming the second electrode 190 may be selected from a metal, an alloy, an electrically conductive compound, and a combination thereof, which have a relatively low work function.

The second electrode 190 may comprise at least one selected from lithium (Li), silver (Ag), magnesium (Mg), aluminum (Al), aluminum-lithium (Al-Li), calcium (Ca), magnesium-indium (Mg-In), magnesium-silver (Mg-Ag), silver-magnesium (Ag-Mg), ytterbium (Yb), silver-ytterbium (Ag-Yb), ITO, and IZO, but embodiments of the present disclosure are not limited thereto. The second electrode 190 may be a transmissive electrode, a semi-transmissive electrode, or a reflective electrode.

The second electrode 190 may have a single-layered structure or a multi-layered structure comprising two or more layers.

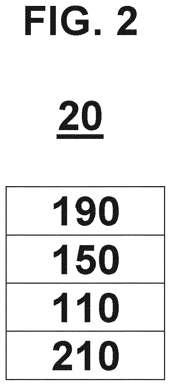

### Description of FIGS. 2 to 4

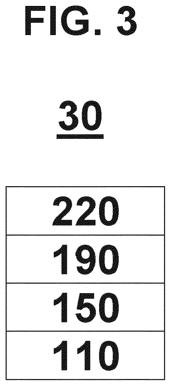

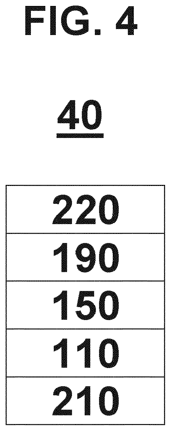

An organic light-emitting device 20 of FIG. 2 comprises a first capping layer 210, the first electrode 110, the organic layer 150, and the second electrode 190 which are sequentially stacked in this stated order, an organic light-emitting device 30 of FIG. 3 comprises the first electrode 110, the organic layer 150, the second electrode 190, and a second capping layer 220 which are sequentially stacked in this stated order, and an organic light-emitting device 40 of FIG. 4 comprises a first capping layer 210, a first electrode 110, an organic layer 150, a second electrode 190, and a second capping layer 220.

Regarding FIGS. 2 to 4, the first electrode 110, the organic layer 150, and the second electrode 190 may be understood by referring to the description presented in connection with FIG. 1.

In the organic layer 150 of each of the organic light-emitting devices 20 and 40, light generated in an emission layer may pass through the first electrode 110, which is a semi-transmissive electrode or a transmissive electrode, and the first capping layer 210 toward the outside, and in the organic layer 150 of each of the organic light-emitting devices 30 and 40, light generated in an emission layer may pass through the second electrode 190, which is a semi-transmissive electrode or a transmissive electrode, and the second capping layer 220 toward the outside.

The first capping layer 210 and the second capping layer 220 may increase external luminescence efficiency according to a principle of constructive interference.

The first capping layer 210 and the second capping layer 220 may each independently be an organic capping layer comprising an organic material, an inorganic capping layer comprising an inorganic material, or a composite capping layer comprising an organic material and an inorganic material.

At least one selected from the first capping layer 210 and the second capping layer 220 may each independently comprise at least one material selected from carbocyclic compounds, amine-based compounds, porphyrine derivatives, phthalocyanine derivatives, naphthalocyanine derivatives, alkali metal complexes, and alkaline earth metal complexes. The carbocyclic compound, and the amine-based compound may be optionally substituted with a substituent containing at least one element selected from O, N, S, Se, Si, F, Cl, Br, and I. In an embodiment, at least one of the first capping layer 210 and the second capping layer 220 may each independently comprise an amine-based compound.

In an embodiment, at least one selected from the first capping layer 210 and the second capping layer 220 may each independently comprise the compound represented by Formula 201 or the compound represented by Formula 202.

In one or more embodiments, at least one of the first capping layer 210 and the second capping layer 220 may each independently comprise a compound selected from Compounds HT28 to HT33 and Compounds CP1 to CP5, but embodiments of the present disclosure are not limited thereto.

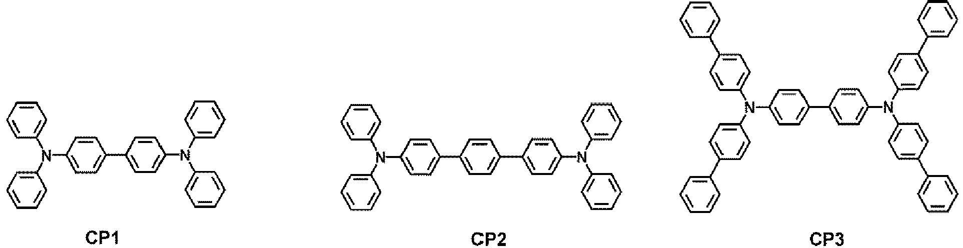

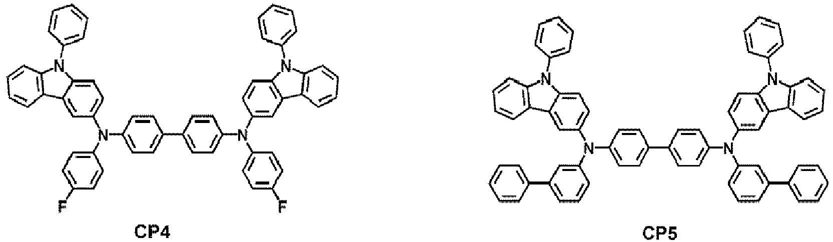

Hereinbefore, the organic light-emitting device according to an embodiment has been described in connection with FIGS. 1-4. However, embodiments of the present disclosure are not limited thereto.

Layers constituting the hole transport region, an emission layer, and layers constituting the electron transport region may be formed in a certain region by using one or more suitable methods selected from vacuum deposition, spin coating, casting, Langmuir-Blodgett (LB) deposition, ink-jet printing, laser-printing, and laser-induced thermal imaging.

When layers constituting the hole transport region, an emission layer, and layers constituting the electron transport region are formed by vacuum deposition, the deposition may be performed at a deposition temperature of about 100 °C to about 500 °C, a vacuum degree of about 10⁻⁸ torr to about 10⁻³ torr, and a deposition speed of about 0.01 Å/sec to about 100 Å/sec by taking into account a material to be comprised in a layer to be formed and the structure of a layer to be formed.

When layers constituting the hole transport region, an emission layer, and layers constituting the electron transport region are formed by spin coating, the spin coating may be performed at a coating speed of about 2,000 rpm to about 5,000 rpm and at a heat treatment temperature of about 80 °C to 200 °C by taking into account a material to be comprised in a layer to be formed and the structure of a layer to be formed.

### General definition of at least some of the substituents

The term "C₁-C₆₀ alkyl group," as used herein, refers to a linear or branched aliphatic saturated hydrocarbon monovalent group having 1 to 60 carbon atoms, and examples thereof comprise a methyl group, an ethyl group, a propyl group, an isobutyl group, a sec-butyl group, a tert-butyl group, a pentyl group, an isoamyl group, and a hexyl group. The term "C₁-C₆₀ alkylene group," as used herein, refers to a divalent group having substantially the same structure as the C₁-C₆₀ alkyl group.

The term "C₂-C₆₀ alkenyl group," as used herein, refers to a hydrocarbon group having at least one carbon-carbon double bond at a main chain (e.g., in the middle) or at a terminal end (e.g., the terminus) of the C₂-C₆₀ alkyl group, and examples thereof comprise an ethenyl group, a propenyl group, and a butenyl group. The term "C₂-C₆₀ alkenylene group," as used herein, refers to a divalent group having substantially the same structure as the C₂-C₆₀ alkenyl group.

The term "C₂-C₆₀ alkynyl group," as used herein, refers to a hydrocarbon group having at least one carbon-carbon triple bond at a main chain (e.g., in the middle) or at a terminal end (e.g., the terminus) of the C₂-C₆₀ alkyl group, and examples thereof comprise an ethynyl group, and a propynyl group. The term "C₂-C₆₀ alkynylene group," as used herein, refers to a divalent group having substantially the same structure as the C₂-C₆₀ alkynyl group.

The term "C₁-C₆₀ alkoxy group," as used herein, refers to a monovalent group represented by -OA₁₀₁ (wherein A₁₀₁ is the C₁-C₆₀ alkyl group), and examples thereof comprise a methoxy group, an ethoxy group, and an isopropyloxy group.

The term "C₃-C₁₀ cycloalkyl group," as used herein, refers to a monovalent saturated hydrocarbon monocyclic group having 3 to 10 carbon atoms, and examples thereof comprise a cyclopropyl group, a cyclobutyl group, a cyclopentyl group, a cyclohexyl group, and a cycloheptyl group. The term "C₃-C₁₀ cycloalkylene group," as used herein, refers to a divalent group having substantially the same structure as the C₃-C₁₀ cycloalkyl group.

The term "C₁-C₁₀ heterocycloalkyl group," as used herein, refers to a monovalent monocyclic group having at least one heteroatom selected from N, O, Si, P, and S as a ring-forming atom and 1 to 10 carbon atoms, and examples thereof comprise a 1,2,3,4-oxatriazolidinyl group, a tetrahydrofuranyl group, and a tetrahydrothiophenyl group. The term "C₁-C₁₀ heterocycloalkylene group," as used herein, refers to a divalent group having substantially the same structure as the C₁-C₁₀ heterocycloalkyl group.

The term "C₃-C₁₀ cycloalkenyl group," as used herein, refers to a monovalent monocyclic group that has 3 to 10 carbon atoms and at least one carbon-carbon double bond in the ring thereof and no aromaticity (e.g., is not aromatic), and examples thereof comprise a cyclopentenyl group, a cyclohexenyl group, and a cycloheptenyl group. The term "C₃-C₁₀ cycloalkenylene group," as used herein, refers to a divalent group having substantially the same structure as the C₃-C₁₀ cycloalkenyl group.

The term "C₁-C₁₀ heterocycloalkenyl group," as used herein, refers to a monovalent monocyclic group that has at least one heteroatom selected from N, O, Si, P, and S as a ring-forming atom, 1 to 10 carbon atoms, and at least one double bond in its ring. Examples of the C₁-C₁₀ heterocycloalkenyl group comprise a 4,5-dihydro-1,2,3,4-oxatriazolyl group, a 2,3-dihydrofuranyl group, and a 2,3-dihydrothiophenyl group. The term "C₁-C₁₀ heterocycloalkenylene group," as used herein, refers to a divalent group having substantially the same structure as the C₁-C₁₀ heterocycloalkenyl group.

The term "C₆-C₆₀ aryl group," as used herein, refers to a monovalent group having a carbocyclic aromatic system having 6 to 60 carbon atoms, and the term "C₆-C₆₀ arylene group," as used herein, refers to a divalent group having a carbocyclic aromatic system having 6 to 60 carbon atoms. Examples of the C₆-C₆₀ aryl group comprise a fluorenyl group, a phenyl group, a naphthyl group, an anthracenyl group, a phenanthrenyl group, a pyrenyl group, and a chrysenyl group. When the C₆-C₆₀ aryl group and the C₆-C₆₀ arylene group each comprise two or more rings, the two or more rings may be fused to each other (e.g., combined together).

The term "C₁-C₆₀ heteroaryl group," as used herein, refers to a monovalent group having a heteroocyclic aromatic system that has at least one heteroatom selected from N, O, Si, P, and S as a ring-forming atom, in addition to 1 to 60 carbon atoms. The term "C₁-C₆₀ heteroarylene group," as used herein, refers to a divalent group having a heterocyclic aromatic system that has at least one heteroatom selected from N, O, Si, P, and S as a ring-forming atom, in addition to 1 to 60 carbon atoms. Examples of the C₁-C₆₀ heteroaryl group comprise a carbazolyl group, a pyridinyl group, a pyrimidinyl group, a pyrazinyl group, a pyridazinyl group, a triazinyl group, a quinolinyl group, and an isoquinolinyl group. When the C₁-C₆₀ heteroaryl group and the C₁-C₆₀ heteroarylene group each comprise two or more rings, the two or more rings may be condensed with each other (e.g., combined together with each other).

The term "C₆-C₆₀ aryloxy group," as used herein, refers to -OA₁₀₂ (wherein A₁₀₂ is the C₆-C₆₀ aryl group), and the term "C₆-C₆₀ arylthio group," as used herein, refers to -SA₁₀₃ (wherein A₁₀₃ is the C₆-C₆₀ aryl group).

The term "monovalent non-aromatic condensed polycyclic group," as used herein, refers to a monovalent group (for example, having 8 to 60 carbon atoms) having two or more rings condensed with each other (e.g., combined together with each other), only carbon atoms as ring-forming atoms, and no aromaticity in its entire molecular structure (e.g., is not aromatic). An example of the monovalent non-aromatic condensed polycyclic group is a fluorenyl group. The term "divalent non-aromatic condensed polycyclic group," as used herein, refers to a divalent group having substantially the same structure as the monovalent non-aromatic condensed polycyclic group. For example, the monovalent non-aromatic condensed polycyclic group may be monovalent non-aromatic condensed C₈-C₆₀ polycyclic group.

The term "monovalent non-aromatic condensed heteropolycyclic group" as used herein refers to a monovalent group (for example, having 1 to 60 carbon atoms) having two or more rings condensed to each other, at least one heteroatom selected from N, O, Si, P, and S, other than carbon atoms, as a ring-forming atom, and no aromaticity in its entire molecular structure. An example of the monovalent non-aromatic condensed heteropolycyclic group is a carbazolyl group. The term "divalent non-aromatic condensed heteropolycyclic group" as used herein refers to a divalent group having substantially the same structure as the monovalent non-aromatic condensed heteropolycyclic group. For example, the monovalent non-aromatic condensed heteropolycyclic group may be monovalent non-aromatic condensed 8 to 60 membered heteropolycyclic group.

The term "C₅-C₆₀ carbocyclic group" as used herein refers to a monocyclic or polycyclic group that comprises only carbon as a ring-forming atom and comprises (or consists of) 5 to 60 carbon atoms. The C₅-C₆₀ carbocyclic group may be an aromatic carbocyclic group, or a non-aromatic carbocyclic group. The C₅-C₆₀ carbocyclic group may be a ring, such as benzene, a monovalent group, such as a phenyl group, or a divalent group, such as a phenylene group. In one or more embodiments, depending on the number of substituents connected to the C₅-C₆₀ carbocyclic group, the C₅-C₆₀ carbocyclic group may be a trivalent group, or a quadrivalent group.

The term "C₁-C₆₀ heterocyclic group," as used herein, refers to a group having substantially the same structure as the C₅-C₆₀ carbocyclic group, except that as a ring-forming atom, at least one heteroatom selected from N, O, Si, P, and S is used in addition to carbon (the number of carbon atoms may be in a range of 1 to 60).

In the present specification, at least one substituent of the substituted C₅-C₆₀ carbocyclic group, the substituted C₁-C₆₀ heterocyclic group, the substituted C₃-C₁₀ cycloalkylene group, the substituted C₁-C₁₀ heterocycloalkylene group, the substituted C₃-C₁₀ cycloalkenylene group, the substituted C₁-C₁₀ heterocycloalkenylene group, the substituted C₆-C₆₀ arylene group, the substituted C₁-C₆₀ heteroarylene group, the substituted divalent non-aromatic condensed polycyclic group, the substituted divalent non-aromatic condensed heteropolycyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group may be selected from:
deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F,-Cl, -Br, -I, a hydroxyl group, a cyano group, a nitro group, an amidino group, a hydrazino group, a hydrazono group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -Si(Q₁₁)(Q₁₂)(Q₁₃),-N(O₁₁)(O₁₂), -B(O₁₁)(O₁₂), -C(=O)(O₁₁), -S(=O)₂(O₁₁), and -P(=O)(O₁₁)(O₁₂);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C6-C60 arylthio group, a C1-C60 heteroaryl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one selected from deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -Si(Q₂₁)(Q₂₂)(Q₂₃),-N(Q₂₁)(Q₂₂), -B(Q₂₁)(Q₂₂), -C(=O)(Q₂₁), -S(=O)₂(Q₂₁), and -P(=O)(Q₂₁)(Q₂₂); and
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and -P(=O)(Q₃₁)(Q₃₂),
wherein Q₁₁ to Q₁₃, Q₂₁ to Q₂₃, and Q₃₁ to Q₃₃ may each independently be selected from hydrogen, deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, a monovalent non-aromatic condensed heteropolycyclic group, a biphenyl group, and a terphenyl group.

Certain groups are specified herein as being substituted or unsubstituted. Unless otherwise specified, when a group is substituted it is typically substituted with 1, 2, 3 or 4 substituents. For example, when a group is substituted it may substituted with 1, 2 or 3 substituents; 1 or 2 substituents; or 1 substituent. Groups which are not specified as being substituted or unsubstituted are typically unsubstituted.

The term "Ph," as used herein, refers to a phenyl group, the term "Me," as used herein, refers to a methyl group, the term "Et," as used herein, refers to an ethyl group, the term "ter-Bu" or "Bu^{t}," as used herein, refers to a tert-butyl group, and the term "OMe," as used herein, refers to a methoxy group.

The term "biphenyl group," as used herein, refers to "a phenyl group substituted with a phenyl group." In other words, the "biphenyl group" is a substituted phenyl group having a C₆-C₆₀ aryl group as a substituent.

The term "terphenyl group," as used herein, refers to "a phenyl group substituted with a biphenyl group." In other words, the "terphenyl group" is a substituted phenyl group having, as a substituent, a C₆-C₆₀ aryl group substituted with a C₆-C₆₀ aryl group.

*, *', and *" as used herein, unless defined otherwise, each refer to a binding site to a neighboring atom in a corresponding formula.

Hereinafter, compounds according to embodiments and an organic light-emitting device according to embodiments will be described in more detail with reference to Synthesis Examples and Examples. The wording "B was used instead of A" used in describing Synthesis Examples refers to that an identical molar equivalent of B was used in place of A.

### Examples

### Intermediate 1: Synthesis of C(1)

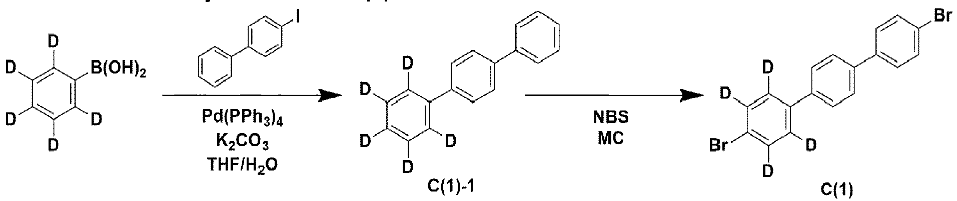

Synthesis of C(1)-1: (phenyl-d5)boronic acid (1 eq, 1.27 g), 4-iodo-1,1'-biphenyl (1 eq, 2.8 g), Pd(PPh₃)₄ (0.05 eq, 0.56 g), K₂CO₃ (3 eq, 4.14 g), 20 ml of tetrahydrofuran (THF), and 5 ml of H₂O were added to a one-neck round flask and stirred at 80 °C for 6 hours. The resultant reaction product was worked up with ethylacetate (EA)/H₂O and separated using column chromatography (Dichloromethane (MC)/hexane (Hx) = 1/50). 1.88 g of the obtained compound (C(1)-1) was obtained (Yield = 80%, Purity = 99%).

Synthesis of C(1): C(1)-1 (1 eq, 1.88 g), N-bromosuccinimide (NBS) (2.2 eq, 3.1 g), and 20 ml of MC were added to a one-neck round flask and stirred at 30 °C for 6 hours. The resultant reaction product was worked up with EA/H₂O and separated using column chromatography (MC/Hx = 1/100). 2.8 g of the obtained compound (C(1)) was obtained (Yield = 90%, Purity = 98%).

### Intermediate 2: Synthesis of C(2)

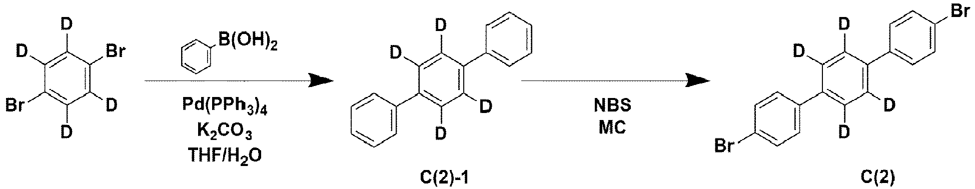

Synthesis of C(2)-1: 1,4-dibromobenzene-2,3,5,6-d4 (1 eq, 2.4 g), phenylboronic acid (2.2 eq, 2.7 g), Pd(PPh₃)₄ (0.05 eq, 0.56 g), K₂CO₃ (3 eq, 4.14 g), 20 ml of THF, and 5 ml of H₂O were added to a one-neck round flask and stirred at 80 °C for 6 hours. The resultant reaction product was worked up with EA/H₂O and separated using column chromatography (MC/Hx = 1/50). 1.87 g of the obtained compound (C(2)-1) was obtained (Yield = 78%, Purity = 99%).

Synthesis of C(2): C(2)-1 (1 eq, 1.87 g), NBS (2.2 eq, 3.05 g), and 20 ml of MC were added to a one-neck round flask and stirred at 30 °C for 6 hours. The resultant reaction product was worked up with EA/H₂O and separated using column chromatography (MC/Hx = 1/100). 2.8 g of the obtained compound (C(2)) was obtained (Yield = 90%, Purity = 98%).

### Intermediate 3: Synthesis of C(3)

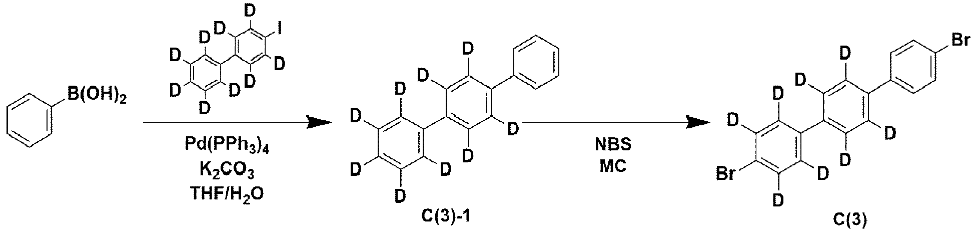

Synthesis of C(3)-1: phenylboronic acid (1 eq, 1.21 g), 4-iodo-1,1'-biphenyl-2,2',3,3',4',5,5',6,6'-d9 (1 eq, 2.89 g), Pd(PPh₃)₄ (0.05 eq, 0.56 g), K₂CO₃ (3 eq, 4.14 g), 20 ml of THF, 5 ml of H₂O were added to a one-neck round flask and stirred at 80 °C for 6 hours. The resultant reaction product was worked up with EA/H₂O and separated using column chromatography (MC/Hx = 1/50). 1.91 g of the obtained compound (C(3)-1) was obtained (Yield = 80%, Purity = 99%).

Synthesis of C(3): C(3)-1 (1 eq, 1.91 g), NBS (2.2 eq, 3.1 g), and 20 ml of MC were added to a one-neck round flask and stirred at 30 °C for 6 hours. The resultant reaction product was worked up with EA/H₂O and separated using column chromatography (MC/Hx = 1/100). 2.78 g of the obtained compound (C(3)) was obtained (Yield = 88%, Purity = 98%).

### Intermediate 4: Synthesis of C(4)

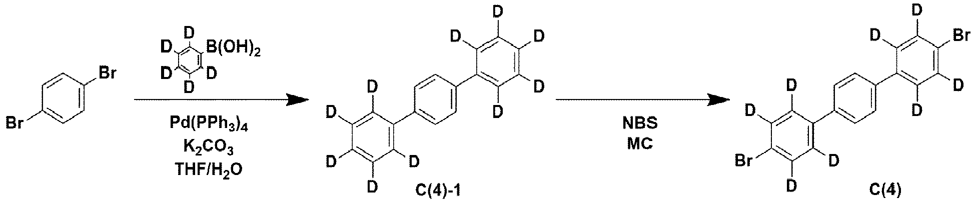

Synthesis of C(4)-1: 1,4-dibromobenzene (1 eq, 2.36 g), (phenyl-d5)boronic acid (2.2 eq, 2.8 g), Pd(PPh₃)₄ (0.05 eq, 0.56 g), K₂CO₃(3 eq, 4.14 g), 20 ml of THF, and 5 ml of H₂O were added to a one-neck round flask and stirred at 80 °C for 6 hours. The resultant reaction product was worked up with EA/H₂O and separated using column chromatography (MC/Hx = 1/50). 2.1 g of the obtained compound (C(4)-1) was obtained (Yield = 88%, Purity = 99%).

Synthesis of C(4): C(4)-1 (1 eq, 2.1 g), NBS (2.2 eq, 3.45 g), and 20 ml of MC were added to a one-neck round flask and stirred at 30 °C for 6 hours. The resultant reaction product was worked up with EA/H₂O and separated using column chromatography (MC/Hx = 1/100). 3.13 g of the obtained compound (C(4)) was obtained (Yield = 90%, Purity = 99%).

### Intermediate 5: Synthesis of C(5)

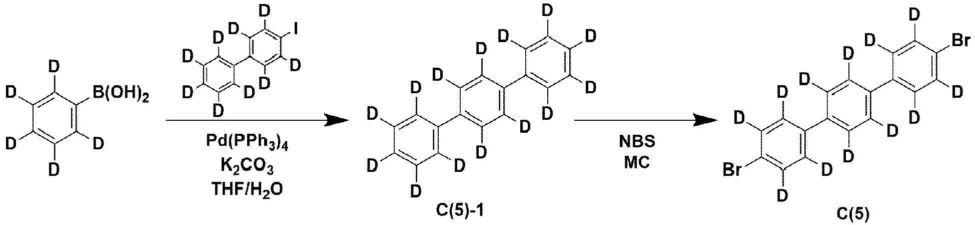

Synthesis of C(5)-1: (phenyl-d5)boronic acid (1 eq, 1.27 g), 4-iodo-1,1'-biphenyl-2,2',3,3',4',5,5',6,6'-d9 (1 eq, 2.89 g), Pd(PPh₃)₄ (0.05 eq, 0.56 g), K₂CO₃(3 eq, 4.14 g), 20 ml of THF, and 5 ml of H₂O were added to a one-neck round flask and stirred at 80 °C for 6 hours. The resultant reaction product was worked up with EA/H₂O and separated using column chromatography (MC/Hx = 1/50). 1.88 g of the obtained compound (C(5)-1) was obtained (Yield = 80%, Purity = 99%).

Synthesis of C(5): C(1)-1 (1 eq, 1.88 g), NBS (2.2 eq, 3.1 g), and 20 ml of MC were added to a one-neck round flask and stirred at 30 °C for 6 hours. The resultant reaction product was worked up with EA/H₂O and separated using column chromatography (MC/Hx = 1/100). 2.67 g of the obtained compound (C(5)) was obtained (Yield = 86%, Purity = 98%).

### Synthesis Example 1: Synthesis of Compound 1

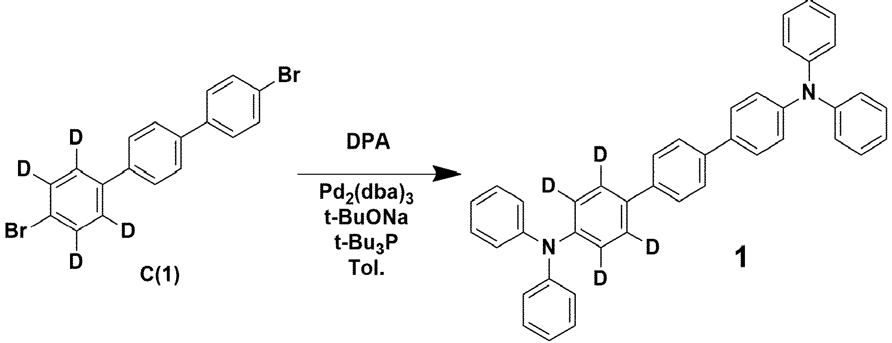

Intermediate C(1) (1 eq, 3.92 g), Diphenylamine (DPA) (2.2 eq, 3.72 g), tris(dibenzylideneacetone)dipalladium (Pd₂(dba)₃) (0.05 eq, 0.46 g), t-BuONa (3 eq, 2.88 g), t-Bu₃P (0.1 eq, 0.11 ml), and 100 ml of toluene (ToI.) were added to a one-neck round flask and stirred at 100 °C for 2 hours. The resultant reaction product was worked up with EA/H₂O and separated using column chromatography (MC/Hx = 1/5). 4.08 g of the obtained compound (Compound 1) was obtained. (Yield = 72%, Purity = 96%). Purity of the obtained product was increased by ether recrystallization. When the purity level reached 99.5% or more, sublimation purification was performed thereon. 3.5 g of the obtained compound was obtained (Purity ≥99.9%).

### Synthesis Example 2: Synthesis of Compound 2

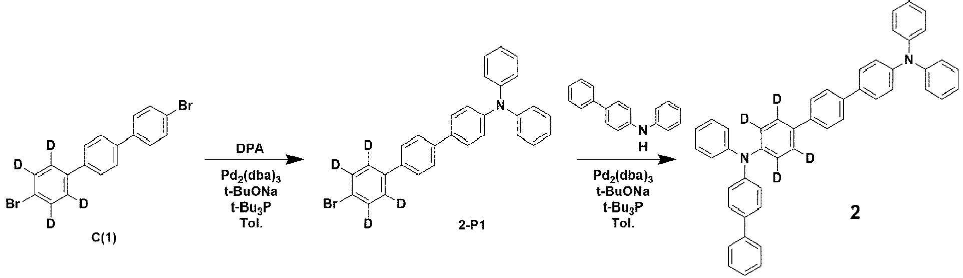

Synthesis of 2-P1: Intermediate C(1) (1 eq, 3.92 g), DPA (1 eq, 1.69 g), Pd₂(dba)₃ (0.03 eq, 0.27 g), t-BuONa (1.5 eq, 1.44 g), t-Bu₃P (0.05 eq, 0.06 ml), and 100 ml of ToI. were added to a one-neck round flask and stirred at 100 °C for 2 hours. The resultant reaction product was worked up with EA/H₂O and separated using column chromatography (MC/Hx = 1/5). 3.36 g of the obtained compound (2-P1) was obtained (Yield = 70%, Purity = 99%).

Synthesis of Compound 2: 2-P1 (1 eq, 3.36 g), N-phenyl-[1,1'-biphenyl]-4-amine (1 eq, 1.71 g), Pd₂(dba)₃ (0.03 eq, 0.189 g), t-BuONa (1.5 eq, 1 g), t-Bu₃P (0.05 eq, 0.04 ml), and 100 ml of ToI. were added to a one-neck round flask and stirred at 100 °C for 2 hours. The resultant reaction product was worked up with EA/H₂O and separated using column chromatography (MC/Hx = 1/5). 3.7 g of the obtained compound (Compound 2) was obtained (Yield = 82%, Purity = 98%). Purity of the obtained product was increased by ether recrystallization. When the purity level reached 99.8% or more, sublimation purification was performed thereon. 3.3 g of the obtained compound was obtained (Purity ≥99.9%).

### Synthesis Example 3: Synthesis of Compound 5

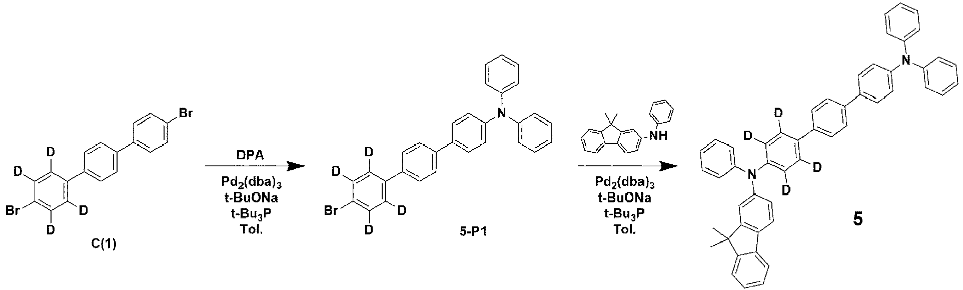

Synthesis of 5-P1: Intermediate C(1) (1 eq, 3.92 g), DPA (1 eq, 1.69 g), Pd₂(dba)₃ (0.03 eq, 0.27 g), t-BuONa (1.5 eq, 1.44 g), t-Bu₃P (0.05 eq, 0.06 ml), and 100 ml of ToI. were added to a one-neck round flask and stirred at 100 °C for 2 hours. The resultant reaction product was worked up with EA/H₂O and separated using column chromatography (MC/Hx = 1/5). 3.36 g of the obtained compound (5-P1) was obtained (Yield = 70%, Purity = 99%).

Synthesis of Compound 5: 5-P1 (1 eq, 3.36 g), 9,9-dimethyl-N-phenyl-9H-fluoren-2-amine (1 eq, 1.99 g), Pd₂(dba)₃ (0.03 eq, 0.189 g), t-BuONa (1.5 eq, 1 g), t-Bu₃P (0.05 eq, 0.04 ml), and 100 ml of ToI. were added to a one-neck round flask and stirred at 100 °C for 2 hours. The resultant reaction product was worked up with EA/H₂O and separated using column chromatography (MC/Hx = 1/5). 3.4 g of the obtained compound (Compound 5) was obtained (Yield = 75%, Purity = 98%). Purity of the obtained product was increased by ether recrystallization. When the purity level reached 99.8% or more, sublimation purification was performed thereon. 3 g of the obtained compound was obtained (Purity ≥99.9%).

### Synthesis Example 4: Synthesis of Compound 11

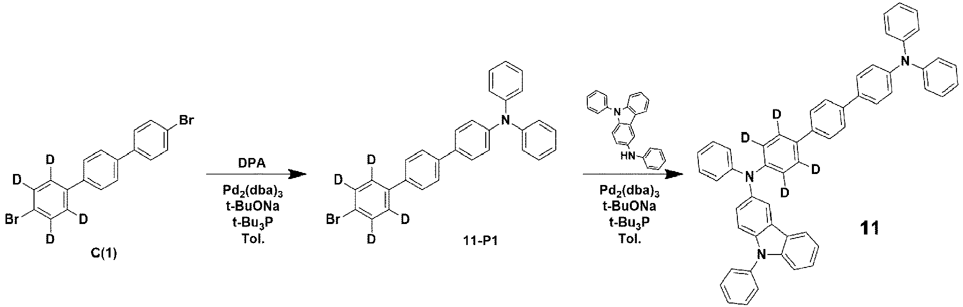

Synthesis of 11-P1: Intermediate C(1) (1 eq, 3.92 g), DPA (1 eq, 1.69 g), Pd₂(dba)₃ (0.03 eq, 0.27 g), t-BuONa (1.5 eq, 1.44 g), t-Bu₃P (0.05 eq, 0.06 ml), and 100 ml of ToI. were added to a one-neck round flask and stirred at 100 °C for 2 hours. The resultant reaction product was worked up with EA/H₂O and separated using column chromatography (MC/Hx = 1/5). 3.36 g of the obtained compound (11-P1) was obtained (Yield = 70%, Purity = 99%).

Synthesis of Compound 11: 11-P1 (1 eq, 3.36 g), N,9-diphenyl-9H-carbazol-3-amine (1 eq, 2.34 g), Pd₂(dba)₃ (0.03 eq, 0.189 g), t-BuONa (1.5 eq, 1 g), t-Bu₃P (0.05 eq, 0.04 ml), and 100 ml of ToI. were added to a one-neck round flask and stirred at 100 °C for 2 hours. The resultant reaction product was worked up with EA/H₂O and separated using column chromatography (MC/Hx = 1/5). 2.86 g of the obtained compound (Compound 11) was obtained (Yield = 63%, Purity = 98%). Purity of the obtained product was increased by ether recrystallization. When the purity level reached 99.8% or more, sublimation purification was performed thereon. 2.5 g of the obtained compound was obtained (Purity ≥99.9%).

### Synthesis Example 5: Synthesis of Compound 14

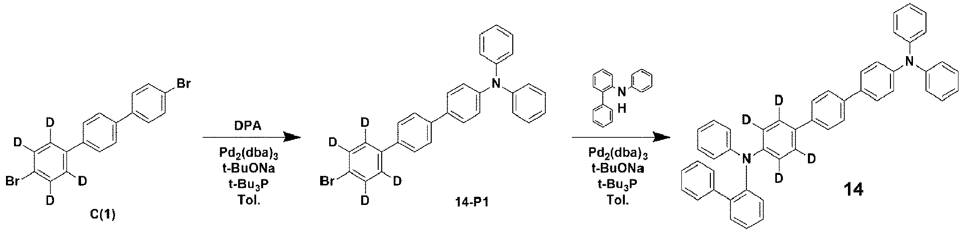

Synthesis of 14-P1: Intermediate C(1) (1 eq, 3.92 g), DPA (1 eq, 1.69 g), Pd₂(dba)₃ (0.03 eq, 0.27 g), t-BuONa (1.5 eq, 1.44 g), t-Bu₃P (0.05 eq, 0.06 ml), and 100 ml of ToI. were added to a one-neck round flask and stirred at 100 °C for 2 hours. The resultant reaction product was worked up with EA/H₂O and separated using column chromatography (MC/Hx = 1/5). 3.36 g of the obtained compound (14-P1) was obtained (Yield = 70%, Purity = 99%).

Synthesis of Compound 14: 14-P1 (1 eq, 3.36 g), N-phenyl-[1,1'-biphenyl]-2-amine (1 eq, 1.7 g), Pd₂(dba)₃ (0.03 eq, 0.189 g), t-BuONa (1.5 eq, 1 g), t-Bu₃P (0.05 eq, 0.04 ml), and 100 ml of ToI. were added to a one-neck round flask and stirred at 100 °C for 2 hours. The resultant reaction product was worked up with EA/H₂O and separated using column chromatography (MC/Hx = 1/5). 3.58 g of the obtained compound (Compound 14) was obtained (Yield = 79%, Purity = 97%). Purity of the obtained product was increased by ether recrystallization. When the purity level reached 99.8% or more, sublimation purification was performed thereon. 3.3 g of the obtained compound was obtained (Purity ≥99.9%).

### Synthesis Example 6: Synthesis of Compound 16

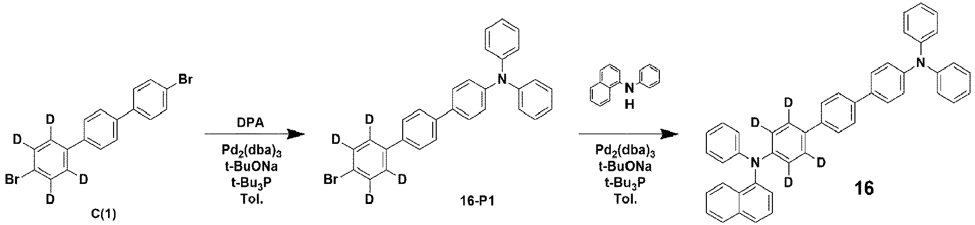

Synthesis of 16-P1: Intermediate C(1) (1 eq, 3.92 g), DPA (1 eq, 1.69 g), Pd₂(dba)₃ (0.03 eq, 0.27 g), t-BuONa (1.5 eq, 1.44 g), t-Bu₃P (0.05 eq, 0.06 ml), and 100 ml of ToI. were added to a one-neck round flask and stirred at 100 °C for 2 hours. The resultant reaction product was worked up with EA/H₂O and separated using column chromatography (MC/Hx = 1/5). 3.36 g of the obtained compound (16-P1) was obtained (Yield = 70%, Purity = 99%).

Synthesis of Compound 16: 16-P1 (1 eq, 3.36 g), N-phenylnaphthalen-1-amine (1 eq, 1.53 g), Pd₂(dba)₃ (0.03 eq, 0.189 g), t-BuONa (1.5 eq, 1 g), t-Bu₃P (0.05 eq, 0.04 ml), and 100 ml of ToI. were added to a one-neck round flask and stirred at 100 °C for 2 hours. The resultant reaction product was worked up with EA/H₂O and separated using column chromatography MC/Hx = 1/5). 3.76 g of the obtained compound (Compound 16) was obtained (Yield = 83%, Purity = 99%). Purity of the obtained product was increased by ether recrystallization. When the purity level reached 99.8% or more, sublimation purification was performed thereon. 3.5 g of the obtained compound was obtained (Purity ≥99.9%).

### Synthesis Example 7: Synthesis of Compound 17

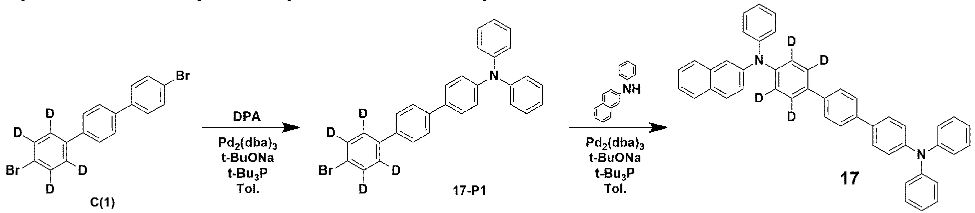

Synthesis of 17-P1: Intermediate C(1) (1 eq, 3.92 g), DPA (1 eq, 1.69 g), Pd₂(dba)₃ (0.03 eq, 0.27 g), t-BuONa (1.5 eq, 1.44 g), t-Bu₃P (0.05 eq, 0.06 ml), and 100 ml of ToI. were added to a one-neck round flask and stirred at 100 °C for 2 hours. The resultant reaction product was worked up with EA/H₂O and separated using column chromatography (MC/Hx = 1/5). 3.36 g of the obtained compound (17-P1) was obtained (Yield = 70%, Purity = 99%).

Synthesis of Compound 17: 17-P1 (1 eq, 3.36 g), N-phenylnaphthalen-1-amine (1 eq, 1.53 g), Pd₂(dba)₃ (0.03 eq, 0.189 g), t-BuONa (1.5 eq, 1 g), t-Bu₃P (0.05 eq, 0.04 ml), and 100 ml of ToI. were added to a one-neck round flask and stirred at 100 °C for 2 hours. The resultant reaction product was worked up with EA/H₂O and separated using column chromatography (MC/Hx = 1/5). 3.76 g of the obtained compound (Compound 17) was obtained (Yield = 83%, Purity = 99%). Purity of the obtained product was increased by ether recrystallization. When the purity level reached 99.8% or more, sublimation purification was performed thereon. 3.5 g of the obtained compound was obtained (Purity ≥99.9%).

### Synthesis Example 8: Synthesis of Compound 19

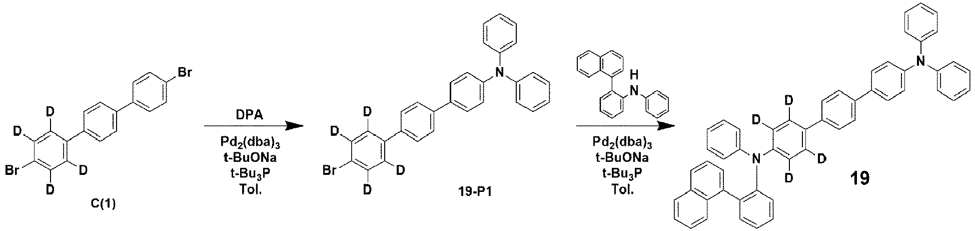

Synthesis of 19-P1: Intermediate C(1) (1 eq, 3.92 g), DPA (1 eq, 1.69 g), Pd₂(dba)₃ (0.03 eq, 0.27 g), t-BuONa (1.5 eq, 1.44 g), t-Bu₃P (0.05 eq, 0.06 ml), and 100 ml of ToI. were added to a one-neck round flask and stirred at 100 °C for 2 hours. The resultant reaction product was worked up with EA/H₂O and separated using column chromatography (MC/Hx = 1/5). 3.36 g of the obtained compound (19-P1) was obtained (Yield = 70%, Purity = 99%).

Synthesis of Compound 19: 19-P1 (1 eq, 3.36 g), 2-(naphthalen-1-yl)-N-phenylaniline (1 eq, 2.06 g), Pd₂(dba)₃ (0.03 eq, 0.189 g), t-BuONa (1.5 eq, 1 g), t-Bu₃P (0.05 eq, 0.04 ml), and 100 ml of ToI. were added to a one-neck round flask and stirred at 100 °C for 2 hours. The resultant reaction product was worked up with EA/H₂O and separated using column chromatography (MC/Hx = 1/5). 3.62 g of the obtained compound (Compound 19) was obtained (Yield = 80%, Purity = 99%). Purity of the obtained product was increased by ether recrystallization. When the purity level reached 99.8% or more, sublimation purification was performed thereon. 3.42 g of the obtained compound was obtained (Purity ≥99.9%).

### Synthesis Example 9: Synthesis of Compound 25

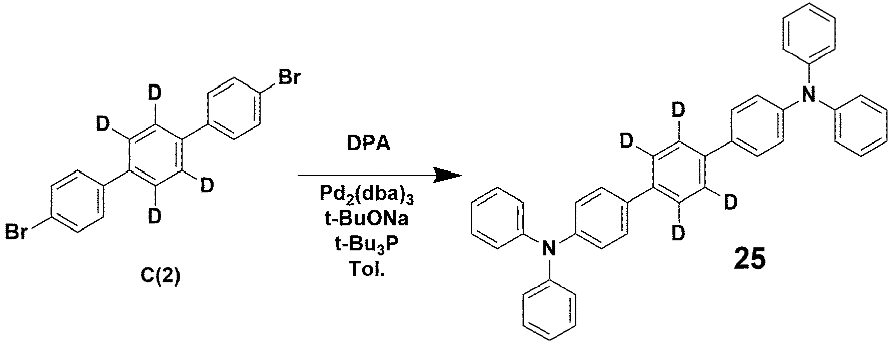

Intermediate C(2) (1 eq, 3.92 g), DPA (2.2 eq, 3.72 g), Pd₂(dba)₃ (0.05 eq, 0.46 g), t-BuONa (3 eq, 2.88 g), t-Bu₃P (0.1 eq, 0.11 ml), and 100 ml of ToI. were added to a one-neck round flask and stirred at 100 °C for 2 hours. The resultant reaction product was worked up with EA/H₂O and separated using column chromatography (MC/Hx = 1/5). 4.25 g of the obtained compound (Compound 25) was obtained (Yield = 75%, Purity = 98%). Purity of the obtained product was increased by ether recrystallization. When the purity level reached 99.7% or more, sublimation purification was performed thereon. 4 g of the obtained compound was obtained (Purity>=99.9%).

### Synthesis Example 10: Synthesis of Compound 26

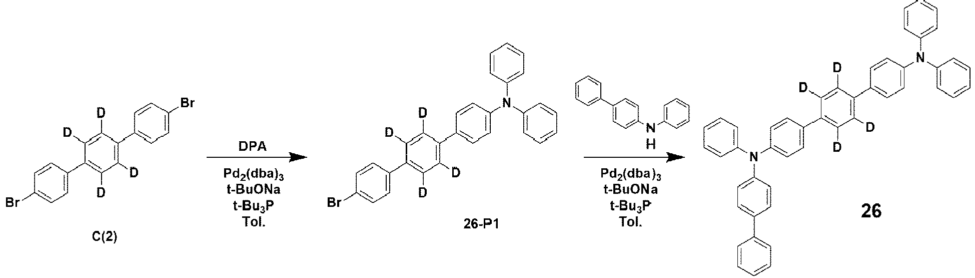

Synthesis of 26-P1: Intermediate C(2) (1 eq, 3.92 g), DPA (1 eq, 1.69 g), Pd₂(dba)₃ (0.03 eq, 0.27 g), t-BuONa (1.5 eq, 1.44 g), t-Bu₃P (0.05 eq, 0.06 ml), and 100 ml of ToI. were added to a one-neck round flask and stirred at 100 °C for 2 hours. The resultant reaction product was worked up with EA/H₂O and separated using column chromatography (MC/Hx = 1/5). 3.84 g of the obtained compound (26-P1) was obtained (Yield = 80%, Purity = 99%).

Synthesis of Compound 26: 26-P1 (1 eq, 3.84 g), N-phenyl-[1,1'-biphenyl]-4-amine (1 eq, 1.95 g), Pd₂(dba)₃ (0.03 eq, 0.216 g), t-BuONa (1.5 eq, 1.14 g), t-Bu₃P (0.05 eq, 0.046 ml), and 100 ml of ToI. were added to a one-neck round flask and stirred at 100 °C for 2 hours. The resultant reaction product was worked up with EA/H₂O and separated using column chromatography (MC/Hx = 1/5). 4.23 g of the obtained compound (Compound 26) was obtained (Yield = 82%, Purity = 98%). Purity of the obtained product was increased by ether recrystallization. When the purity level reached 99.6% or more, sublimation purification was performed thereon. 3.9 g of the obtained compound was obtained (Purity ≥99.9%).

### Synthesis Example 11: Synthesis of Compound 29

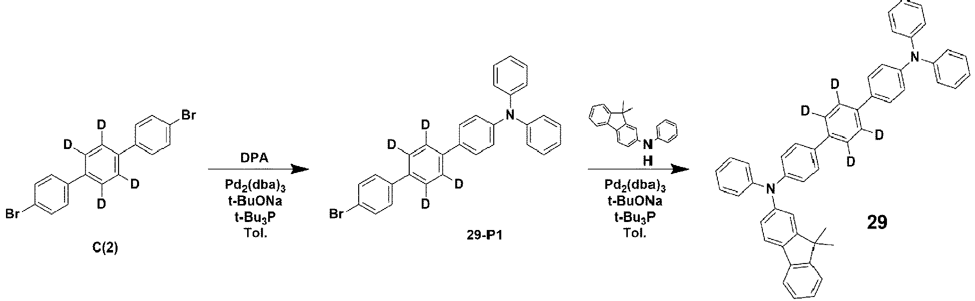

Synthesis of 29-P1: Intermediate C(2) (1 eq, 3.92 g), DPA (1 eq, 1.69 g), Pd₂(dba)₃ (0.03 eq, 0.27 g), t-BuONa (1.5 eq, 1.44 g), t-Bu₃P (0.05 eq, 0.06 ml), and 100 ml of ToI. were added to a one-neck round flask and stirred at 100 °C for 2 hours. The resultant reaction product was worked up with EA/H₂O and separated using column chromatography (MC/Hx = 1/5). 3.84 g of the obtained compound (29-P1) was obtained (Yield = 80%, Purity = 99%).

Synthesis of Compound 29: 29-P1 (1 eq, 3.84 g), 9,9-dimethyl-N-phenyl-9H-fluoren-2-amine (1 eq, 2.28 g), Pd₂(dba)₃ (0.03 eq, 0.216 g), t-BuONa (1.5 eq, 1.14 g), t-Bu₃P (0.05 eq, 0.046 ml), and 100 ml of ToI. were added to a one-neck round flask and stirred at 100 °C for 2 hours. The resultant reaction product was worked up with EA/H₂O and separated using column chromatography (MC/Hx = 1/5). 4.13 g of the obtained compound (Compound 29) was obtained (Yield = 80%, Purity = 98%). Purity of the obtained product was increased by ether recrystallization. When the purity level reached 99.6% or more, sublimation purification was performed thereon. 3.7 g of the obtained compound was obtained (Purity ≥99.9%).

### Synthesis Example 12: Synthesis of Compound 38

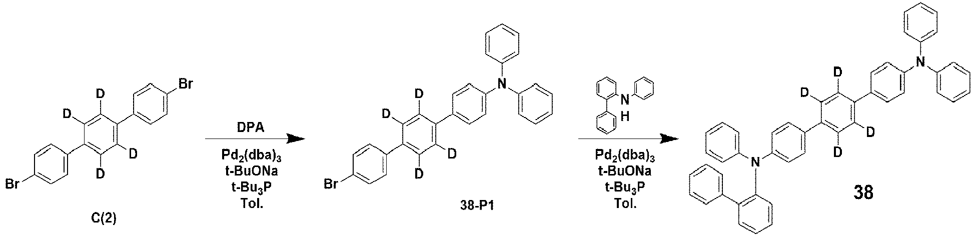

Synthesis of 38-P1: Intermediate C(2) (1 eq, 3.92 g), DPA (1 eq, 1.69 g), Pd₂(dba)₃ (0.03 eq, 0.27 g), t-BuONa (1.5 eq, 1.44 g), t-Bu₃P (0.05 eq, 0.06 ml), and 100 ml of ToI. were added to a one-neck round flask and stirred at 100 °C for 2 hours. The resultant reaction product was worked up with EA/H₂O and separated using column chromatography (MC/Hx = 1/5). 3.84 g of the obtained compound (38-P1) was obtained (Yield = 80%, Purity = 99%).

Synthesis of Compound 38: 38-P1 (1 eq, 3.84 g), N-phenyl-[1,1'-biphenyl]-2-amine (1 eq, 1.95 g), Pd₂(dba)₃ (0.03 eq, 0.216 g), t-BuONa (1.5 eq, 1.14 g), t-Bu₃P (0.05 eq, 0.046 ml), and 100 ml of ToI. were added to a one-neck round flask and stirred at 100 °C for 2 hours. The resultant reaction product was worked up with EA/H₂O and separated using column chromatography (MC/Hx = 1/5). 3.97 g of the obtained compound (Compound 38) was obtained (Yield = 77%, Purity = 99%). Purity of the obtained product was increased by ether recrystallization. When the purity level reached 99.6% or more, sublimation purification was performed thereon. 3.65 g of the obtained compound was obtained (Purity ≥99.9%).

### Synthesis Example 13: Synthesis of Compound 40

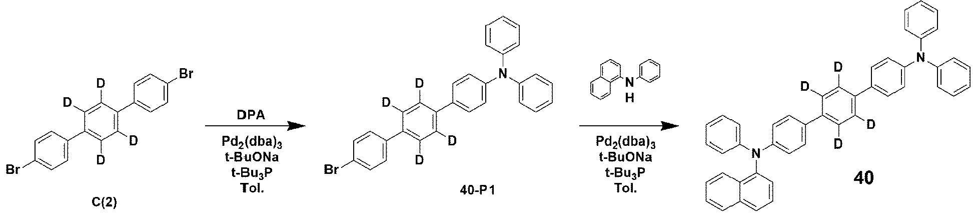

Synthesis of 40-P1: Intermediate C(2) (1 eq, 3.92 g), DPA (1 eq, 1.69 g), Pd₂(dba)₃ (0.03 eq, 0.27 g), t-BuONa (1.5 eq, 1.44 g), t-Bu₃P (0.05 eq, 0.06 ml), and 100 ml of ToI. were added to a one-neck round flask and stirred at 100 °C for 2 hours. The resultant reaction product was worked up with EA/H₂O and separated using column chromatography (MC/Hx = 1/5). 3.36 g of the obtained compound (40-P1) was obtained (Yield = 70%, Purity = 99%).

Synthesis of Compound 40: 40-P1 (1 eq, 3.36 g), N-phenylnaphthalen-1-amine (1 eq, 1.53 g), Pd₂(dba)₃ (0.03 eq, 0.189 g), t-BuONa (1.5 eq, 1 g), t-Bu₃P (0.05 eq, 0.04 ml), and 100 ml of ToI. were added to a one-neck round flask and stirred at 100 °C for 2 hours. The resultant reaction product was worked up with EA/H₂O and separated using column chromatography (MC/Hx = 1/5). 3.76 g of the obtained compound (Compound 40) was obtained (Yield = 83%, Purity = 99%). Purity of the obtained product was increased by ether recrystallization. When the purity level reached 99.8% or more, sublimation purification was performed thereon. 3.5 g of the obtained compound was obtained (Purity ≥99.9%).

### Synthesis Example 14: Synthesis of Compound 41

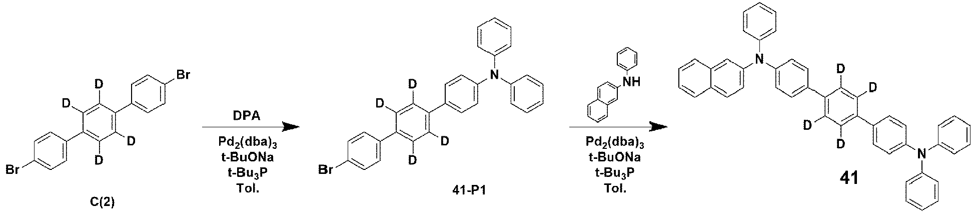

Synthesis of 41-P1: Intermediate C(2) (1 eq, 3.92 g), DPA (1 eq, 1.69 g), Pd₂(dba)₃ (0.03 eq, 0.27 g), t-BuONa (1.5 eq, 1.44 g), t-Bu₃P (0.05 eq, 0.06 ml), and 100 ml of ToI. were added to a one-neck round flask and stirred at 100 °C for 2 hours. The resultant reaction product was worked up with EA/H₂O and separated using column chromatography (MC/Hx = 1/5). 3.36 g of the obtained compound was obtained (Yield = 70%, Purity = 99%).

Synthesis of Compound 41: 41-P1 (1 eq, 3.36 g), N-phenylnaphthalen-1 - amine (1 eq, 1.53 g), Pd₂(dba)₃ (0.03 eq, 0.189 g), t-BuONa (1.5 eq, 1 g), t-Bu₃P (0.05 eq, 0.04 ml), and 100 ml of ToI. were added to a one-neck round flask and stirred at 100 °C for 2 hours. The resultant reaction product was worked up with EA/H₂O and separated using column chromatography (MC/Hx = 1/5). 3.76 g of the obtained compound (Compound 41) was obtained (Yield = 83%, Purity = 99%). Purity of the obtained product was increased by ether recrystallization. When the purity level reached 99.8% or more, sublimation purification was performed thereon. 3.5 g of the obtained compound was obtained (Purity ≥99.9%).

### Synthesis Example 15: Synthesis of Compound 49

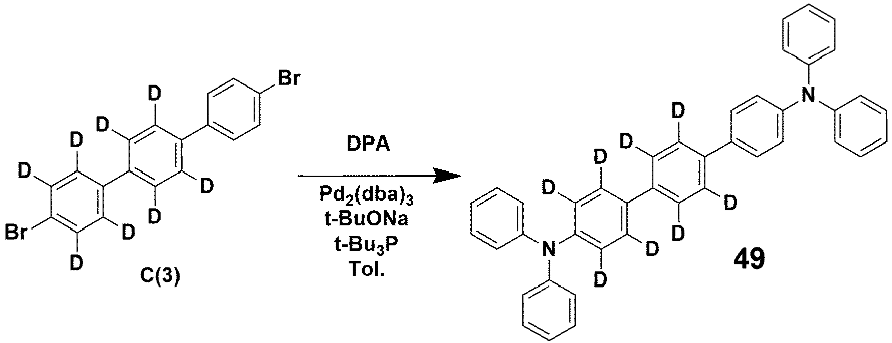

Intermediate C(3) (1 eq, 3.96 g), DPA (2.2 eq, 3.72 g), Pd₂(dba)₃ (0.05 eq, 0.46 g), t-BuONa (3 eq, 2.88 g), t-Bu₃P (0.1 eq, 0.11 ml), and 100 ml of ToI. were added to a one-neck round flask and stirred at 100 °C for 2 hours. The resultant reaction product was worked up with EA/H₂O and separated using column chromatography (MC/Hx = 1/5). 4.34 g of the obtained compound (Compound 49) was obtained (Yield = 76%, Purity = 98%). Purity of the obtained product was increased by ether recrystallization. When the purity level reached 99.7% or more, sublimation purification was performed thereon. 4.1 g of the obtained compound was obtained (Purity ≥99.9%).

### Synthesis Example 16: Synthesis of Compound 50

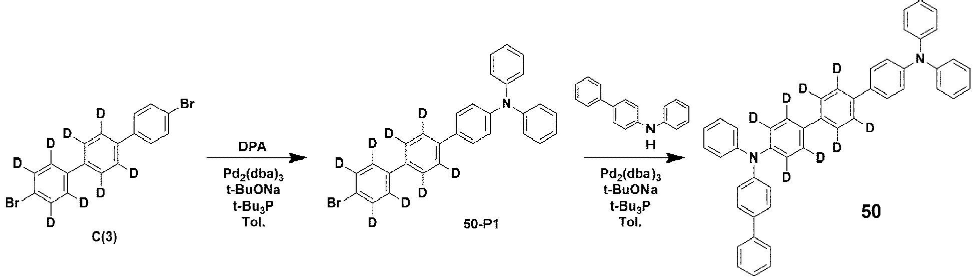

Synthesis of 50-P1: Intermediate C(3) (1 eq, 3.96 g), DPA (1 eq, 1.69 g), Pd₂(dba)₃ (0.03 eq, 0.27 g), t-BuONa (1.5 eq, 1.44 g), t-Bu₃P (0.05 eq, 0.06 ml), and 100 ml of ToI. were added to a one-neck round flask and stirred at 100 °C for 2 hours. The resultant reaction product was worked up with EA/H₂O and separated using column chromatography (MC/Hx = 1/5). 3.87 g of the obtained compound (50-P1) was obtained (Yield = 80%, Purity = 99%).

Synthesis of Compound 50: 50-P1 (1 eq, 3.87 g), N-phenyl-[1,1'-biphenyl]-4-amine (1 eq, 1.95 g), Pd₂(dba)₃ (0.03 eq, 0.216 g), t-BuONa (1.5 eq, 1.14 g), t-Bu₃P (0.05 eq, 0.046 ml), and 100 ml of ToI. were added to a one-neck round flask and stirred at 100 °C for 2 hours. The resultant reaction product was worked up with EA/H₂O and separated using column chromatography (MC/Hx = 1/5). 4 g of the obtained compound (Compound 50) was obtained (Yield = 77%, Purity = 98.5%). Purity of the obtained product was increased by ether recrystallization. When the purity level reached 99.7% or more, sublimation purification was performed thereon. 3.6 g of the obtained compound was obtained (Purity ≥99.9%).

### Synthesis Example 17: Synthesis of Compound 53

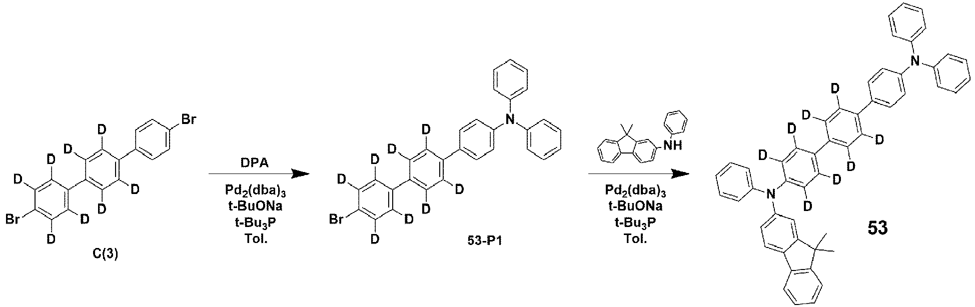

Synthesis of 53-P1: Intermediate C(3) (1 eq, 3.96 g), DPA (1 eq, 1.69 g), Pd₂(dba)₃ (0.03 eq, 0.27 g), t-BuONa (1.5 eq, 1.44 g), t-Bu₃P (0.05 eq, 0.06 ml), and 100 ml of ToI. were added to a one-neck round flask and stirred at 100 °C for 2 hours. The resultant reaction product was worked up with EA/H₂O and separated using column chromatography (MC/Hx = 1/5). 3.87 g of the obtained compound (53-P1) was obtained (Yield = 80%, Purity = 99%).

Synthesis of Compound 53: 53-P1 (1 eq, 3.87 g), 9,9-dimethyl-N-phenyl-9H-fluoren-2-amine (1 eq, 2.28 g), Pd₂(dba)₃ (0.03 eq, 0.216 g), t-BuONa (1.5 eq, 1.14 g), t-Bu₃P (0.05 eq, 0.046 ml), and 100 ml of ToI. were added to a one-neck round flask and stirred at 100 °C for 2 hours. The resultant reaction product was worked up with EA/H₂O and separated using column chromatography (MC/Hx = 1/5). 4.2 g of the obtained compound (Compound 53) was obtained (Yield = 81%, Purity = 98.9%). Purity of the obtained product was increased by ether recrystallization. When the purity level reached 99.7% or more, sublimation purification was performed thereon. 3.8 g of the obtained compound was obtained (Purity ≥99.9%).

### Synthesis Example 18: Synthesis of Compound 62

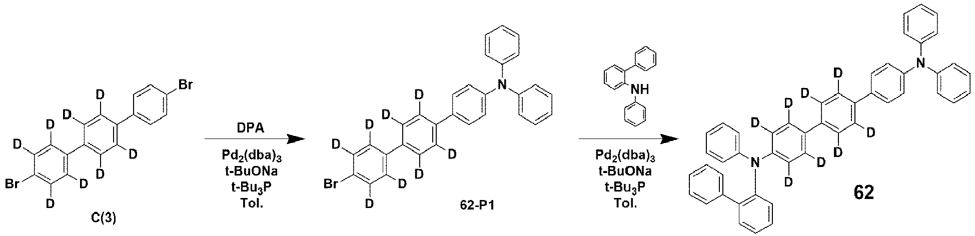

Synthesis of 62-P1: Intermediate C(3) (1 eq, 3.96 g), DPA (1 eq, 1.69 g), Pd₂(dba)₃ (0.03 eq, 0.27 g), t-BuONa (1.5 eq, 1.44 g), t-Bu₃P (0.05 eq, 0.06 ml), and 100 ml of ToI. were added to a one-neck round flask and stirred at 100 °C for 2 hours. The resultant reaction product was worked up with EA/H₂O and separated using column chromatography (MC/Hx = 1/5). 3.87 g of the obtained compound (62-P1) was obtained (Yield = 80%, Purity = 99%).

Synthesis of Compound 62: 62-P1 (1 eq, 3.87 g), N-phenyl-[1,1'-biphenyl]-2-amine (1 eq, 1.95 g), Pd₂(dba)₃ (0.03 eq, 0.216 g), t-BuONa (1.5 eq, 1.14 g), t-Bu₃P (0.05 eq, 0.046 ml), and 100 ml of ToI. were added to a one-neck round flask and stirred at 100 °C for 2 hours. The resultant reaction product was worked up with EA/H₂O and separated using column chromatography (MC/Hx = 1/5). 4 g of the obtained compound (Compound 62) was obtained (Yield = 77%, Purity = 99%). Purity of the obtained product was increased by ether recrystallization. When the purity level reached 99.88% or more, sublimation purification was performed thereon. 3.6 g of the obtained compound was obtained (Purity ≥99.9%).

### Synthesis Example 19: Synthesis of Compound 64

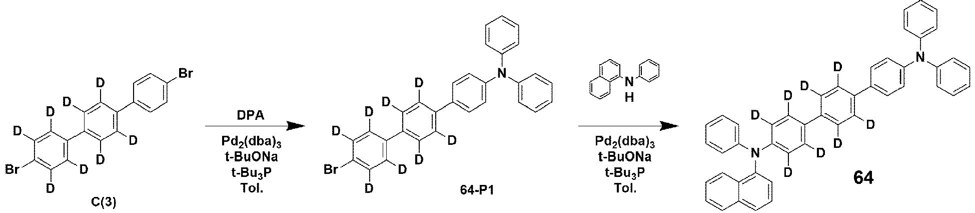

Synthesis of 64-P1: Intermediate C(3) (1 eq, 3.92 g), DPA (1 eq, 1.69 g), Pd₂(dba)₃ (0.03 eq, 0.27 g), t-BuONa (1.5 eq, 1.44 g), t-Bu₃P (0.05 eq, 0.06 ml), and 100 ml of ToI. were added to a one-neck round flask and stirred at 100 °C for 2 hours. The resultant reaction product was worked up with EA/H₂O and separated using column chromatography (MC/Hx = 1/5). 3.36 g of the obtained compound (64-P1) was obtained (Yield = 70%, Purity = 99%).

Synthesis of Compound 64: 64-P1 (1 eq, 3.36 g), N-phenylnaphthalen-1-amine (1 eq, 1.53 g), Pd₂(dba)₃ (0.03 eq, 0.189 g), t-BuONa (1.5 eq, 1 g), t-Bu₃P (0.05 eq, 0.04 ml), and 100 ml of ToI. were added to a one-neck round flask and stirred at 100 °C for 2 hours. The resultant reaction product was worked up with EA/H₂O and separated using column chromatography (MC/Hx = 1/5). 3.76 g of the obtained compound (Compound 64) was obtained (Yield = 83%, Purity = 99%). Purity of the obtained product was increased by ether recrystallization. When the purity level reached 99.8% or more, sublimation purification was performed thereon. 3.5 g of the obtained compound was obtained (Purity ≥99.9%).

### Synthesis Example 20: Synthesis of Compound 65

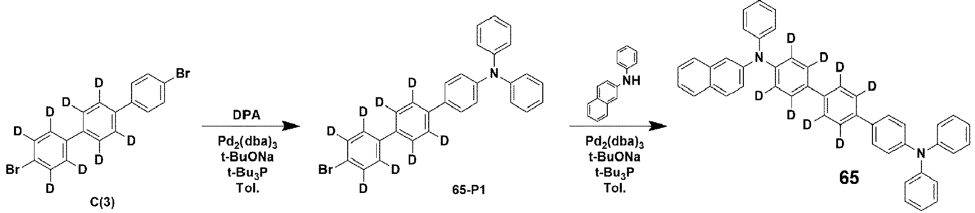

Synthesis of 65-P1: Intermediate C(3) (1 eq, 3.92 g), DPA (1 eq, 1.69 g), Pd₂(dba)₃ (0.03 eq, 0.27 g), t-BuONa (1.5 eq, 1.44 g), t-Bu₃P (0.05 eq, 0.06 ml), and 100 ml of ToI. were added to a one-neck round flask and stirred at 100 °C for 2 hours. The resultant reaction product was worked up with EA/H₂O and separated using column chromatography (MC/Hx = 1/5). 3.36 g of the obtained compound (65-P1) was obtained (Yield = 70%, Purity = 99%).

Synthesis of Compound 65: 65-P1 (1 eq, 3.36 g), N-phenylnaphthalen-1-amine (1 eq, 1.53 g), Pd₂(dba)₃ (0.03 eq, 0.189 g), t-BuONa (1.5 eq, 1 g), t-Bu₃P (0.05 eq, 0.04 ml), and 100 ml of ToI. were added to a one-neck round flask and stirred at 100 °C for 2 hours. The resultant reaction product was worked up with EA/H₂O and separated using column chromatography (MC/Hx = 1/5). 3.76 g of the obtained compound (Compound 65) was obtained (Yield = 83%, Purity = 99%). Purity of the obtained product was increased by ether recrystallization. When the purity level reached 99.8% or more, sublimation purification was performed thereon. 3.5 g of the obtained compound was obtained (Purity ≥99.9%).

### Synthesis Example 21: Synthesis of Compound 73

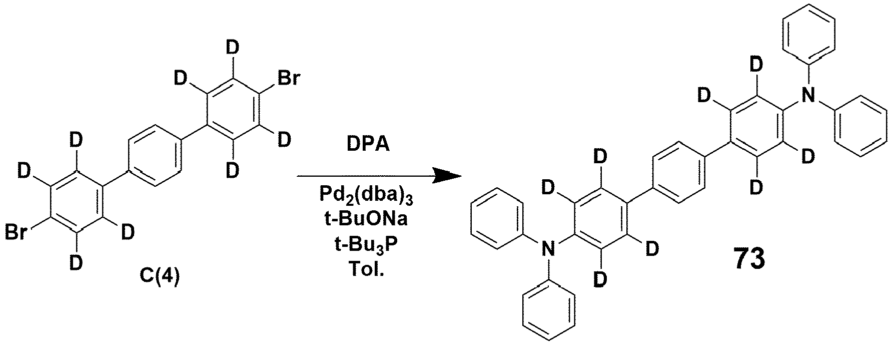

Intermediate C(4) (1 eq, 3.96 g), DPA (2.2 eq, 3.72 g), Pd₂(dba)₃ (0.05 eq, 0.46 g), t-BuONa (3 eq, 2.88 g), t-Bu₃P (0.1 eq, 0.11 ml), and 100 ml of ToI. were added to a one-neck round flask and stirred at 100 °C for 2 hours. The resultant reaction product was worked up with EA/H₂O and separated using column chromatography (MC/Hx = 1/5). 4 g of the obtained compound (Compound 73) was obtained (Yield = 70%, Purity = 99.2%). Purity of the obtained product was increased by ether recrystallization. When the purity level reached 99.65% or more, sublimation purification was performed thereon. 3.7 g of the obtained compound was obtained (Purity ≥99.9%).

### Synthesis Example 22: Synthesis of Compound 74

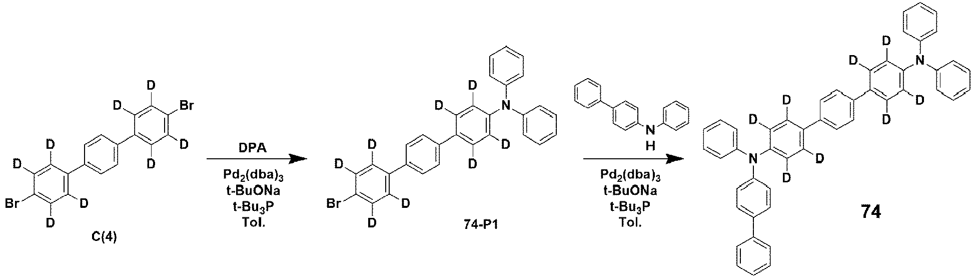

Synthesis of 74-P1: Intermediate C(4) (1 eq, 3.96 g), DPA (1 eq, 1.69 g), Pd₂(dba)₃ (0.03 eq, 0.27 g), t-BuONa (1.5 eq, 1.44 g), t-Bu₃P (0.05 eq, 0.06 ml), and 100 ml of ToI. were added to a one-neck round flask and stirred at 100 °C for 2 hours. The resultant reaction product was worked up with EA/H₂O and separated using column chromatography (MC/Hx = 1/5). 3.87 g of the obtained compound (74-P1) was obtained (Yield = 80%, Purity = 99%).

Synthesis of Compound 74: 74-P1 (1 eq, 3.87 g), N-phenyl-[1,1'-biphenyl]-4-amine (1 eq, 1.95 g), Pd₂(dba)₃ (0.03 eq, 0.216 g), t-BuONa (1.5 eq, 1.14 g), t-Bu₃P (0.05 eq, 0.046 ml), and 100 ml of ToI. were added to a one-neck round flask and stirred at 100 °C for 2 hours. The resultant reaction product was worked up with EA/H₂O and separated using column chromatography (MC/Hx = 1/5). 4.36 g of the obtained compound (Compound 74) was obtained (Yield = 84%, Purity = 97%). Purity of the obtained product was increased by ether recrystallization. When the purity level reached 99.5% or more, sublimation purification was performed thereon. 4.1 g of the obtained compound was obtained (Purity ≥99.9%).

### Synthesis Example 23: Synthesis of Compound 77

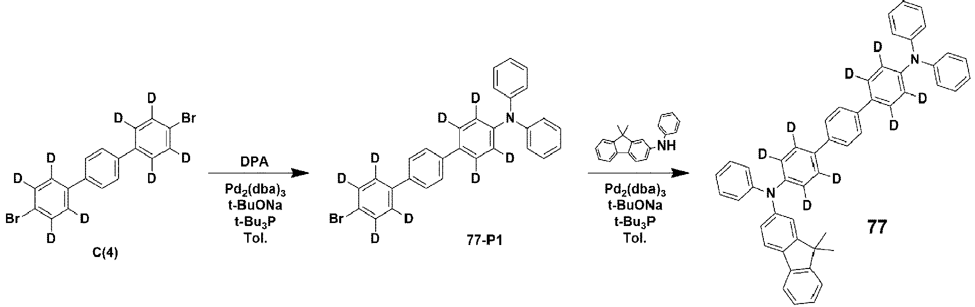

Synthesis of 77-P1: Intermediate C(4) (1 eq, 3.96 g), DPA (1 eq, 1.69 g), Pd₂(dba)₃ (0.03 eq, 0.27 g), t-BuONa (1.5 eq, 1.44 g), t-Bu₃P (0.05 eq, 0.06 ml), and 100 ml of ToI. were added to a one-neck round flask and stirred at 100 °C for 2 hours. The resultant reaction product was worked up with EA/H₂O and separated using column chromatography (MC/Hx = 1/5). 3.87 g of the obtained compound (77-P1) was obtained (Yield = 80%, Purity = 99%).

Synthesis of Compound 77: 77-P1 (1 eq, 3.87 g), 9,9-dimethyl-N-phenyl-9H-fluoren-2-amine (1 eq, 2.28 g), Pd₂(dba)₃ (0.03 eq, 0.216 g), t-BuONa (1.5 eq, 1.14 g), t-Bu₃P (0.05 eq, 0.046 ml), and 100 ml of ToI. were added to a one-neck round flask and stirred at 100 °C for 2 hours. The resultant reaction product was worked up with EA/H₂O and separated using column chromatography (MC/Hx = 1/5). 4.2 g of the obtained compound (Compound 77) was obtained (Yield = 81%, Purity = 99%). Purity of the obtained product was increased by ether recrystallization. When the purity level reached 99.8% or more, sublimation purification was performed thereon. 3.85 g of the obtained compound was obtained (Purity ≥99.9%).

### Synthesis Example 24: Synthesis of Compound 86

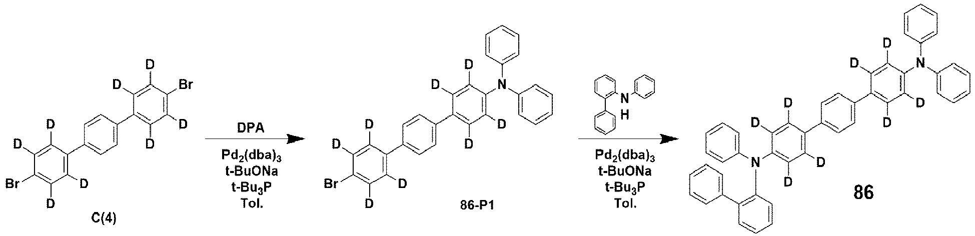

Synthesis of 86-P1: Intermediate C(4) (1 eq, 3.96 g), DPA (1 eq, 1.69 g), Pd₂(dba)₃ (0.03 eq, 0.27 g), t-BuONa (1.5 eq, 1.44 g), t-Bu₃P (0.05 eq, 0.06 ml), and 100 ml of ToI. were added to a one-neck round flask and stirred at 100 °C for 2 hours. The resultant reaction product was worked up with EA/H₂O and separated using column chromatography (MC/Hx = 1/5). 3.87 g of the obtained compound (86-P1) was obtained (Yield = 80%, Purity = 99%).

Synthesis of Compound 86: 86-P1 (1 eq, 3.87 g), N-phenyl-[1,1'-biphenyl]-2-amine (1 eq, 1.95 g), Pd₂(dba)₃ (0.03 eq, 0.216 g), t-BuONa (1.5 eq, 1.14 g), t-Bu₃P (0.05 eq, 0.046 ml), and 100 ml of ToI. were added to a one-neck round flask and stirred at 100 °C for 2 hours. The resultant reaction product was worked up with EA/H₂O and separated using column chromatography (MC/Hx = 1/5). 4.36 g of the obtained compound (Compound 86) was obtained (Yield = 84%, Purity = 98.1%). Purity of the obtained product was increased by ether recrystallization. When the purity level reached 99% or more, sublimation purification was performed thereon. 4 g of the obtained compound was obtained (Purity ≥99.9%).

### Synthesis Example 25: Synthesis of Compound 88

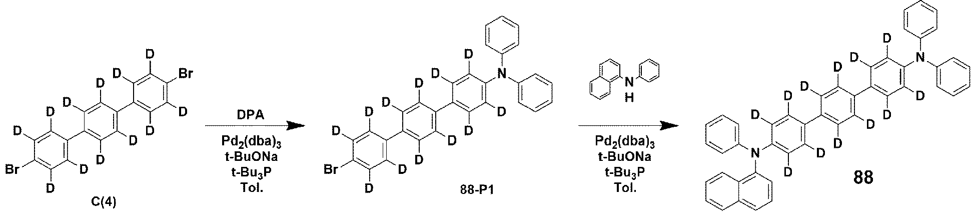

Synthesis of 88-P1: Intermediate C(4) (1 eq, 3.92 g), DPA (1 eq, 1.69 g), Pd₂(dba)₃ (0.03 eq, 0.27 g), t-BuONa (1.5 eq, 1.44 g), t-Bu₃P (0.05 eq, 0.06 ml), and 100 ml of ToI. were added to a one-neck round flask and stirred at 100 °C for 2 hours. The resultant reaction product was worked up with EA/H₂O and separated using column chromatography (MC/Hx = 1/5). 3.36 g of the obtained compound (88-P1) was obtained (Yield = 70%, Purity = 99%).

Synthesis of Compound 88: 88-P1 (1 eq, 3.36 g), N-phenylnaphthalen-1-amine (1 eq, 1.53 g), Pd₂(dba)₃ (0.03 eq, 0.189 g), t-BuONa (1.5 eq, 1 g), t-Bu₃P (0.05 eq, 0.04 ml), and 100 ml of ToI. were added to a one-neck round flask and stirred at 100 °C for 2 hours. The resultant reaction product was worked up with EA/H₂O and separated using column chromatography (MC/Hx = 1/5). 3.76 g of the obtained compound (Compound 88) was obtained (Yield = 83%, Purity = 99%). Purity of the obtained product was increased by ether recrystallization. When the purity level reached 99.8% or more, sublimation purification was performed thereon. 3.5 g of the obtained compound was obtained (Purity ≥99.9%).

### Synthesis Example 26: Synthesis of Compound 89

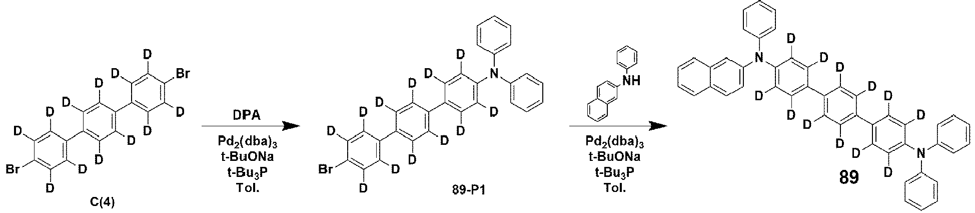

Synthesis of 89-P1: Intermediate C(4) (1 eq, 3.92 g), DPA (1 eq, 1.69 g), Pd₂(dba)₃ (0.03 eq, 0.27 g), t-BuONa (1.5 eq, 1.44 g), t-Bu₃P (0.05 eq, 0.06 ml), and 100 ml of ToI. were added to a one-neck round flask and stirred at 100 °C for 2 hours. The resultant reaction product was worked up with EA/H₂O and separated using column chromatography (MC/Hx = 1/5). 3.36 g of the obtained compound (89-P1) was obtained (Yield = 70%, Purity = 99%).

Synthesis of Compound 89: 89-P1 (1 eq, 3.36 g), N-phenylnaphthalen-1-amine (1 eq, 1.53 g), Pd₂(dba)₃ (0.03 eq, 0.189 g), t-BuONa (1.5 eq, 1 g), t-Bu₃P (0.05 eq, 0.04 ml), and 100 ml of ToI. were added to a one-neck round flask and stirred at 100 °C for 2 hours. The resultant reaction product was worked up with EA/H₂O and separated using column chromatography (MC/Hx = 1/5). 3.76 g of the obtained compound (89) was obtained (Yield = 83%, Purity = 99%). Purity of the obtained product was increased by ether recrystallization. When the purity level reached 99.8% or more, sublimation purification was performed thereon. 3.5 g of the obtained compound was obtained (Purity ≥99.9%)

### Synthesis Example 27: Synthesis of Compound 97

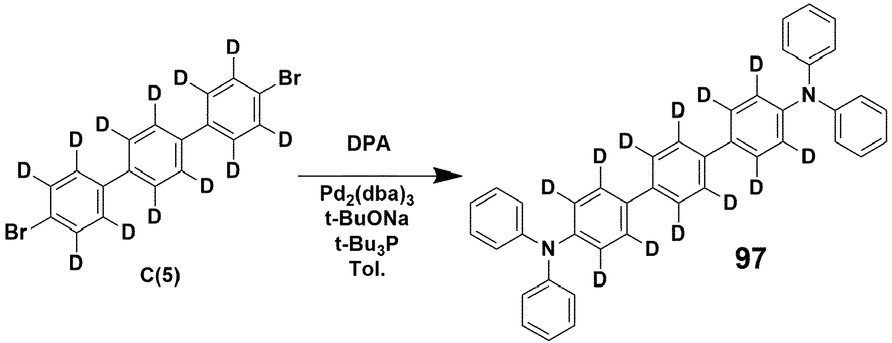

Intermediate C(5) (1 eq, 4 g), DPA (2.2 eq, 3.72 g), Pd₂(dba)₃ (0.05 eq, 0.46 g), t-BuONa (3 eq, 2.88 g), t-Bu₃P (0.1 eq, 0.11 ml), and 100 ml of ToI. were added to a one-neck round flask and stirred at 100 °C for 2 hours. The resultant reaction product was worked up with EA/H₂O and separated using column chromatography (MC/Hx = 1/5). 5 g of the obtained compound (Compound 97) was obtained (Yield = 88%, Purity = 99%). Purity of the obtained product was increased by ether recrystallization. When the purity level reached 99.9% or more, sublimation purification was performed thereon. 4.66 g of the obtained compound was obtained (Purity ≥99.9%).

### Synthesis Example 28: Synthesis of Compound 110

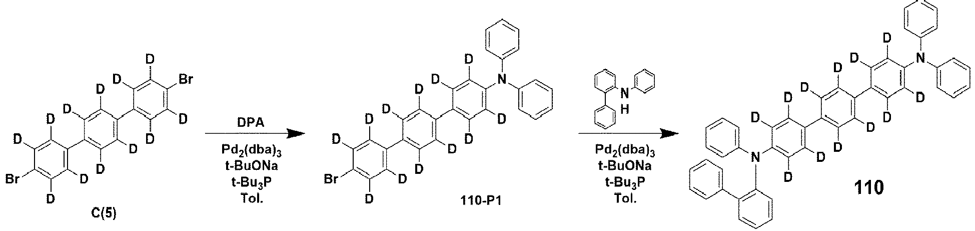

Synthesis of 110-P1: Intermediate C(5) (1 eq, 4 g), DPA (1 eq, 1.69 g), Pd₂(dba)₃ (0.03 eq, 0.27 g), t-BuONa (1.5 eq, 1.44 g), t-Bu₃P (0.05 eq, 0.06 ml), and 100 ml of ToI. were added to a one-neck round flask and stirred at 100 °C for 2 hours. The resultant reaction product was worked up with EA/H₂O and separated using column chromatography (MC/Hx = 1/5). 4.15 g of the obtained compound (110-P1) was obtained (Yield = 85%, Purity = 97%).

Synthesis of Compound 110: 110-P1 (1 eq, 4.15 g), N-phenyl-[1,1 '-biphenyl]-2-amine (1 eq, 2.08 g), Pd₂(dba)₃ (0.03 eq, 0.216 g), t-BuONa (1.5 eq, 1.14 g), t-Bu₃P (0.05 eq, 0.046 ml), and 100 ml of ToI. were added to a one-neck round flask and stirred at 100 °C for 2 hours. The resultant reaction product was worked up with EA/H₂O and separated using column chromatography (MC/Hx = 1/5). 4.37 g of the obtained compound (Compound 110) was obtained (Yield = 79%, Purity = 98.8%). Purity of the obtained product was increased by ether recrystallization. When the purity level reached 99.7% or more, sublimation purification was performed thereon. 4 g of the obtained compound was obtained (Purity ≥99.9%).

¹H NMR and MS/FAB of the compounds synthesized according to Synthesis Examples 1 to 28 are shown in Table 1 below.

Even compounds other than the compounds shown in Table 1 may be easily recognized by those skilled in the art by referring to the above synthesis routes and source materials.

**Table 1**

| Compound | ¹H NMR (CDCl₃, 400 MHz) | MS/FAB | |
|---|---|---|---|
| | | found | calc. |
| 1 | 7.55 (d, 2H), 7.37 (d, 2H), 7.25∼7.24 (m, 12H), 7.08∼7.00 (m, 12H) | 568.76 | 568.28 |
| 2 | 7.75 (d, 2H), 7.55∼7.37 (m, 11H), 7.25∼7.24 (m, 10H), 7.08∼7.00 (m, 9H) | 644.85 | 644.31 |
| 5 | 7.90∼7.86 (m, 2H), 7.55 (d, 3H), 7.38∼7.24 (m, 16H), 7.08∼7.00 (m, 9H), 1.69 (s, 6H) | 684.92 | 684.34 |
| 11 | 8.55 (d, 1H), 7.94 (d, 1H), 7.62∼7.50 (m, 8H), 7.37∼7.33 (m, 5H), 7.25∼7.00 (m, 20H) | 733.95 | 733.34 |
| 14 | 8.10 (d, 1H), 7.55 (d, 2H), 7.43∼7.37 (m, 7H), 7.25∼7.24 (m, 10H), 7.14∼7.00 (m, 12H) | 644.85 | 644.31 |
| 16 | 8.15∼8.00(d, 2H), 7.81 (d, 1H), 7.63∼7.49 (m, 6H), 7.37 (d, 2H), 7.25∼7.24 (m, 10H), 7.08∼7.00 (m, 9H) | 618.82 | 618.30 |
| 17 | 7.78∼7.71 (d, 2H), 7.55∼7.54 (m, 3H), 7.45∼7.37 (d, 5H), 7.25∼7.24 (m, 10H), 7.11∼7.00 (m, 10H) | 618.82 | 618.30 |
| 19 | 8.95 (d, 1H), 8.50 (d, 1H), 8.20 (d, 1H), 8.10∼8.09 (d, 2H), 7.77 (m, 1H), 7.55∼7.52(m, 3H), 7.39∼7.37(m, 5H), 7.24∼7.00(m, 20H) | 694.91 | 694.33 |
| 25 | 7.55 (d, 4H), 7.37 (d, 4H), 7.24 (m, 8H), 7.08∼7.00 (m, 12H) | 568.76 | 568.28 |
| 26 | 7.75 (d, 2H), 7.55∼7.37 (m, 15H), 7.24 (m, 6H), 7.08∼7.00 (m, 9H) | 644.85 | 644.31 |
| 29 | 7.90∼7.86 (m, 2H), 7.55 (d, 5H), 7.38∼7.00 (m, 23H), 1.69 (s, 6H) | 684.92 | 684.34 |
| 38 | 8.10 (d, 1H), 7.55 (d, 4H), 7.43∼7.37 (m, 9H), 7.24 (m, 6H), 7.14∼7.00 (m, 12H) | 644.85 | 644.31 |
| 40 | 8.15∼8.00(d, 2H), 7.81 (d, 1H), 7.63∼7.49 (m, 8H), 7.37 (d, 4H), 7.24 (m, 6H), 7.08∼7.00 (m, 9H) | 618.82 | 618.30 |
| 41 | 7.78∼7.71 (d, 2H), 7.55∼7.54 (m, 5H), 7.45∼7.37 (d, 7H), 7.25∼7.24 (m, 6H), 7.11∼7.00 (m, 10H) | 618.82 | 618.30 |
| 49 | 7.55 (d, 2H), 7.37 (d, 2H), 7.24 (m, 8H), 7.08∼7.00 (m, 12H) | 572.78 | 572.32 |
| 50 | 7.75 (d, 2H), 7.55∼7.37 (m, 11H), 7.24 (m, 6H), 7.08∼7.00 (m, 9H) | 648.88 | 648.34 |
| 53 | 7.90∼7.86 (m, 2H), 7.55 (d, 3H), 7.38∼7.00 (m, 21H), 1.69 (s, 6H) | 688.94 | 688.37 |
| 62 | 8.10 (d, 1H), 7.55 (d, 2H), 7.43∼7.37 (m, 7H), 7.24 (m, 6H), 7.14∼7.00 (m, 12H) | 648.88 | 648.34 |
| 64 | 8.15∼8.00(d, 2H), 7.81 (d, 1H), 7.63∼7.49 (m, 6H), 7.37 (d, 2H), 7.24 (m, 6H), 7.08∼7.00 (m, 9H) | 618.82 | 618.30 |
| 65 | 7.78∼7.71 (d, 2H), 7.55∼7.54 (m, 3H), 7.45∼7.37 (d, 5H), 7.25∼7.24 (m, 6H), 7.11∼7.00 (m, 10H) | 618.82 | 618.30 |
| 73 | 7.25∼7.24 (m, 12H), 7.08∼7.00 (m, 12H) | 572.78 | 572.32 |
| 74 | 7.75 (d, 2H), 7.55∼7.37 (m, 7H), 7.25∼7.24 (m, 10H), 7.08∼7.00 (m, 9H) | 648.88 | 648.34 |
| 77 | 7.90∼7.86 (m, 2H), 7.55 (d, 1H), 7.38∼7.24 (m, 14H), 7.08∼7.00 (m, 9H), 1.69 (s, 6H) | 688.94 | 688.37 |
| 86 | 8.10 (d, 1H), 7.43∼7.37 (m, 5H), 7.25∼7.24 (m, 10H), 7.14∼7.00 (m, 12H) | 648.88 | 648.34 |
| 88 | 8.15∼8.00(d, 2H), 7.81 (d, 1H), 7.63∼7.49 (m, 2H), 7.37 (d, 2H), 7.24 (m, 10H), 7.08∼7.00 (m, 9H) | 618.82 | 618.30 |
| 89 | 7.78∼7.71 (d, 2H), 7.55∼7.54 (m, 1H), 7.45∼7.37 (d, 3H), 7.25∼7.24 (m, 6H), 7.11∼7.00 (m, 10H) | 618.82 | 618.30 |
| 97 | 7.24 (m, 8H), 7.08∼7.00 (m, 12H) | 576.81 | 576.33 |
| 110 | 8.10 (d, 1H), 7.43∼7.37 (m, 5H), 7.24 (m, 6H), 7.14∼7.00 (m, 12H) | 652.90 | 652.36 |

### Comparative Example 1

As an anode, a 15 Ω/cm² (1,200 Å) ITO glass substrate, which was obtained from Corning Inc., was cut to a size of 50 mm x 50 mm x 0.7 mm, sonicated with isopropyl alcohol and pure water each for 5 minutes, and then cleaned by exposure to ultraviolet rays and ozone for 30 minutes. The ITO glass substrate was provided to a vacuum deposition apparatus.

On the glass substrate, first, 2-TNATA was vacuum-deposited to form a hole injection layer having a thickness of 600 Å, and 4,4'-bis[N-(1-naphthyl)-N-phenylamino]biphenyl (hereinafter, referred to as NPB) which is a hole transport material was vacuum-deposited as a hole transport compound to form a hole transport layer having a thickness of 300 Å.

9,10-Di(2-naphthyl)anthracene (ADN) which is a blue fluorescence host and 4,4'-bis[2-(4-(N,N-diphenylamino)phenyl)vinyl]biphenyl (hereinafter, referred to as DPAVBi) which is a blue phosphorescence dopant compound were co-deposited on the hole transport layer to a weight ratio of 98 : 2 to form an emission layer having a thickness of 300 Å.

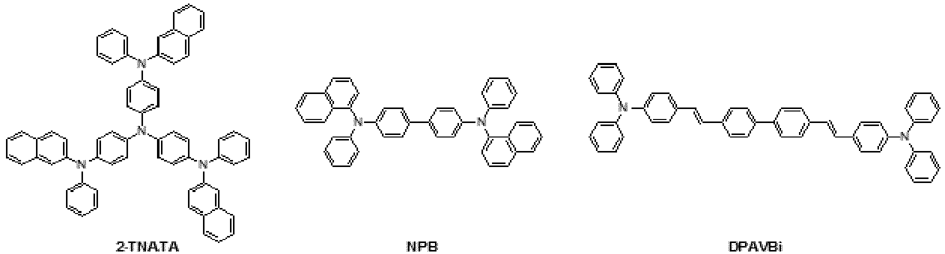

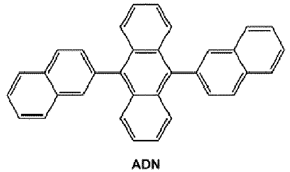

Then, Alq₃ was deposited on the emission layer to form an electron transport layer having a thickness of 300 Å, and then, LiF, which is a halogenated alkali metal, was deposited on the electron transport layer to form an electron injection layer having a thickness of 10 Å and Al was vacuum-deposited thereon to form an Al electrode having a thickness of 3000 Å(cathode) to form an LiF/AI electrode, thereby completing the manufacture of an organic light-emitting device.

### Examples 1 to 28

Organic light-emitting devices were manufactured in substantially the same manner as in Comparative Example 1, except that compounds shown in Table 2 were each used instead of NPB in forming a hole transport layer.

### Comparative Examples 2 to 7

Organic light-emitting devices were manufactured in substantially the same manner as in Comparative Example 1, except that Compounds A to F were respectively used instead of NPB in forming a hole transport layer.

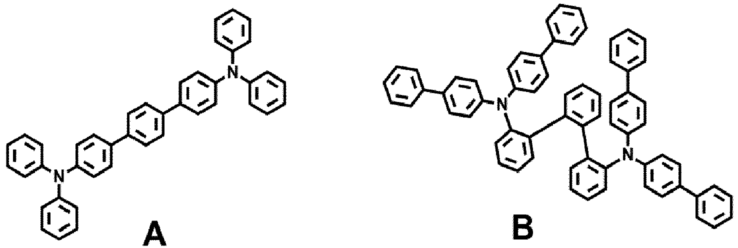

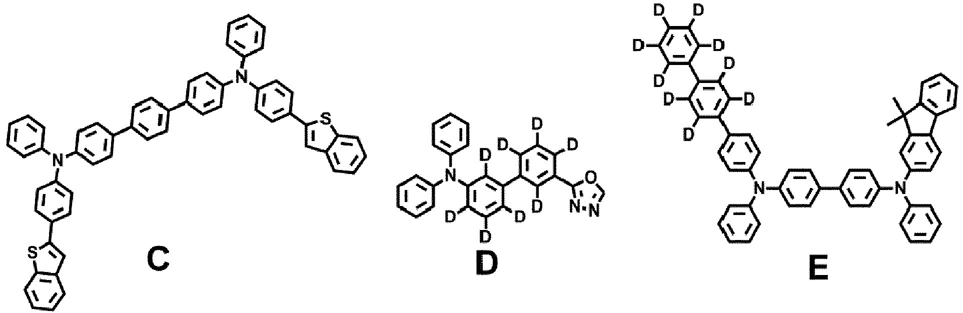

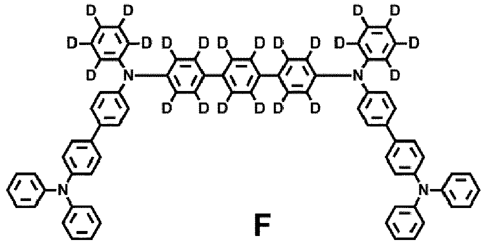

### Evaluation Example 1

The driving voltage, current density, luminance, efficiency, emission color, and a half lifespan of the organic light-emitting devices manufactured according to Examples 1 to 28, and Comparative Examples 1 to 7 were measured at 600 nit by using Keithley SMU 236 and a luminance photometer PR650, and results thereof are shown in Table 2.

**Table 2**

| | Hole transport material | Driving voltage (V) | Current density (mA/cm²) | Luminance (cd/m²) | Efficiency (cd/A) | Emission color | Half-lifespan (hr@100 mA/cm²) |
|---|---|---|---|---|---|---|---|
| Comparative Example 1 | NPB | 7.01 | 50 | 2645 | 5.29 | Blue | 258 |
| Comparative Example 2 | Compound A | 5.00 | 50 | 3120 | 6.54 | Blue | 240 |
| Comparative Example 3 | Compound B | 5.34 | 50 | 3310 | 6.84 | Blue | 340 |
| Comparative Example 4 | Compound C | 4.87 | 50 | 3294 | 6.74 | Blue | 322 |
| Comparative Example 5 | Compound D | 4.30 | 50 | 2920 | 6.14 | Blue | 219 |
| Comparative Example 6 | Compound E | 4.79 | 50 | 3320 | 6.60 | Blue | 330 |
| Comparative Example 7 | compound F | 5.51 | 50 | 3190 | 6.75 | Blue | 310 |
| Example 1 | Compound 1 | 4.32 | 50 | 3670 | 7.34 | Blue | 362 |
| Example 2 | Compound 2 | 4.21 | 50 | 3715 | 7.43 | Blue | 353 |
| Example 3 | Compound 5 | 4.22 | 50 | 3665 | 7.33 | Blue | 372 |
| Example 4 | Compound 11 | 4.26 | 50 | 3730 | 7.46 | Blue | 374 |
| Example 5 | Compound 14 | 4.26 | 50 | 3730 | 7.46 | Blue | 374 |
| Example 6 | Compound 16 | 4.25 | 50 | 3630 | 7.26 | Blue | 384 |
| Example 7 | Compound 17 | 4.25 | 50 | 3630 | 7.26 | Blue | 384 |
| Example 8 | Compound 19 | 4.41 | 50 | 3725 | 7.45 | Blue | 343 |
| Example 9 | Compound 25 | 4.22 | 50 | 3774 | 7.35 | Blue | 366 |
| Example 10 | Compound 26 | 4.21 | 50 | 3705 | 7.43 | Blue | 363 |
| Example 11 | Compound 29 | 4.24 | 50 | 3765 | 7.33 | Blue | 370 |
| Example 12 | Compound 38 | 4.26 | 50 | 3738 | 7.46 | Blue | 369 |
| Example 13 | Compound 40 | 4.25 | 50 | 3630 | 7.26 | Blue | 384 |
| Example 14 | Compound 41 | 4.25 | 50 | 3630 | 7.26 | Blue | 384 |
| Example 15 | Compound 49 | 4.20 | 50 | 3729 | 7.46 | Blue | 371 |
| Example 16 | Compound 50 | 4.20 | 50 | 3700 | 7.26 | Blue | 380 |
| Example 17 | Compound 53 | 4.24 | 50 | 3725 | 7.45 | Blue | 365 |
| Example 18 | Compound 62 | 4.27 | 50 | 3770 | 7.34 | Blue | 366 |
| Example 19 | Compound 64 | 4.25 | 50 | 3630 | 7.26 | Blue | 382 |
| Example 20 | Compound 65 | 4.25 | 50 | 3630 | 7.26 | Blue | 383 |
| Example 21 | Compound 73 | 4.21 | 50 | 3725 | 7.43 | Blue | 362 |
| Example 22 | Compound 74 | 4.20 | 50 | 3695 | 7.33 | Blue | 372 |
| Example 23 | Compound 77 | 4.23 | 50 | 3730 | 7.46 | Blue | 371 |
| Example 24 | Compound 86 | 4.25 | 50 | 3710 | 7.46 | Blue | 374 |
| Example 25 | Compound 88 | 4.25 | 50 | 3630 | 7.26 | Blue | 380 |
| Example 26 | Compound 89 | 4.25 | 50 | 3630 | 7.26 | Blue | 382 |
| Example 27 | Compound 97 | 4.20 | 50 | 3739 | 7.26 | Blue | 380 |
| Example 28 | Compound 110 | 4.28 | 50 | 3724 | 7.45 | Blue | 373 |

From Table 2, it can be seen that the organic light-emitting devices of Examples 1 to 28 had a lower driving voltage and higher efficiency than the organic light-emitting devices of Comparative Examples 1 to 7. In addition, it can be seen that the organic light-emitting devices of Examples 1 to 28 exhibit high luminance and a long lifespan compared to the organic light-emitting devices of Comparative Examples 1 to 7.

The organic light-emitting device comprising the amine compound according to embodiments of the present disclosure may have a low driving voltage, high luminance, high efficiency, and a long lifespan.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the scope as defined by the following claims.

## Claims

1. An amine compound represented by Formula 1: 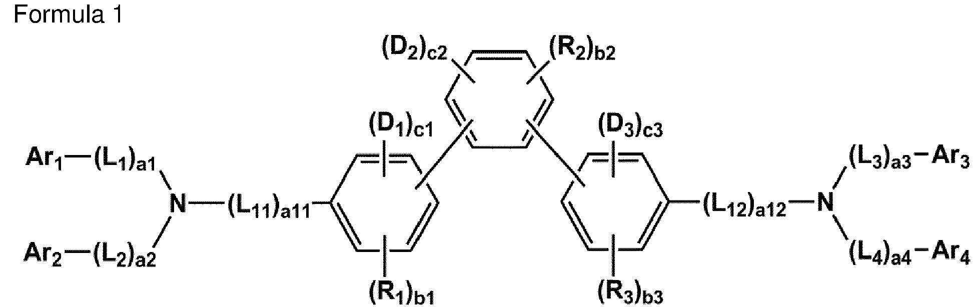
wherein L₁ to L₄, L₁₁, and L₁₂ are each independently a substituted or unsubstituted C₅-C₆₀ carbocyclic group, or a substituted or unsubstituted C₁-C₆₀ heterocyclic group,
a1 to a4, a11, and a12 are each independently an integer from 0 to 5,
Ar₁ to Ar₄ are each independently a substituted or unsubstituted C₅-C₆₀ carbocyclic group, or a substituted or unsubstituted C₁-C₆₀ heterocyclic group,
provided up to one of Ar₁ to Ar₄ comprises an amine group,
R₁ to R₃ are each indepednently hydrogen, deuterium, -F, -Cl, -Br, -I, a hydroxyl group, a cyano group, a nitro group, an amidino group, a hydrazino group, a hydrazono group, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group,-Si(Q₁)(Q₂)(Q₃), -N(Q₁)(Q₂), -B(Q₁)(Q₂), -C(=O)(Q₁), -S(=O)₂(Q₁), or -P(=O)(Q₁)(Q₂),
D₁ to D₃ are each deuterium,
b1 to b3 and c1 to c3 are each independently an integer from 0 to 4,
the sum of b1 and c1, the sum of b2 and c2, and the sum of b3 and c3 are each 4,
the sum of c1, c2, and c3 is 1 or more, and
at least one substituent of the substituted C₅-C₆₀ carbocyclic group, the substituted C₁-C₆₀ heterocyclic group, the substituted C₁-C₆₀ alkyl group, the substituted C₂-C₆₀ alkenyl group, the substituted C₂-C₆₀ alkynyl group, the substituted C₁-C₆₀ alkoxy group, the substituted C₃-C₁₀ cycloalkyl group, the substituted C₁-C₁₀ heterocycloalkyl group, the substituted C₃-C₁₀ cycloalkenyl group, the substituted C₁-C₁₀ heterocycloalkenyl group, the substituted C₆-C₆₀ aryl group, the substituted C₆-C₆₀ aryloxy group, the substituted C₆-C₆₀ arylthio group, the substituted C₁-C₆₀ heteroaryl group, the substituted monovalent non-aromatic condensed polycyclic group, and the substituted monovalent non-aromatic condensed heteropolycyclic group is selected from:
deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, and a C₁-C₆₀ alkoxy group;
a C₁-C₆₀ alkyl group, a C₂-C₆₀ alkenyl group, a C₂-C₆₀ alkynyl group, and a C₁-C₆₀ alkoxy group, each substituted with at least one selected from deuterium, -F, -Cl,-Br, -I, a hydroxyl group, a cyano group, a nitro group, an amidino group, a hydrazino group, a hydrazono group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -Si(Q₁₁)(Q₁₂)(Q₁₃), -N(Q₁₁)(Q₁₂), -B(Q₁₁)(Q₁₂), -C(=O)(Q₁₁),-S(=O)₂(Q₁₁), and -P(=O)(Q₁₁)(Q₁₂);
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group;
a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, and a monovalent non-aromatic condensed heteropolycyclic group, each substituted with at least one selected from deuterium, -F, -CI, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, -Si(Q₂₁)(Q₂₂)(Q₂₃),-N(Q₂₁)(Q₂₂), -B(Q₂₁)(Q₂₂), -C(=O)(Q₂₁), -S(=O)₂(Q₂₁), and -P(=O)(Q₂₁)(Q₂₂); and
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), and-P(=O)(Q₃₁)(Q₃₂),
wherein Q₁ to Q₃, Q₁₁ to Q₁₃, Q₂₁ to Q₂₃, and Q₃₁ to Q₃₃ are each independently selected from hydrogen, deuterium, -F, -Cl, -Br, -I, a hydroxyl group, a cyano group, a nitro group, an amidino group, a hydrazino group, a hydrazono group, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, a monovalent non-aromatic condensed heteropolycyclic group, a biphenyl group, and a terphenyl group.

2. The amine compound of claim 1, wherein L₁ to L₄, L₁₁, and L₁₂ are each independently selected from:
a phenylene group, a pentalenylene group, an indenylene group, a naphthylene group, an azulenylene group, a heptalenylene group, an indacenylene group, an acenaphthylene group, a fluorenylene group, a spiro-bifluorenylene group, a spiro-fluorene-benzofluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, a ovalenylene group, and a carbazolylene group; and
a phenylene group, a pentalenylene group, an indenylene group, a naphthylene group, an azulenylene group, a heptalenylene group, an indacenylene group, an acenaphthylene group, a fluorenylene group, a spiro-bifluorenylene group, a spiro-fluorene-benzofluorenylene group, a benzofluorenylene group, a dibenzofluorenylene group, a phenalenylene group, a phenanthrenylene group, an anthracenylene group, a fluoranthenylene group, a triphenylenylene group, a pyrenylene group, a chrysenylene group, a naphthacenylene group, a picenylene group, a perylenylene group, a pentaphenylene group, a hexacenylene group, a pentacenylene group, a rubicenylene group, a coronenylene group, an ovalenylene group, and a carbazolylene group, each substituted with at least one selected from deuterium, -F, -Cl, -Br, -I, a hydroxyl group, a cyano group, a nitro group, an amidino group, a hydrazino group, a hydrazono group, a C₁-C₂₀ alkyl group, a C₁-C₂₀ alkoxy group, a cyclopentyl group, a cyclohexyl group, a cycloheptyl group, a cyclopentenyl group, a cyclohexenyl group, a phenyl group, a biphenyl group, a terphenyl group, a pentalenyl group, an indenyl group, a naphthyl group, an azulenyl group, a heptalenyl group, an indacenyl group, an acenaphthyl group, a fluorenyl group, a spiro-bifluorenyl group, a benzofluorenyl group, a dibenzofluorenyl group, a phenalenyl group, a phenanthrenyl group, an anthracenyl group, a fluoranthenyl group, a triphenylenyl group, a pyrenyl group, a chrysenyl group, a naphthacenyl group, a picenyl group, a perylenyl group, a pentaphenyl group, a hexacenyl group, a pentacenyl group, a rubicenyl group, a coronenyl group, an ovalenyl group, a pyrrolyl group, a thiophenyl group, a furanyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a carbazolyl group, a phenanthridinyl group, an acridinyl group, a phenanthrolinyl group, a phenazinyl group, a benzimidazolyl group, a benzofuranyl group, a benzothiophenyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a dibenzosilolylene group, a benzocarbazolyl group, a dibenzocarbazolyl group, a thiadiazolyl group, an imidazopyridinyl group, an imidazopyrimidinyl group, -Si(Q₃₁)(Q₃₂)(Q₃₃), -N(Q₃₁)(Q₃₂),-B(Q₃₁)(Q₃₂), -C(=O)(Q₃₁), -S(=O)₂(Q₃₁), and -P(=O)(Q₃₁)(Q₃₂),
wherein Q₃₁ to Q₃₃ are each independently selected from a C₁-C₁₀ alkyl group, a C₁-C₁₀ alkoxy group, a phenyl group, a phenyl group substituted with a C₁-C₁₀ alkyl group, a biphenyl group, a terphenyl group, and a naphthyl group.

3. The amine compound of claim 1 or 2, wherein a11 and a12 are each 0.

4. The amine compound of any preceding claim, wherein Ar₁ to Ar₄ are each independently selected from:
a phenyl group, a naphthyl group, a fluorenyl group, a spiro-bifluorenyl group, a spiro-fluorene-benzofluorenyl group, a benzofluorenyl group, a dibenzofluorenyl group, a benzonaphthofluorenyl group, a pyrenyl group, a phenalenyl group, a phenanthrenyl group, an anthracenyl group, a fluoranthenyl group, a triphenylenyl group, a pyrrolyl group, a thiophenyl group, a furanyl group, a silolyl group, an imidazolyl group, a pyrazolyl group, a thiazolyl group, an isothiazolyl group, an oxazolyl group, an isoxazolyl group, a pyridinyl group, a pyrimidyl group, a quinolinyl group, an isoquinolinyl group, a benzoquinolinyl group, a naphthyridinyl group, a quinoxalinyl group, a quinazolinyl group, a carbazolyl group, a phenanthridinyl group, an acridinyl group, a phenanthrolinyl group, a phenazinyl group, a triazinyl group, a dibenzofuranyl group, a dibenzothiophenyl group, a biphenyl group, and a terphenyl group; and
a phenyl group, a naphthyl group, a fluorenyl group, a spiro-bifluorenyl group, a spiro-fluorene-benzofluorenyl group, a benzofluorenyl group, a dibenzofluorenyl group, a benzonaphthofluorenyl group, a pyrenyl group, a phenalenyl group, a phenanthrenyl group, an anthracenyl group, a fluoranthenyl group, a triphenylenyl group, a pyrrolyl group, a thiophenyl group, a furanyl group, a silolyl group, an imidazolyl group, a pyrazolyl group, a thiazolyl group, an isothiazolyl group, an oxazolyl group, an isoxazolyl group, a pyridinyl group, a pyrimidyl group, a quinolinyl group, an isoquinolinyl group, a benzoquinolinyl group, a naphthyridinyl group, a quinoxalinyl group, a quinazolinyl group, a carbazolyl group, a phenanthridinyl group, an acridinyl group, a phenanthrolinyl group, a phenazinyl group, a triazinyl group, a dibenzofuranyl group, a dibenzothiophenyl group, a biphenyl group, and a terphenyl group, each substituted with at least one selected from deuterium, -F, -Cl, -Br, -I, a hydroxyl group, a cyano group, a nitro group, an amidino group, a hydrazino group, a hydrazono group, a methyl group, an ethyl group, a propyl group, an isobutyl group, a sec-butyl group, a ter-butyl group, pentyl group, an isoamyl group, a hexyl group, a C₁-C₂₀ alkoxy group, a cyclopentyl group, a cyclohexyl group, a cycloheptyl group, a cyclopentenyl group, a cyclohexenyl group, a phenyl group, a naphthyl group, a fluorenyl group, a spiro-bifluorenyl group, a spiro-fluorene-benzofluorenyl group, a benzofluorenyl group, a dibenzofluorenyl group, a benzonaphthofluorenyl group, a pyrenyl group, a phenalenyl group, a phenanthrenyl group, an anthracenyl group, a fluoranthenyl group, a triphenylenyl group, a pyrrolyl group, a thiophenyl group, a furanyl group, a silolyl group, an imidazolyl group, a pyrazolyl group, a thiazolyl group, an isothiazolyl group, an oxazolyl group, an isoxazolyl group, a pyridinyl group, a pyrazinyl group, a pyrimidinyl group, a pyridazinyl group, an indolyl group, an isoindolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, a benzofuranyl group, a benzothiophenyl group, a benzosilolyl group, an isobenzothiazolyl group, a benzoxazolyl group, an isobenzoxazolyl group, a triazolyl group, a tetrazolyl group, an oxadiazolyl group, a triazinyl group, a dibenzofuranyl group, a dibenzothiophenyl group, a dibenzosilolyl group, a carbazolyl group, a benzocarbazolyl group, a dibenzocarbazolyl group, a thiadiazolyl group, an imidazopyridinyl group, an imidazopyrimidinyl group, an oxazolopyridinyl group, a thiazolopyridinyl group, a benzonaphthyridinyl group, an azafluorenyl group, an azaspiro-bifluorenyl group, an azacarbazolyl group, an azadibenzofuranyl group, an azadibenzothiophenyl group, an azadibenzosilolyl group, a biphenyl group, and a terphenyl group.

5. The amine compound of any preceding claim, wherein Ar₁ to Ar₄ are each independently a group represented by one of Formulae 5-1 to 5-27: 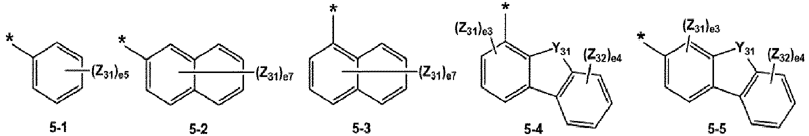 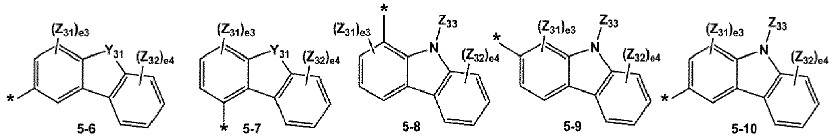 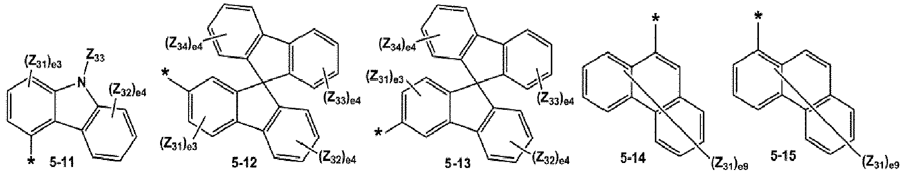 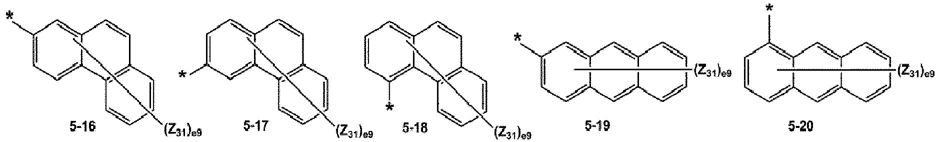 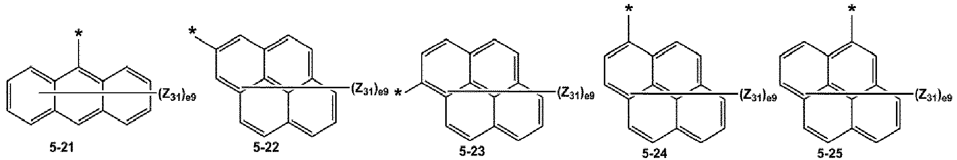 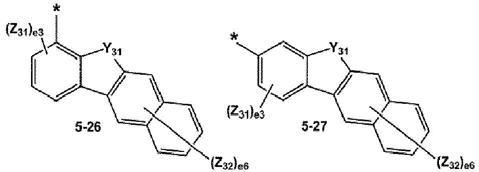 wherein, in Formulae 5-1 to 5-27,
Y₃₁ is O, S, C(Z₃₅)(Z₃₆), or Si(Z₃₇)(Z₃₈);
Z₃₁ to Z₃₈ are each independently any one of hydrogen, deuterium, -F, -CI, -Br, - I, a hydroxyl group, a cyano group, a nitro group, an amidino group, a hydrazino group, a hydrazono group, a C₁-C₂₀ alkyl group, a C₁-C₂₀ alkoxy group, a phenyl group, a biphenyl group, a terphenyl group, a naphthyl group, a fluorenyl group, an anthracenyl group, a phenanthrenyl group, an imidazolyl group, a pyrazole group, a thiazolyl group, an isothiazolyl group, an oxazolyl group, an isoxazolyl group, a pyridinyl group, a pyrazinyl group, pyrimidinyl, a pyridazinyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzimidazolyl group, an isobenzothiazolyl group, a benzoxazolyl group, an isobenzoxazolyl group, a triazolyl group, a tetrazolyl group, an oxadiazolyl group, a triazinyl group, a thiadiazolyl group, an imidazopyridinyl group, an imidazopyrimidinyl group, and an azacarbazolyl group;
e3 is an integer from 0 to 3,
e4 is an integer from 0 to 4,
e5 is an integer from 0 to 5,
e6 is an integer from 0 to 6,
e7 is an integer from 0 to 7,
e9 is an integer from 0 to 9, and
* indicates a binding site to an adjacent atom.

6. The amine compound of any preceding claim, wherein none of Ar₁ to Ar₄ comprises an amine group.

7. The amine compound of any preceding claim, wherein R₁ to R₃ are each independently selected from:
hydrogen, deuterium, -F, -Cl, -Br, -I, a hydroxyl group, a cyano group, a nitro group, a methyl group, an ethyl group, a propyl group, an isobutyl group, a sec-butyl group, a ter-butyl group, a C₁-C₂₀ alkoxy group, a cyclopentyl group, a cyclohexyl group, a phenyl group, a naphthyl group, a fluorenyl group, a carbazolyl group, a dibenzofuranyl group, a dibenzothiophenyl group, a biphenyl group, and a terphenyl group; and
a methyl group, an ethyl group, a propyl group, an isobutyl group, a sec-butyl group, a ter-butyl group, a C₁-C₂₀ alkoxy group, a cyclopentyl group, a cyclohexyl group, a cyclopentyl group, a cyclohexyl group, a phenyl group, a naphthyl group, a fluorenyl group, a carbazolyl group, a dibenzofuranyl group, a dibenzothiophenyl group, a biphenyl group, and a terphenyl group, each substituted with at least one selected from deuterium, -F, -Cl, -Br, -I, a hydroxyl group, a cyano group, a nitro group, an amidino group, a hydrazino group, a hydrazono group, a methyl group, an ethyl group, a propyl group, an isobutyl group, a sec-butyl group, a ter-butyl group, a phenyl group, a naphthyl group, a fluorenyl group, a carbazolyl group, a dibenzofuranyl group, a dibenzothiophenyl group, a biphenyl group, and a terphenyl group; and/or
wherein at least one of R₁ to R₃ is hydrogen.

8. The amine compound of any preceding claim, wherein one of c1 to c3 is 4 and the other two are each 0,
two of c1 to c3 are each 4 and the other one is 0,
c1 to c3 are each 4, or
the sum of c1, c2, and c3 is 4 or more.

9. The amine compound of any preceding claim, wherein a moiety represented by 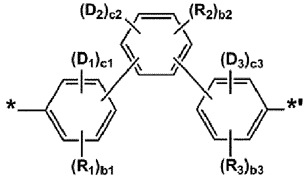 in Formula 1 is a group represented by one of Formulae 2-1 to 2-6: 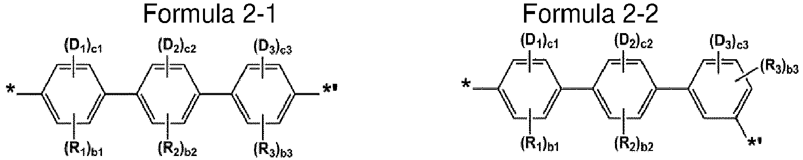 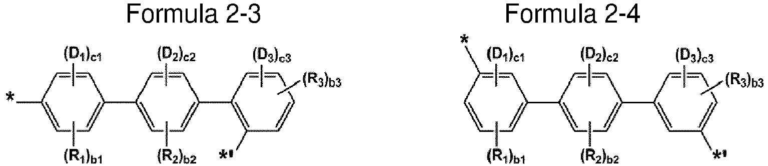 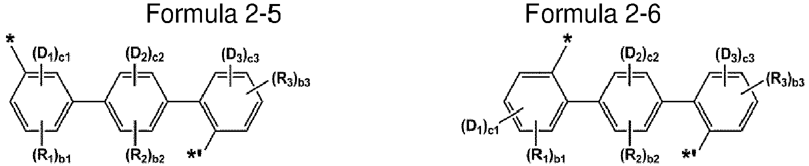 wherein, in Formulae 2-1 to 2-6,
R₁ to R₃, D₁ to D₃, b1 to b3, and c1 to c3 are the same as described in connection with claim 1, and
* and *' each indicate a binding site to a neighboring atom.

10. The amine compound of any preceding claim, wherein the amine compound represented by Formula 1 is selected from Compounds 1 to 192:

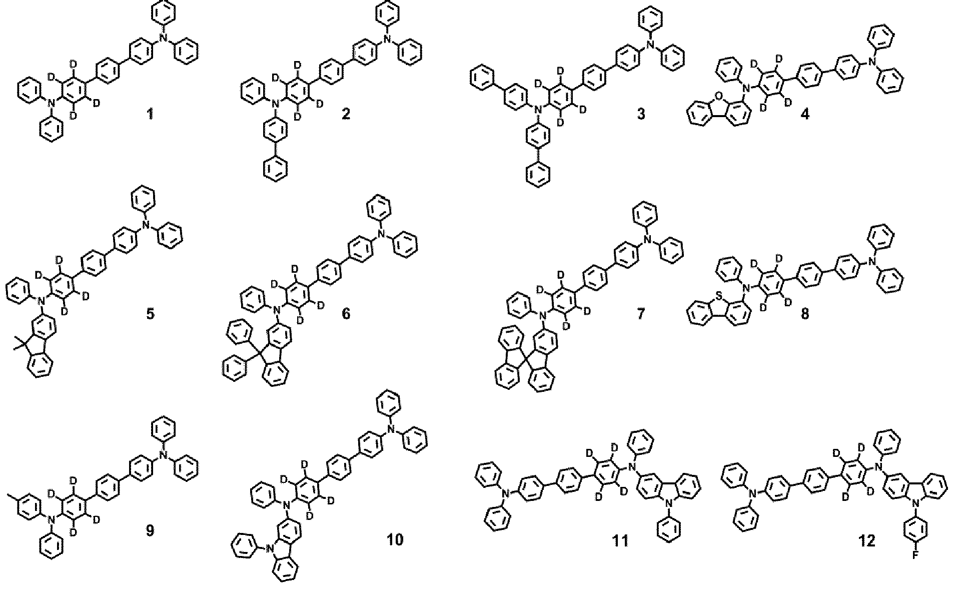

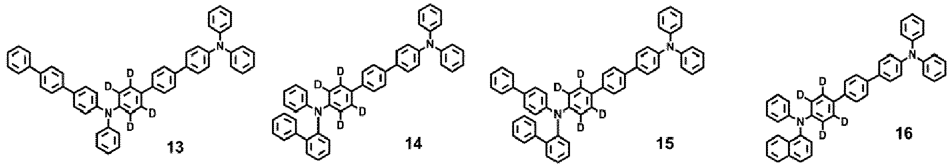

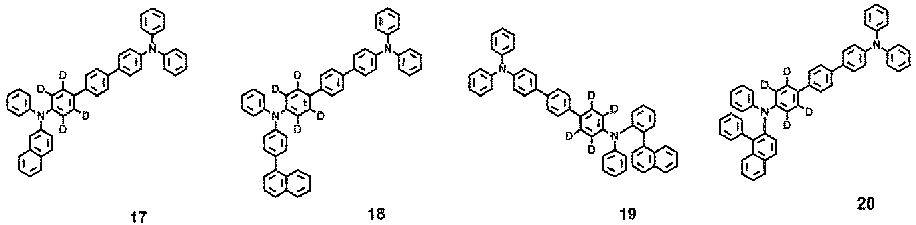

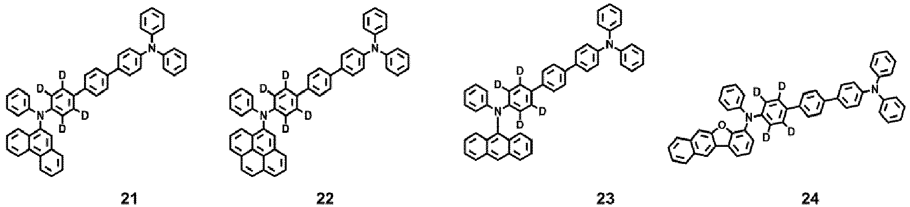

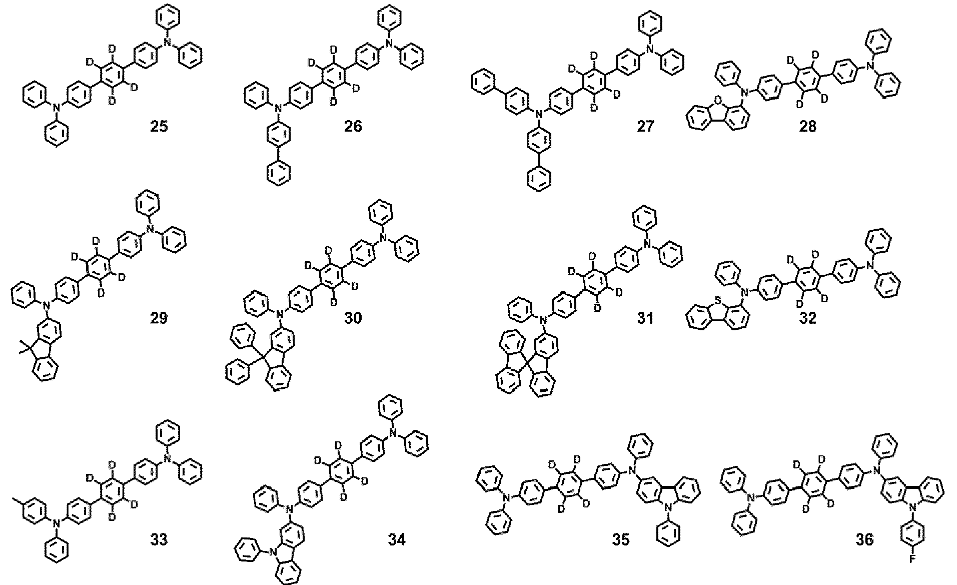

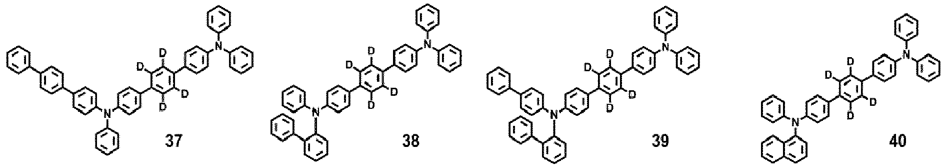

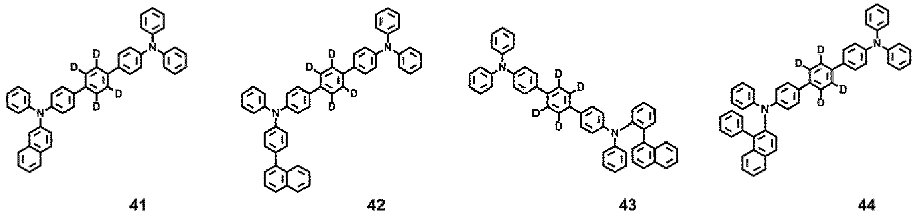

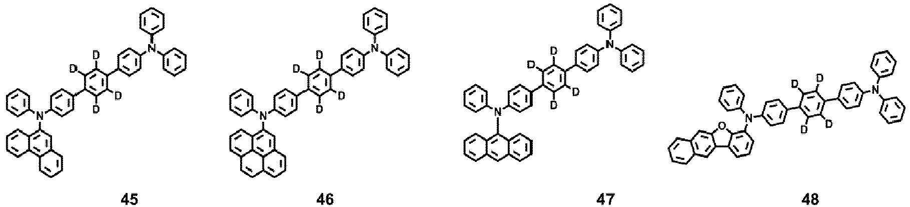

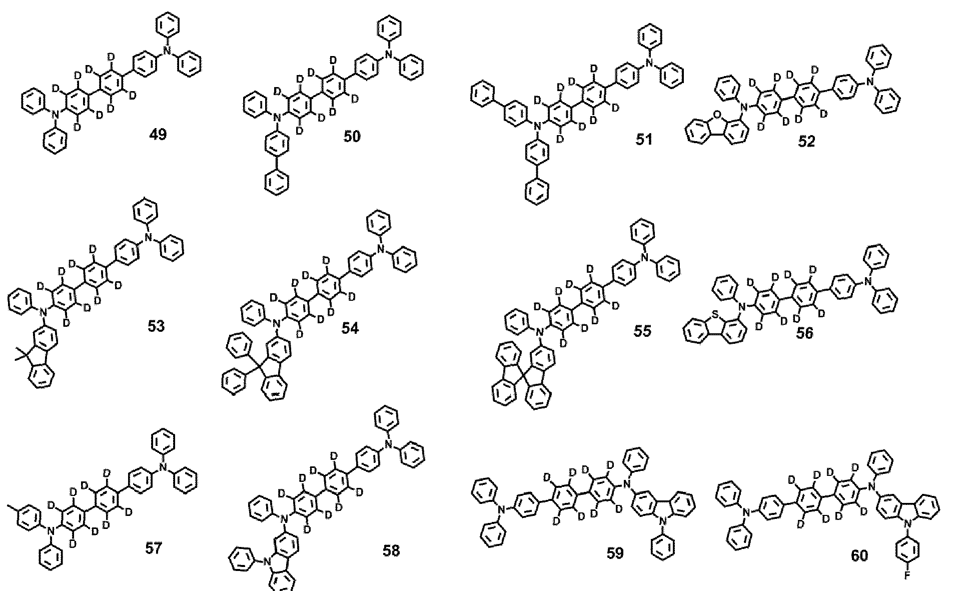

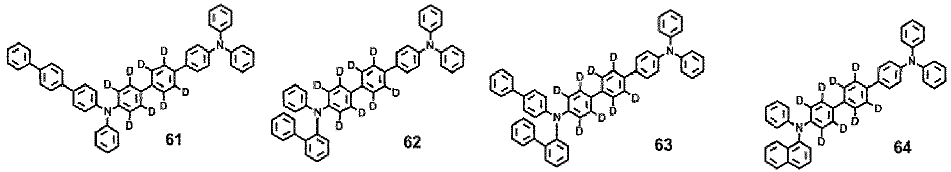

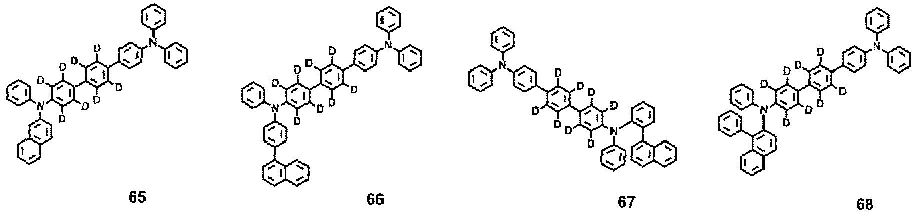

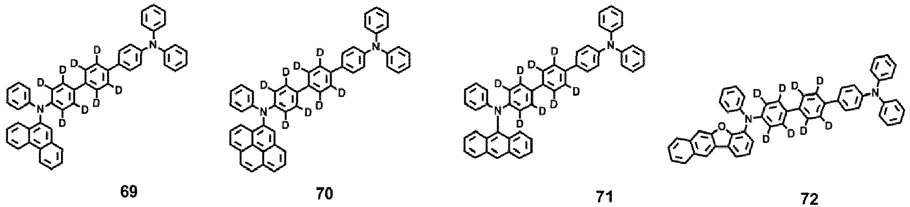

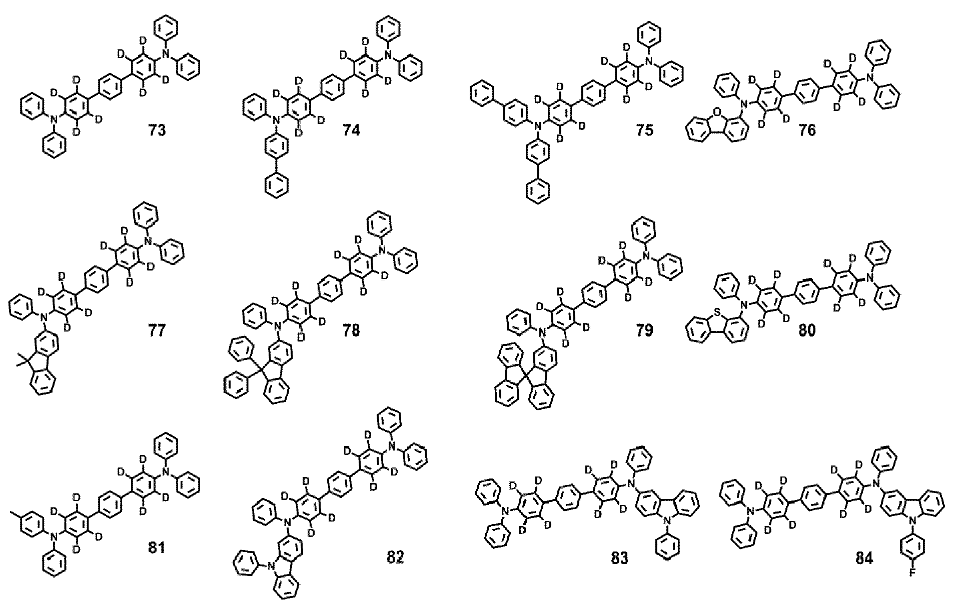

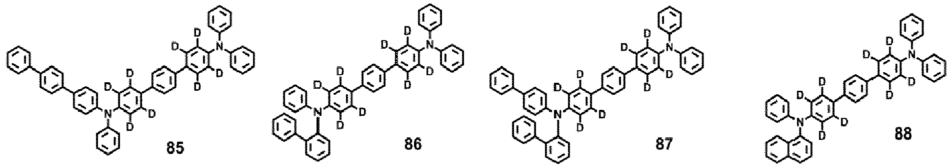

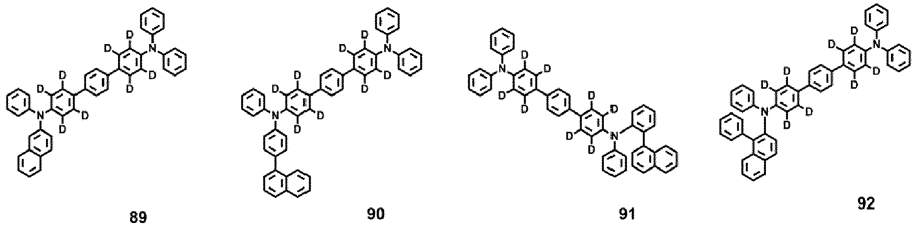

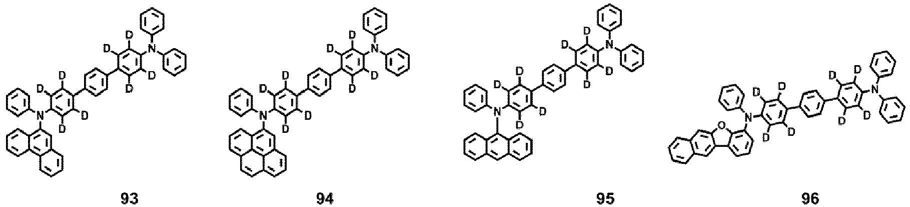

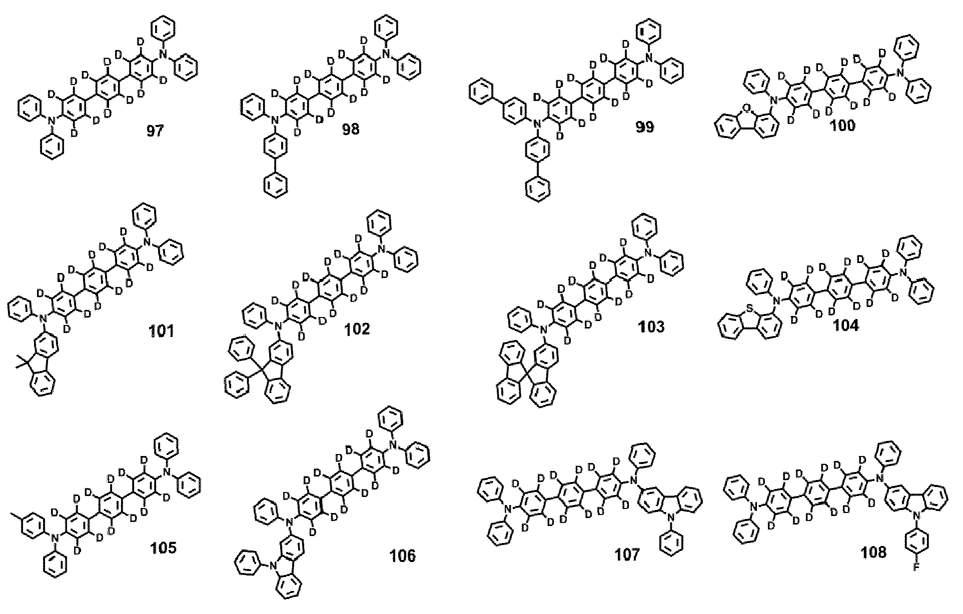

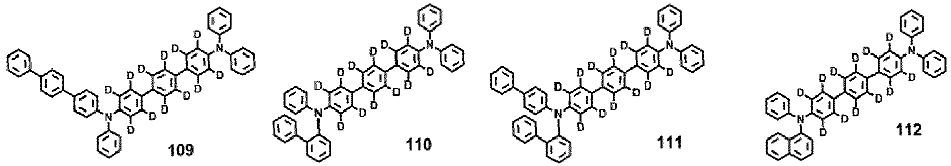

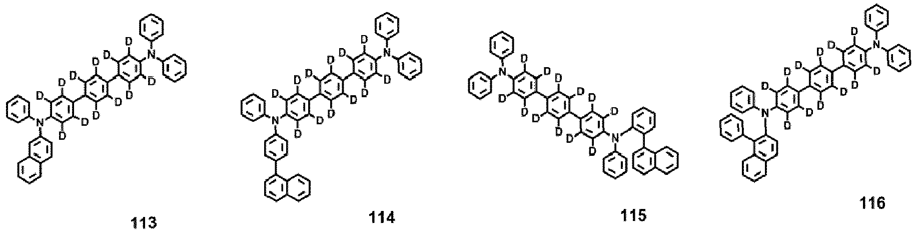

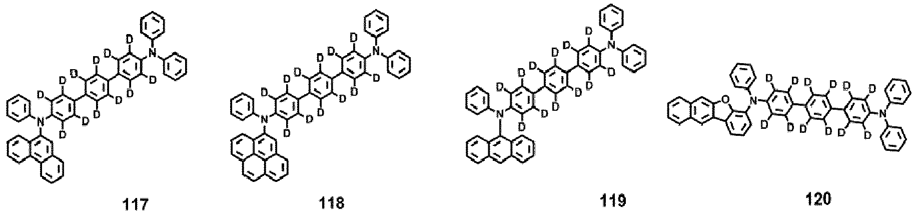

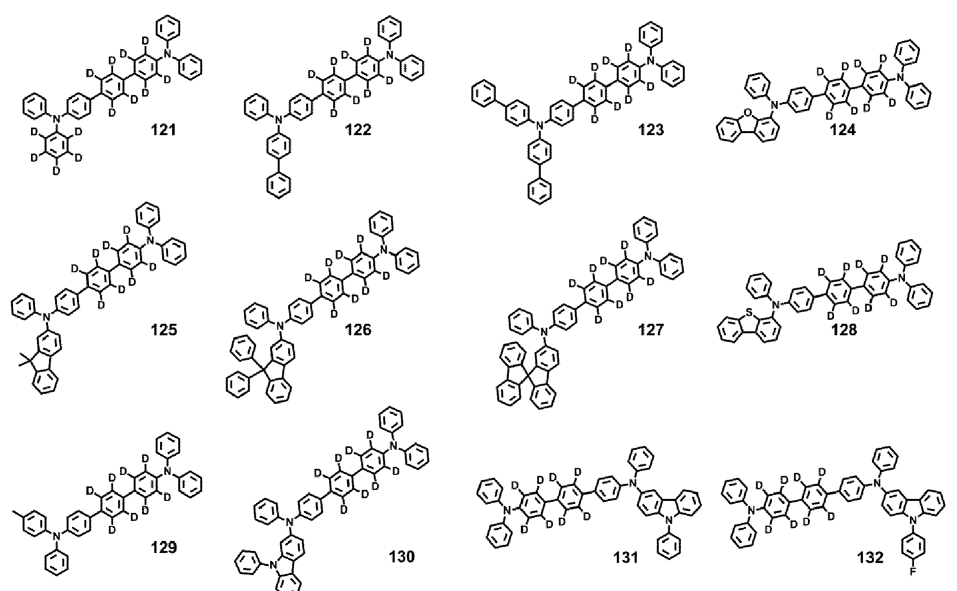

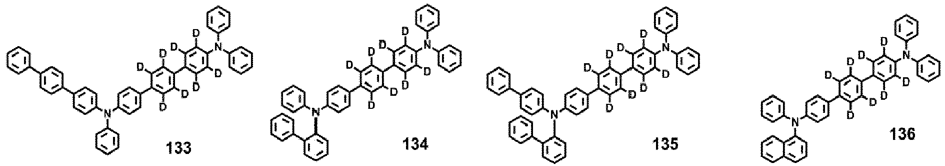

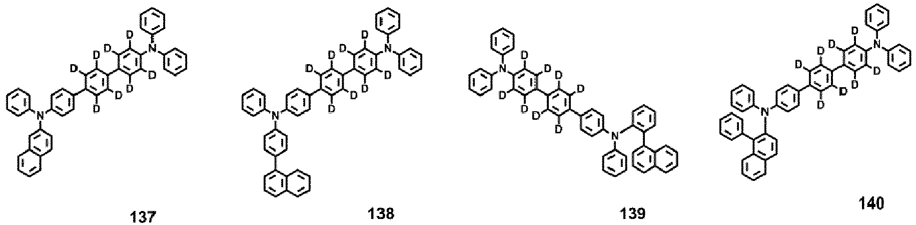

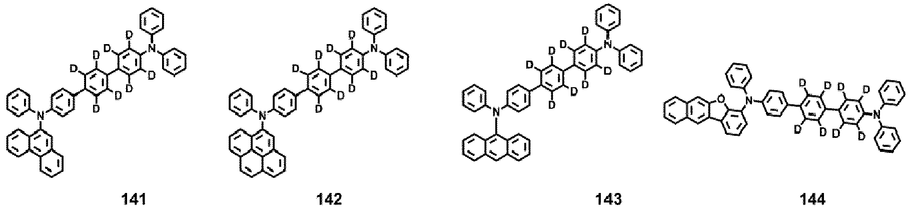

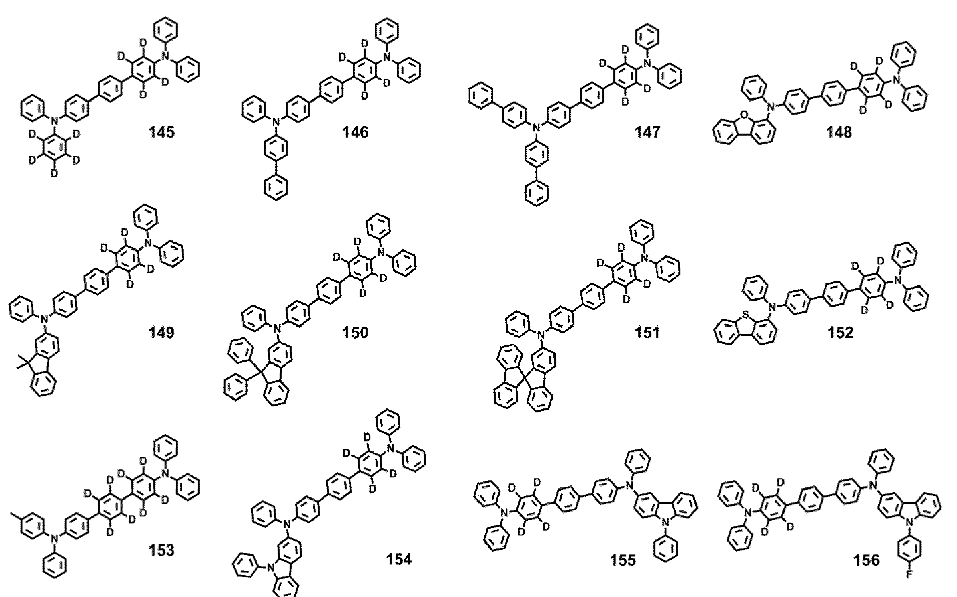

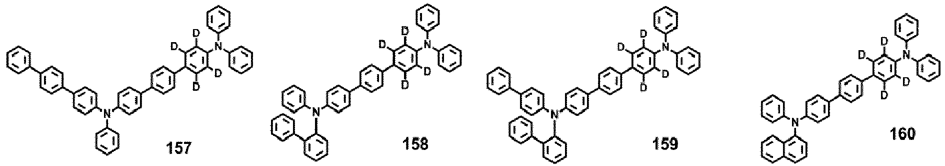

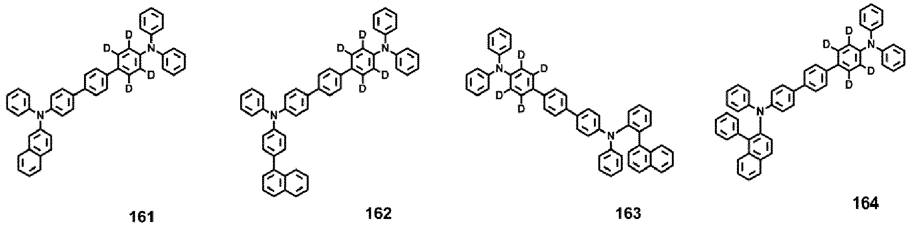

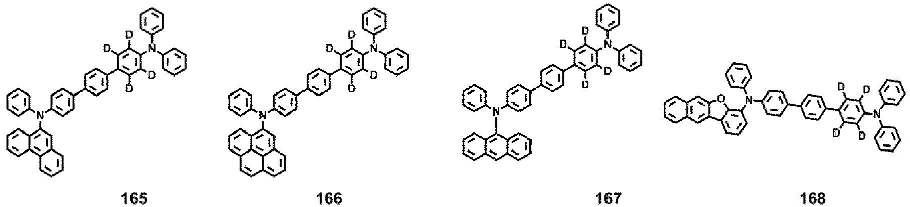

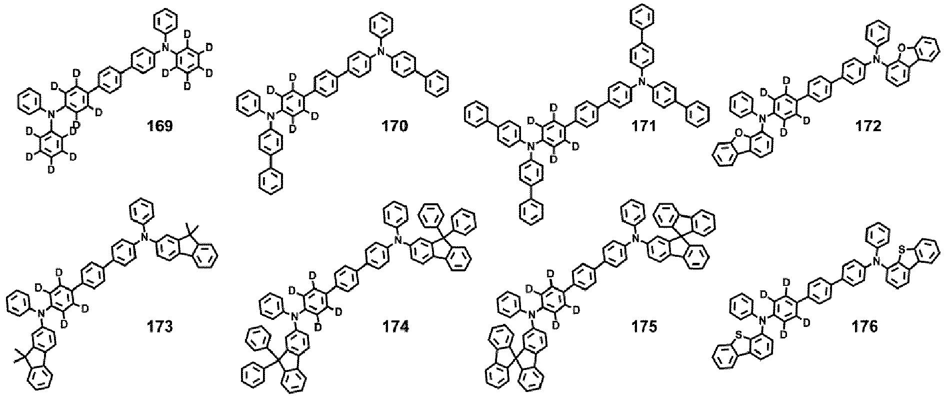

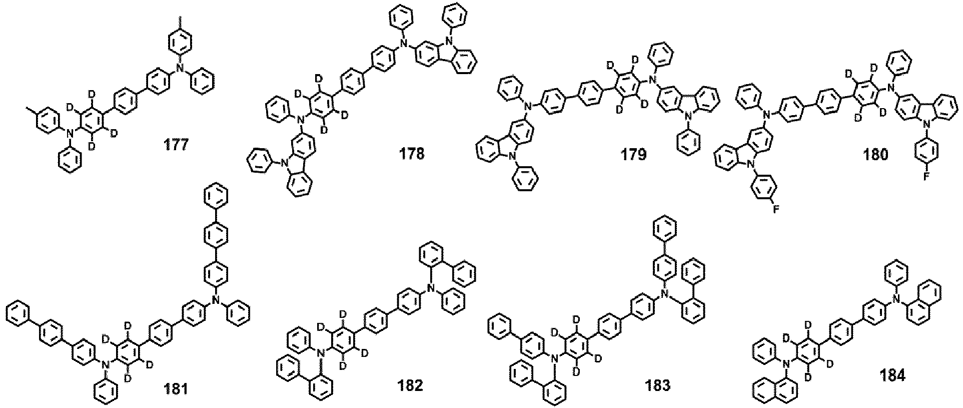

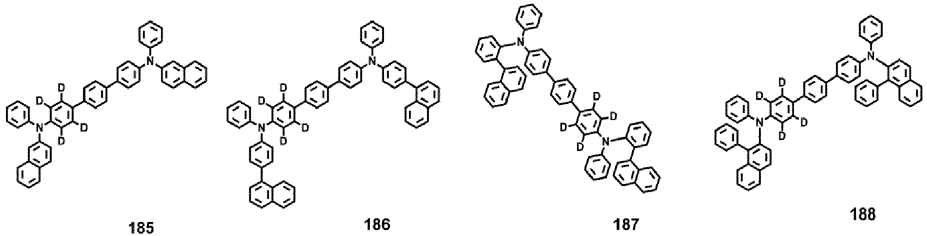

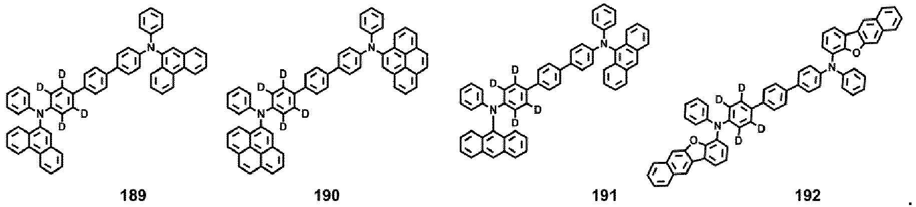

11. An organic light-emitting device comprising:
a first electrode;
a second electrode facing the first electrode;
an organic layer between the first electrode and the second electrode and comprising an emission layer; and
at least one amine compound represented by Formula 1 as defined in any preceding claim.

12. The organic light-emitting device of claim 11, wherein the first electrode is an anode,
the second electrode is a cathode,
the organic layer further comprises a hole transport region between the first electrode and the emission layer and an electron transport region between the emission layer and the second electrode,
the hole transport region comprises a hole injection layer, a hole transport layer, an emission auxiliary layer, an electron blocking layer, or any combination thereof, and
the electron transport region comprises a buffer layer, a hole blocking layer, an electron control layer, an electron transport layer, an electron injection layer, or any combination thereof.

13. The organic light-emitting device of claim 12, wherein the at least one amine compound is comprised in the hole transport region, preferably wherein the hole transport region comprises the hole transport layer, and the at least one amine compound is comprised in the hole transport layer; and/or
wherein the hole transport region further comprises a p-dopant having a lowest unoccupied molecular orbital (LUMO) energy level of less than about -3.5 eV.

14. The organic light-emitting device of any one of claims 11 to 13, wherein the emission layer comprises a host and a dopant, and
the dopant comprises a fluorescent dopant comprising a styryl-based compound, and/or
the dopant comprises a phosphorescent dopant.

15. The organic light-emitting device of any one of claims 12 to14, wherein the electron transport region comprises an alkali metal, an alkaline earth metal, a rare earth metal, an alkali metal compound, an alkaline earth-metal compound, a rare earth metal compound, an alkali metal complex, an alkaline earth-metal complex, a rare earth metal complex, or any combinations thereof.
